# FASCICULE DE BREVET EUROPEEN

(11) **EP 1 465 880 B1**
(45) Date de publication et mention de la délivrance du brevet: **05.08.2009**
(21) Numéro de dépôt: 02796876.7
(22) Date de dépôt: 27.11.2002
(51) Int. Cl.: C07D 249/12, C07D 405/12, C07D 403/12, C07D 405/04, C07D 417/04, C07D 409/04, C07D 403/04, C07D 401/04, A61K 31/4196, A61P 35/00, A61P 5/00, A61P 25/00

(54) **DERIVES DE 5-SULFANYL-4H-1,2,4-TRIAZOLES POUR TRAITER LES DÉSORDRES ASSOCIÉS À LA SOMATOSTATINE**
5-SULFANYL-4H-1,2,4-TRIAZOLDERIVATE UND DEREN VERWENDUNG ZUR BEHANDLUNG VON SOMATOSTATINE VERMITTELTEN KRANKHEITEN
5-SULPHANYL-4H-1,2,4-TRIAZOLE DERIVATIVES AND THEIR USE TO TREAT DISORDERS ASSOCIATED WITH SOMATOSTATINE

(30) Priorité: 28.11.2001 FR 0115342; 21.06.2002 FR 0207697
(43) Date de publication de la demande: 13.10.2004
(73) Titulaire: IPSEN PHARMA, 92100 Boulogne-Billancourt (FR)
(72) Inventeur: GALCERA CONTOUR, Marie-Odile, F-91070 Bondoufle (FR); SIDHU, Alban, F-91120 Palaiseau (FR); ROUBERT, Pierre, F-75014 Paris (FR); THURIEAU, Christophe, F-75116 Paris (FR)
(74) Mandataire: Bourgouin, André
(86) Numéro de dépôt international: PCT/FR2002/004055
(87) Numéro de publication internationale: WO 2003/045926

(56) Documents cités:
- WO-A-99/64401
- FR-A- 2 796 945
- FR-A- 2 802 206
- FOKS H ET AL: "SYNTHESIS OF NEW 5-SUBSTITUTED 1,2,4-TRIAZOLE-3-THIONE DERIVATIVES" PHOSPHORUS, SULFUR AND SILICON AND THE RELATED ELEMENTS, GORDON AND BREACH SCIENCE PUBLISHERS, AMSTERDAM, GB, no. 164, 2000, pages 67-81, XP008004230 ISSN: 1042-6507

## Description

La présente demande a pour objet de nouveaux dérivés de 5-sulfanyl-4*H*-1,2,4-triazoles et leurs procédés de préparation par des méthodes de synthèse en parallèle en phase liquide. Ces produits ayant une bonne affinité pour certains sous-types de récepteurs de la somatostatine, ils sont particulièrement intéressants pour traiter les états pathologiques ou les maladies dans lesquels un (ou plusieurs) des récepteurs de la somatostatine est (sont) impliqué(s). L'invention concerne également des compositions pharmaceutiques contenant lesdits produits et leur utilisation pour la préparation d'un médicament.

La somatostatine (SST) est un tétradécapeptide cyclique qui a été isolé pour la première fois de l'hypothalamus en tant que substance inhibitrice de l'hormone de croissance (Brazeau P. et al., Science 1973, 179, 77-79). Elle intervient également en tant que neurotransmetteur dans le cerveau (Reisine T. et al., Neuroscience 1995, 67, 777-790 ; Reisine et al., Endocrinology 1995, 16, 427-442). L'hétérogénéité des fonctions biologiques de la somatostatine et les relations structure-activité de ses analogues peptidiques, ont amené la découverte de 5 sous-types de récepteurs liés à la membrane (Yamada et al., Proc. Natl. Acad. Sci. U.S.A, 89, 251-255, 1992 ; Raynor, K. et al, Mol. Pharmacol., 44, 385-392, 1993). Le clonage moléculaire a permis de montrer que la bioactivité de la somatostatine dépend directement de ces cinq sous-types de récepteurs.

Les rôles fonctionnels de ces récepteurs sont actuellement activement étudiés. L'activation préférentielle des sous-types 2 et 5 a été associée à la suppression, dans les adénomes sécrétant ces hormones, de l'hormone de croissance GH (acromégalie), de l'hormone TSH et de la prolactine ; mais le rôle précis de chaque sous-type reste à déterminer.

Parmi les désordres pathologiques associés à la somatostatine (Moreau J.P. et al., Life Sciences, 1987, 40, 419 ; Harris A.G. et al., The European Journal of Medicine, 1993, 2, 97-105), on peut citer les maladies endocrines associées à un excès d'hormone comme l'hormone de croissance, l'insuline ou le glucagon. Les composés de la présente invention sont ainsi indiqués pour traiter des maladies telles l'acromégalie, les adénomes hypophysaires, la maladie de Cushing, les gonadotrophinomes et les prolactinomes, le diabète et ses complications, la rétinopathie diabétique, la néphropathie diabétique, l'hyperthyroïdie, le gigantisme. Sont concernées également les maladies gastrointestinales, les maladies liées à une hypersécrétion gastrique ou pancréatique exocrine ou endocrine, ou encore à la libération de divers peptides du tractus gastrointestinal. Les composés de la présente invention sont ainsi indiqués pour traiter des maladies telles les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, le reflux gastrooesophagien, le reflux gastroduodénal, la pancréatite, les fistules entérocutanées et pancréatiques mais aussi les diarrhées, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, la maladie de Crohn, les scléroses systémiques, le dumping syndrome, le syndrome du petit intestin, l'hypotension, la sclérodermie et le carcinome thyroïdien médullaire. Les composés de la présente invention sont indiqués pour traiter les maladies liées à l'hyperprolifération cellulaire comme les cancers et plus particulièrement le cancer du sein, le cancer de la prostate, le cancer thyroïdien ainsi que le cancer pancréatique et le cancer colorectal, le cancer du cerveau, le cancer du poumon, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie. D'autres domaines thérapeutiques comme les céphalées y compris les céphalées associées aux tumeurs hypophysaires, les douleurs, les altérations psychiques telles l'anxiété, la dépression et la schizophrénie, la chimiothérapie, la cicatrisation des plaies, l'insuffisance rénale résultant d'un retard de croissance, l'obésité et retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis ainsi que les maladies neurodégénératives telles la démence, l'épilepsie ou la maladie d'Alzheimer. On peut également citer l'ostéoporose.

Des dérivés d'imidazole utiles pour traiter les désordres associés à la somatostatine ont été décrits dans l'art antérieur (WO 99/64401, WO 01/09090).

Les déposants ont trouvé que les composés de formule générale décrite ci-après présentaient une affinité et une sélectivité pour les récepteurs de la somatostatine. Comme la somatostatine et ses analogues peptidiques ont souvent une mauvaise biodisponibilité par voie orale et une faible sélectivité (Robinson, C., Drugs of the Future, 1994, 19, 992 ; Reubi, J.C. et al., TIPS, 1995, 16, 110), lesdits composés, agonistes ou antagonistes non-peptidiques de la somatostatine, peuvent être avantageusement utilisés pour traiter les états pathologiques ou les maladies tels que présentés ci-dessus et dans lesquels un (ou plusieurs) des récepteurs de la somatostatine est (sont) impliqué(s). De manière préférentielle, lesdits composés peuvent être utilisés pour le traitement de l'acromégalie, des adénomes hypophysaires, les hypersécrétions gastriques ou pancréatiques, les tumeurs gastroentéropancréatiques les cancers du sein, de la prostate, de la thyroïde, du poumon et les fibroses.

Les composés de l'invention sont également des analogues de l'urotensine II et sont donc particulièrement intéressants pour traiter les états pathologiques ou les maladies dans lesquels l'urotensine II est impliquée.

Différentes formes d'urotensine II (U-II), peptide cyclique séquencé voilà plus de 20 ans, ont été isolées dans plusieurs espèces de poissons et d'amphibiens. Ces peptides montrent une capacité de contraction des muscles lisses ainsi qu'un pouvoir vasoconstricteur importants. Plus récemment, l'urotensine II a été clonée dans différentes espèces de mammifères, dont l'espèce humaine. L'urotensine humaine (hU-II) est un undécapeptide cyclique où se trouve conservée la partie hexapeptidique cyclique présente également chez les autres formes animales de la protéine (P. Grieco et al., Bioorg. Med. Chem. 2002, 10, 3731-3739). Chez l'homme U-II a montré un effet vasoconstricteur important sur les veines et les artères in vitro. De plus, U-II et son récepteur sont présents dans le cerveau de rat, impliquant un rôle possible de neurotransmetteur ou de neuromodulateur dans le système nerveux central (J.J. Maguire, A.P. Davenport Br J. Pharmacol 2002, 579-588).

Les composés de formule générale décrite ci-après, en tant qu'analogues de l'urotensine II, peuvent être utilisés pour traiter des états pathologiques liés à l'hypertension (portale, pulmonaire, rénale, cérébrale), aux désordres cardiovasculaires (hypertrophie cardiaque, l'arythmie cardiaque, angine), aux désordres pulmonaires (asthme), ainsi qu'à l'athérosclérose et au stroke. De plus, U-II et son récepteur étant présents dans le système nerveux central des mammifères, les composés de l'invention peuvent également être utilisés dans le traitement de l'anxiété, du stress, de la schizophrénie, de la dépression et des altérations des fonctions neuromusculaires.

La présente invention a donc pour objet des composés de formule générale 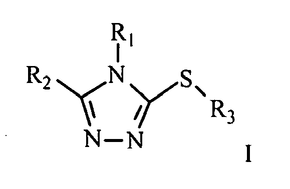 sous forme racémique, d'énantiomère ou toutes combinaisons de ces formes, dans laquelle
R₁ représente un radical de formule -(CH₂)ₙ-[Q]ₚ-(CH₂)ₘ-NXY dans lequel
X et Y représentent, indépendamment, l'atome d'hydrogène ou un (C₁-C₆)alkyle;
p et n représentent 0, et m représente un entier de 2 à 6.
et les deux autres radicaux R₂ et R₃ représentent, indépendamment, un radical de formule -(CH₂)_{n'}[Q']_{p'}[C(X')(Y')]_{m'}Z' dans laquelle
Q' représente -O-, -S-, -C(O)-, -NH-, -CH=CH- ou -C≡C- ;
X', Y' et Z' représentent, indépendamment, un atome d'hydrogène, (C₁-C₆)alkyle, (C₁-C₆)alkoxy, (C₁-C₆)alkoxy-carbonyle, cyano, amino, (C₁-C₆)alkylamino, di((C₁-C₆)alkyl)amino, (C₃-C₇)cycloalkyle, hétérocycloalkyle, aryle ou hétéroaryle, ou bien un radical de formule 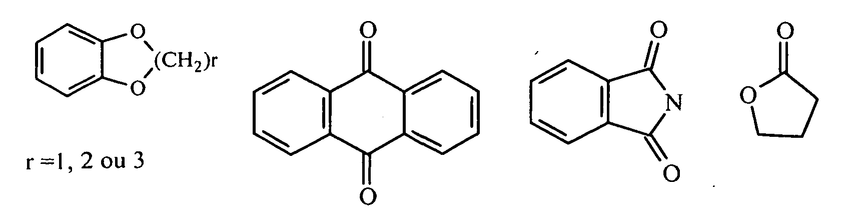 les radicaux (C₃-C₇)cycloalkyle, hétérocycloalkyle, aryle et hétéroaryle étant éventuellement substitués par un ou plusieurs substituants identiques ou différents choisis parmi : -(CH₂)_{q'}-X"-Y", hydroxy, halo, nitro, cyano, amino, (C₁-C₆)alkylamino et di((C₁-C₆)alkyl)amino;
X" représente -O-, -S-, -C(O)-, -C(O)-O-, -SO₂- ou une liaison covalente:
Y" représente un radical (C₁-C₆)alkyle éventuellement substitué par un ou plusieurs radicaux halo identiques ou différents ; ou aryle ou hétéroaryle éventuellement substitué par un ou plusieurs substituants identiques ou différents choisis parmi : (C₁-C₆)alkoxy, hydroxy, halo, nitro cyano, amino, (C₁-C₆)alkylamino et di((C₁-C₆)alkyl)amino;
p' représente 0 ou 1, et n', m' et q' représentent, indépendamment, un entier de 0 à 6;
ou leurs sels d'addition avec les acides minéraux ou organiques pharmaceutiquement acceptables,
à l'exclusion des composés pour lesquels :
- R₃ représente un atome d'hydrogène, R₂ le radical méthyle et R₁ le radical 2-(diméthylamino)éthyle;
- R₃ et R₂ représentent un atome d'hydrogène, et R₁ le radical 2-(diméthylamino)éthyle, 3-(diméthylamino)propyle, 6-(diméthylamino)hexyle, 2-(dipropylamino)éthyle ou 2-(diéthylarmino)éthyle ;
- R₃ représente un atome d'hydrogène, R₂ le radical amino et R₁ le radical 2-(diméthylamino)éthyle,
- R₃ représente un atome d'hydrogène, R₂ le radical 2-hydroxyéthyle et R₁ le radical 2-(diéthylamino)éthyle.

Dans les définitions indiquées ci-dessus, l'expression halo représente le radical fluoro, chloro, bromo ou iodo, de préférence chloro, fluoro ou bromo. L'expression alkyle (lorsqu'il n'est pas donné plus de précision), représente de préférence un radical alkyle ayant de 1 à 6 atomes de carbone, linéaire ou ramifié, tels que les radicaux méthyle, éthyle, propyle, isopropyle, butyle, isobutyle, *sec*-butyle et *tert*-butyle, pentyle ou amyle, isopentyle, neopentyle, hexyle ou isohexyle. Par ailleurs, dans la présente demande, le radical -(CH₂)_{n'}- représenté une chaîne hydrocarbonée de n' atomes de carbone qui peut être linéaire ou ramifiée ; ce radical -(CH₂)_{n'}- peut ainsi représenter les radicaux alkyl tels que définis ci-dessus.

Le terme (C₃-C₇)cycloalkyle désigne un système monocyclique carboné comprenant de 3 à 7 atomes de carbone, et de préférence les cycles cyclopropyle, cyclobutyle, cyclopentyle ou cyclohexyle. L'expression hétérocycloalkyle désigne un cycloalkyle saturé contenant de 2 à 7 atomes de carbone et au moins un hétéroatome. Ce radical peut contenir plusieurs hétéroatomes identiques ou différents. De préférence, les hétéroatomes sont choisis parmi l'oxygène, le soufre ou l'azote. Comme exemples d'hétérocycloalkyle, on peut citer les cycles contenant au moins un atome d'azote tels que pyrrolidine, pyrrolidinone, imidazolidine, pyrrazolidine, isothiazolidine, thiazolidine, isoxazolidine, pipéridine, pipérazine ou morpholine, ou bien tétrahydrofurane ou tétrahydrothiophène.

Les radicaux alkoxy peuvent correspondre aux radicaux alkyle indiqués ci-dessus comme par exemple les radicaux méthoxy, éthoxy, propyloxy ou isopropyloxy mais également butoxy linéaire, secondaire ou tertiaire, pentyloxy. Le terme alkoxycarbonyle désigne de préférence les radicaux dans lesquels le radical alkoxy est tel que défini ci-dessus comme par exemple méthoxycarbonyle, éthoxycarbonyle.

L'expression aryle représente un radical aromatique, constitué d'un cycle ou de cycles condensés, comme par exemple le radical phényle, naphtyle ou fluorényle. L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux thiényle, furyle, pyrrolyle, imidazolyle, pyrazolyle, isothiazolyle, thiazolyle, isoxazolyle, oxazolyle, triazolyle, thiadiazolyle, pyridyle, pyrazyle, pyrimidyle, quinolyle, isoquinolyle, quinoxalyle, naphthyridyle, xanthényle, benzothiényle, benzofuryle, indolyle, benzoxadiazoyle. Les termes aralkyle (arylalkyle), cycloalkyl-alkyle et hétéroaryl-alkyle désignent de préférence les radicaux dans lesquels le radical aryle, cycloalkyle et hétéroaryle respectivement, et alkyle sont tels que définis ci-dessus ; comme exemple de arylalkyle, on peut citer benzyle et phenéthyle.

Les termes alkylamino et dialkylamino désignent de préférence les radicaux dans lesquels les radicaux alkyle sont tels que définis ci-dessus, comme par exemple méthylamino, éthylamino, diméthylamino, diéthylamino ou (méthyl)(éthyl)amino.

De préférence, l'invention concerne des composés de formule I telle que définie ci-dessus et dans laquelle
R₂ représente un radical de formule -(CH₂)_{n'}[Q']_{p'}[C(X')(Y')_{m'}Z'
Q' représente -O- ;
X' représente l'atome d'hydrogène ;
Y' et Z' représentent, indépendamment, un atome d'hydrogène, (C₁-C₆)alkyle, cyano, amino, (C₃-C₇)cycloalkyle, aryle ou hétéroaryle ;
les radicaux aryle et hétéroaryle étant éventuellement substitués par un ou plusieurs substituants identiques ou différents choisis parmi : -(CH₂)_{q'}-X"-Y", hydroxy, halo, nitro, amino, (C₁-C₆)alkylamino, di((C₁-C₆)alkyl)amino ;
X" représente -O-, -S- ou une liaison covalente ;
Y" représente un radical (C₁-C₆)alkyle éventuellement substitué par un ou plusieurs radicaux halo identiques ou différents, ou aryle éventuellement substitué par un ou plusieurs radicaux halo identiques ou différents ;
p' représente 0 ou 1 ; n' représente 0, 1 ou 2 ; et m' représente un entier de 0 à 6 ;

De manière très préférentielle, R₂ représente un radical aryle ou hétéroaryle éventuellement substitué et plus particulièrement naphtyte, phényle, benzothiényle, quinoxalyle, quinolyle, isoquinolyle ou indolyle ; les radicaux phényle, e naphtyle et quinolyle étant éventuellement substitués par un ou plusieurs radicaux (C₁-C₆)alkoxy, halo, nitro, hydroxy, (C₁-C₆) alkyle identiques ou différents, le (C₁-C₆) alkyle étant lui même éventuellement substitué par un ou plusieurs radicaux halo identiques ou différents.

De manière très préférentielle, R₃ représente un radical de formule -(CH₂)_{n'}[Q']_{p'}[C(X')(Y')]_{m'}Z' dans laquelle
X' et Y' représentent l'atome d'hydrogène;
Z' représente indolyle ou benzothiényle, le radical indolyle étant éventuellement substitué par un ou plusieurs substituants identiques ou différents choisis parmi : -(CH₂)_{q'}-X"-Y", (C₁-C₆)alkoxy ou halo, X" représente -SO₂- ou une liaison covalente ;
Y" représente phényle ou alkyle éventuellement substitué par un ou plusieurs radicaux halo identiques ou différents ;
q' représente 0 ou 1 ; p' représente 0 ; n' représente 0 ou 1 ; et m' représente 0 ou 1.

La présente invention a plus particulièrement pour objet également des composés de formule générale I telle que définie ci-dessus dans laquelle

R₁ représente un radical de formule -(CH₂)ₙ-[Q]ₚ-(CH₂)ₘ-NXY dans lequel
X et Y représentent, indépendamment, l'atome d'hydrogène ou un (C₁-C₆)alkyle ;
p et n représentent 0 , et m représente un entier de 2 à 6.

R₂ représente quinoxalyle, quinolyle ou naphtyl, les radicaux quinolyle et naphtyle étant éventuellement substitués par un ou plusieurs radicaux (C₁-C₆)alkyl, (C₁-C₆)alkoxy, halo, identiques ou différents ;

R₃ représente un radical de formule -(CH₂)_{n'}[Q']_{p'}[C(X')(Y')]_{m'}Z' dans laquelle
X' et Y' représentent l'atome d'hydrogène;
Z' représente indolyle éventuellement substitués par un ou plusieurs substituants identiques ou différents choisis parmi : -(CH₂)_{q'}-X"-Y", (C₁-C₆)alkoxy ou halo ;
X" représente une liaison covalente;
Y" représente un radical alkyl éventuellement substitué par un ou plusieurs radicaux halo identiques ou différents ;
q' représente 0 ou 1 ; p' représente 0 ; n' représente 0 ou 1 ; et m' représente 0 ou 1

Dans la présente demande, le symbole -> * correspond au point de rattachement du radical. Lorsque le site de rattachement n'est pas précisé sur le radical, cela signifie que le rattachement s'effectue sur un des sites disponibles de ce radical pour un tel rattachement.

Les composés selon l'invention peuvent être préparés en phase liquide selon le schéma général suivant :

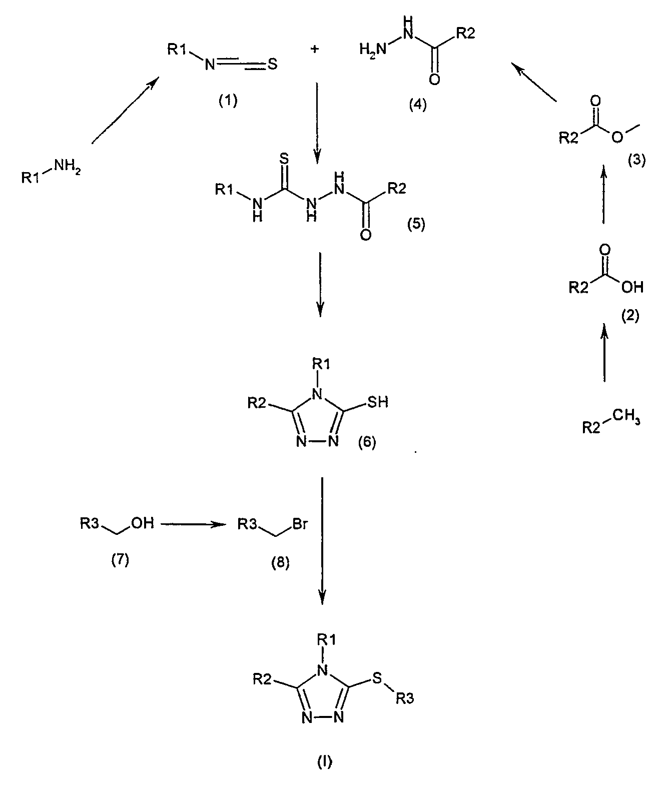

### 1. Préparation d'isothiocyanates (1) :

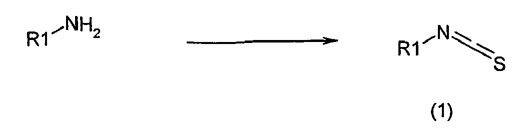

Les isothiocyanates de formule générale (1) peuvent être préparés à partir des amines primaires correspondantes par deux méthodes :

### 1.1 Méthode A :

Une amine primaire est convertie en isothiocyanate par action de O,O-di(2-pyridinyl) thiocarbonate (1 éq.) dans des solvants aprotiques anhydres tel que le dichlorométhane, le tétrahydrofuranne ou le diméthylformamide (Kim, S. ; Lee, J.I. Tetrahedron Lett. 1985, 26 (13), 1661-1664). Le mélange réactionnel est agité à température ambiante pendant 1 à 4 heures puis les solvants sont évaporés et le résidu utilisé dans l'étape suivante sans autre purification.

### Préparation 1 : tert-butyl 4-isothiocyanatobutylcarbamate (C₁₀H₁₈N₂O₂S, M = 230,33)

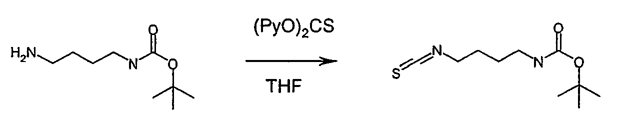

A du O,O-di(2-pyridinyl) thiocarbonate (3,9 g ; 17 mmol) dissous dans du tétrahydrofuranne est ajouté du *tert*-butyl 4-aminobutylcarbamate (3,2 ml ; 17 mmol). La solution est agitée pendant 2 heures à température ambiante. Le solvant est évaporé et le solide obtenu est utilisé sans délais dans l'étape suivante.

Des isothiocyanates de formule générale R₁NCS ont étés synthétisés selon ce mode opératoire avec les groupements R₁ suivants dont les amines primaires et secondaires sont protégées par un groupement *tert*-butoxycarbonyle :

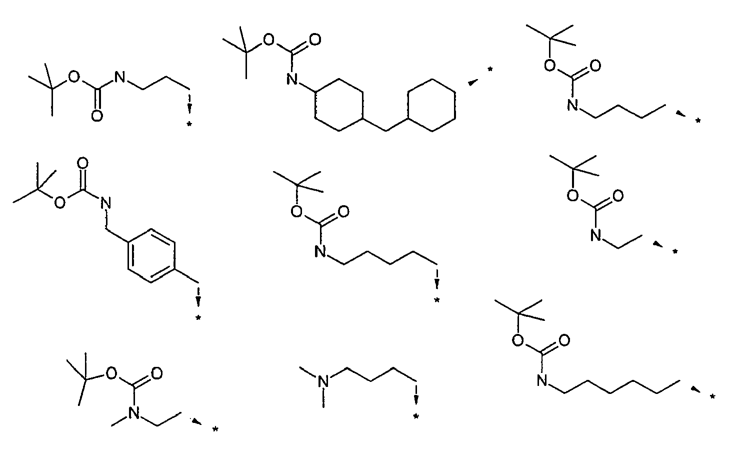

### 1.2 Méthode B :

Une amine primaire est convertie en isothiocyanate par action de dithioxométhane (10 éq.) en présence de résine N-cyclohexylcarbodiimide, N-méthylpolystyrène (Novabiochem ; charge supérieure à 1,5 mmol/g, 1,1 éq.) préalablement gonflée dans un solvant aprotique tel que le dichlorométhane ou le tétrahydrofuranne. Le mélange réactionnel est agité à température ambiante pendant 1 à 4 heures puis le filtrat est évaporé et utilisé dans l'étape suivante sans autre purification.

### Préparation 2 : N,N-diméthyl-4-isothiocyanatobutylamine (C₇H₁₄N₂S, M = 158,27)

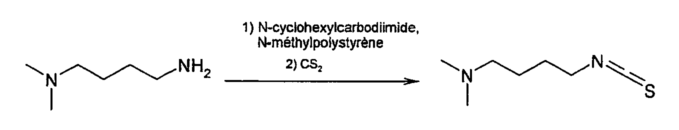

A la résine N-cyclohexylcarbodiimide, N-méthylpolystyrène (1 g ; 1,69 mmol/g ; Novabiochem) dans du dicholorométhane (15 ml), est ajouté du dithioxométhane (1 ml; 16,6 mmol). La suspension est agitée pendant 30 minutes puis la N,N-diméthyl-1,4-butanediamine est ajoutée (0,19 ml ; 1,5 mmol). Le mélange réactionnel est agité pendant 3 heures, puis filtré. Le filtrat est évaporé et utilisé immédiatement dans l'étape suivante.

Des isothiocyanates de formule générale R₁NCS ont été synthétisés selon cette méthode avec les groupements R₁ suivants dont les amines primaires sont protégées par un groupement *tert*-butoxycarbonyle :

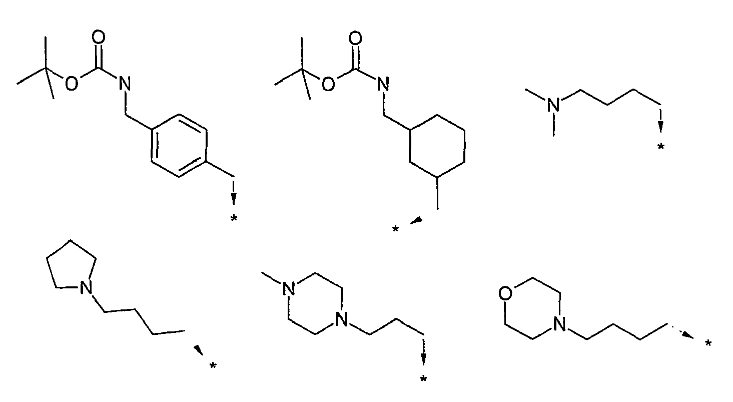

### 2. Préparation d'hydrazides (4) :

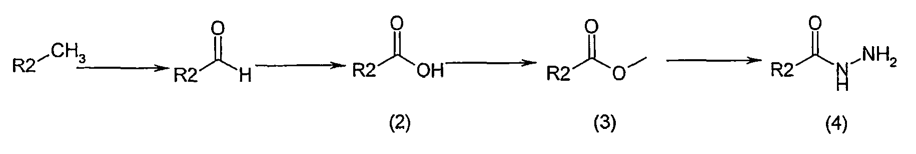

### 2.1 Préparation des acides carboxyliques (2) :

Quand ils ne sont pas commerciaux, les acides carboxyliques de formule générale (2), dans lesquels R2 est un groupement de type aryle ou hétéroaryle, peuvent être préparés à partir du dérivé méthylé correspondant par oxydation en aldéhyde, par exemple par du dioxyde de sélénium, suivie d'une seconde oxydation en acide carboxylique, par utilisation, par exemple de chlorite de sodium (Bu, X.; Deady, L. W. ; Finlay, G. J. ; Baguley, B. C. ; Denny, W. A. J. Med. Chem. 2001, 44, 2004-2014).

### Préparation 3 : acide 6-chloroquinoline-2-carboxylique (C₁₀H₆ClNO₂, M = 207,62)

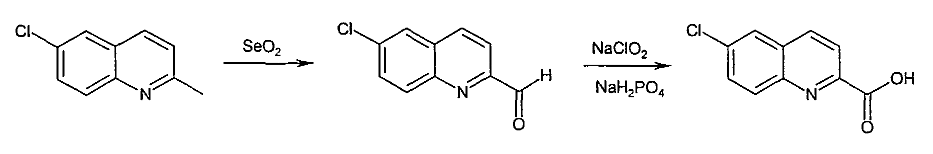

A une suspension de dioxyde de sélénium (1,87 g ; 16,9 mmol ; 6 éq.) dans le dioxane (25 ml) à 80°C, est ajoutée la 6-chloro-2-méthylquinoline (500 mg ; 2,8 mmol). Le mélange réactionnel est agité pendant 3 heures au reflux puis l'insoluble est filtré à chaud. Le dioxane est ensuite évaporé sous pression réduite et l'aldehyde obtenu est utilisé sans purification dans l'étape suivante.

RMN ¹H (DMSO-d₆, 400 MHz) δ : 10,09 (s, 1H, CHO) ; 8,57-8,54 (m, 1H, H arom.) ; 8,27-8,21 (m, 2H, H arom.) ; 8,02-8,00 (m, 1H, H arom.); 7,91-7,88 (m, 1H, H arom.).

Une solution de chlorite de sodium (2,4 g) et de dihydrogénophosphate de sodium (2,4 g) dans l'eau (24 ml) est ajoutée, sur une durée de 5 minutes, à une solution de 6-chloroquinoline-2-carbaldehyde (536 mg; 2,8 mmol) dans l'alcool ter-butylique (56 ml) et le 2-méthylbut-2-ène (14 ml). Le mélange obtenu est agité pendant 4 heures à température ambiante. Les solvants organiques sont évaporés sous pression réduite et de l'eau (30 ml) est ajoutée au résidu. Le précipité obtenu est filtré lavé à l'eau et séché sous vide en présence de P₂O₅. L'acide 6-chloroquinoline-2-carboxylique est obtenu sous forme de poudre blanche (505 mg ; rendement = 87 %). SM/CL : m/z = 208,01 (M + H) tr = 8,55 min (condition 1).

RMN ¹H (DMSO-d₆, 400 MHz) δ : 8,41-8,39 (m, 1H, H arom.); 8,20-8,11 (m, 3H, H arom.) ; 7,82-7,79 (m, 1H, H arom.).

Des acides carboxyliques de formule générale R₂COOH ont été synthétisés selon cette méthode avec les groupements R₂ suivants :

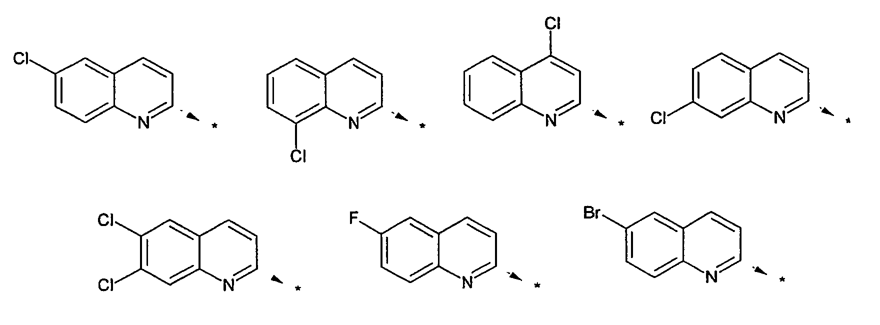

### 2.2 Préparation des esters méthyliques (3) :

Un acide carboxylique est tout d'abord transformé en ester méthylique par exemple par action d'un excès de diazométhane ou d'un substitut de diazométhane tel que le triméthylsilyldiazométhane, dans le méthanol, en présence ou non d'un solvant aprotique tel que le diéthyl éther ou le dichlorométhane (Caturla, F. ; Najera, C. ; Varea, M. Tetrahedron Lett. 1999, 40 (32), 5957-5960). L'excès de diazométhane est neutralisé par l'ajout d'un acide carboxylique tel que par exemple l'acide acétique.

Les esters méthyliques sont isolés après extraction et lavage et utilisés sans autre purification dans l'étape suivante.

### Réparation 4 : 4-fluoro-1-naphtoate de méthyle (C₁₂H₉FO₂, M = 204,20)

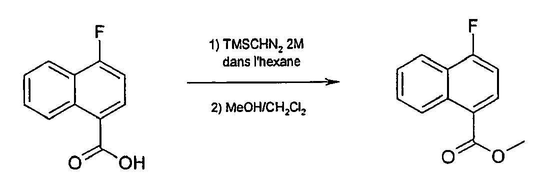

A de l'acide 4-fluoro-1-naphtoïque (1g ; 5,3 mmol) dissous dans un mélange de dichlorométhane (10 ml) et de méthanol (15 ml) est ajoutée une solution de (triméthylsilyl)diazométhane en solution dans de l'hexane (6 ml, 2 mol/l) jusqu'à ce que la solution garde une légère coloration jaune et ne dégaze plus. L'excès de (triméthylsilyl)diazométhane est neutralisé par l'ajout de quelques gouttes d'acide acétique jusqu'à ce que la solution soit incolore. Le mélange réactionnel est évaporé, puis solubilisé dans de l'acétate d'éthyle (20 ml) et lavé avec de l'eau distillée (10 ml), puis une solution saturée de chlorure de sodium (10 ml). La phase organique est séchée sur du sulfate de sodium, puis évaporée et séchée sous vide pour donner une poudre blanche (0,78 g ; rendement = 73 %). SM/CL : m/z = 205,23 (M + H) tr = 11,21 min (condition 1).

Des esters de méthyle de formule R₂COOMe ont été synthétisés avec les groupements R₂ suivants dont les amines primaires et secondaires sont protégées par un groupement *tert*-butoxycarbonyle :

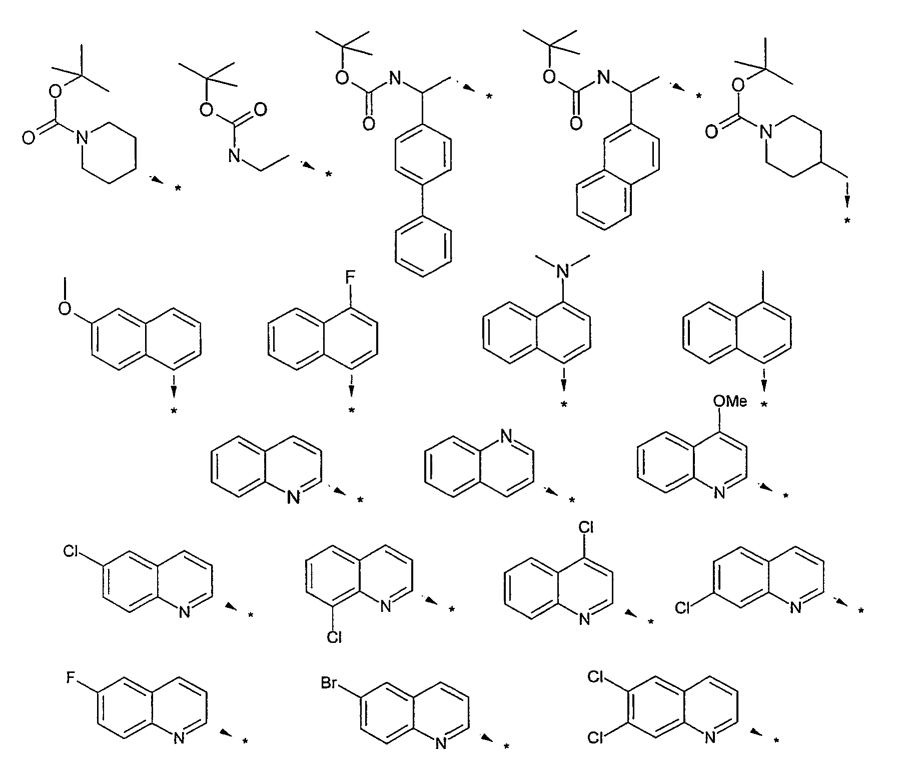

### 2.3 Préparation des hydrazides (4) :

Les hydrazides de formule générale (4) peuvent être obtenus par action d'hydrate d'hydrazine (3 à 10 éq.) sur les esters de formule générale (3) dans un solvant polaire protique tel que l'éthanol ou le méthanol (Leung, H. K. ; Phillips, B. A. ; Cromwell, N. H., J. Heterocycl. Chem. 1976, 13, 247-252). La réaction est maintenue de 18 à 96 heures à température ambiante ou à 50° C. Après évaporation, le milieu réactionnel est repris par un solvant tel que l'acétate d'éthyle et lavé par de l'eau. Les hydrazides sont obtenus après évaporation des phases organiques et concrétisation.

Préparation 5 : 2,2-diphénylacétohydrazide (C₁₄H₁₄N₂O, M = 226,28)

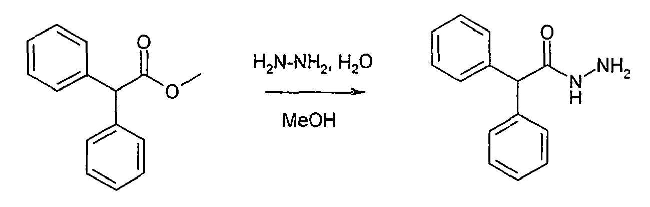

A du diphénylacétate de méthyle (1,19 g; 5 mmol) solubilisé dans du méthanol (15 ml) est ajouté l'hydrate d'hydrazine (7 ml ; 50 mmol). Le mélange réactionnel est agité à température ambiante pendant 60 heures puis le solvant est évaporé. Le résidu est solubilisé dans de l'acétate d'éthyle (20 ml) et lavé avec de l'eau distillée (15 ml) puis une solution aqueuse saturée en chlorure de sodium (15 ml). La phase organique est séchée sur sulfate de magnésium, puis évaporée et séchée sous vide pour donner une poudre blanche (0,94 g; rendement = 83 %).

RMN ¹H (DMSO-d₆, 400 MHz) δ : 9,44 (s large, 1H, NH) ; 7,33-7,20 (m, 10H, arom.) ; 4,82 (s, 1H, CH) ; 4,30 (s large, 2H, NH₂). SM/CL : m/z = 227,30 (M + H) tr = 10,19 min (condition 1).

Des hydrazides de formule R₂CONHNH₂ ont été préparés avec les groupements R₂ suivants dont les amines primaires et secondaires sont protégées par un groupement *tert*-butoxycarbonyle :

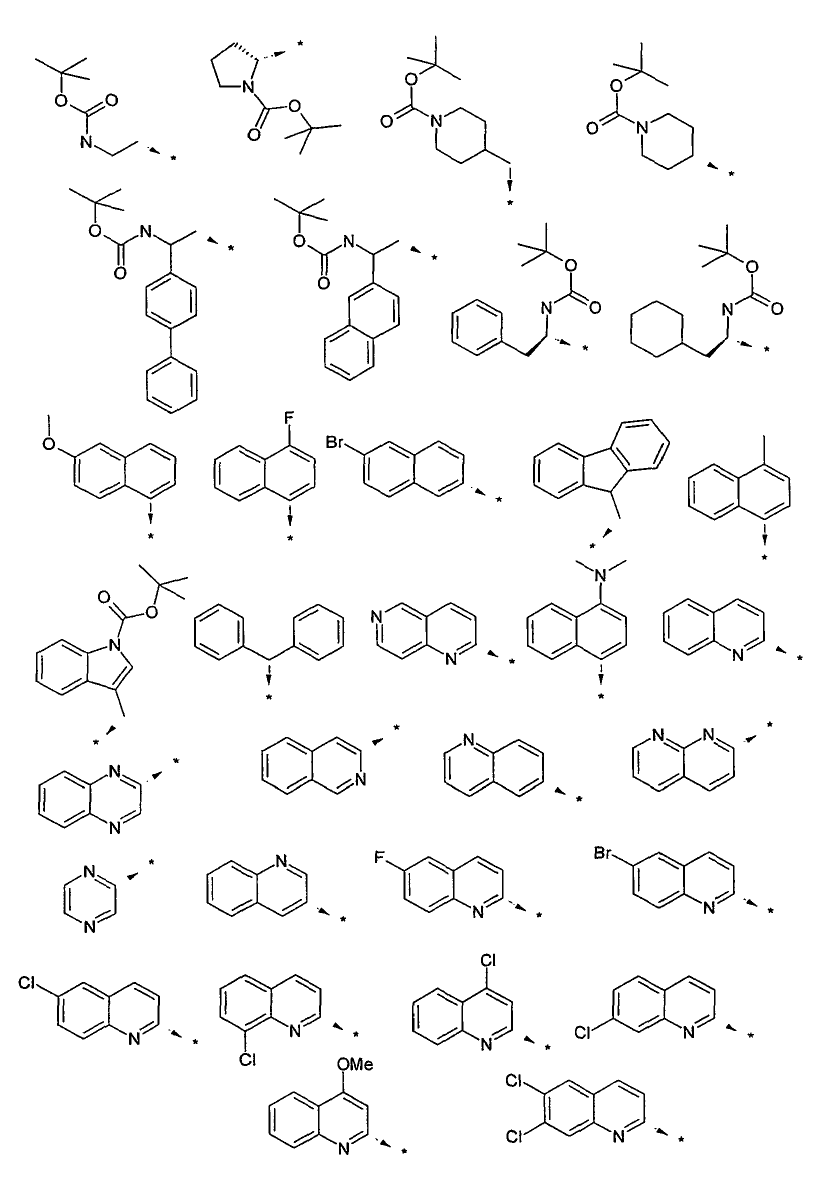

### 3. Préparation des hydrazinecarbothioamides (5) :

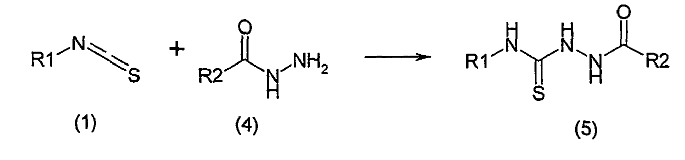

Les isothiocyanates de formule générale (1) (1,1 éq.) sont ajoutés aux hydrazides de formule générale (4) dans un solvant aprotique tel que le dichlorométhane ou le diméthylformamide et le milieu réactionnel est agité à température ambiante pendant 18 à 24 heures. Les hydrazinecarbothioamides (5) sont obtenus après filtration ou évaporation du milieu réactionnel et utilisés dans l'étape suivante sans autre purification.

### Préparation 6 : N-phényl-2-(phénylacétyl)hydrazinecarbothioamide (C₁₅H₁₅N₃OS, M = 285,37)

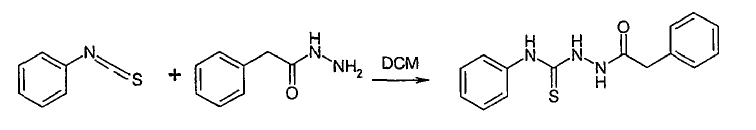

A du 2-phénylacétohydrazide (1,5 g ; 10 mmol) solubilisé dans le dichlorométhane (20 ml), est ajouté le phénylisothiocyanate (1,3 ml ; 11 mmol). La solution est agitée à température ambiante jusqu'à la précipitation du produit. Le solide blanc formé est filtré et lavé avec de l'éther éthylique (10 ml), séché sous vide (2,1 g ; rendement = 74 %).

RMN ¹H (DMSO-d₆, 400 MHz) δ : 10,15 (s large, 1H, NH) ; 9,59 (s large, 2H, NH₂); 7,44-7,42 (m, 2H, arom.) ; 7,35-7,28 (m, 5H, arom.) ; 7,25-7,22 (m, 2H, arom.) ; 7,18-7,14 (m, 1H, arom.) ; 3,52 (s, 2H, CH₂). SM/CL : m /z = 286,26 (M + H) tr = 8,13 min (condition 1).

Les hydrazinecarbothioamides de formule générale (5) ont été synthétisés pour la préparation des composés de l'invention avec les groupes R₁ et R₂ suivants dont les amines primaires et secondaires sont protégées par un groupement *tert*-butoxycarbonyle :

R₁ :

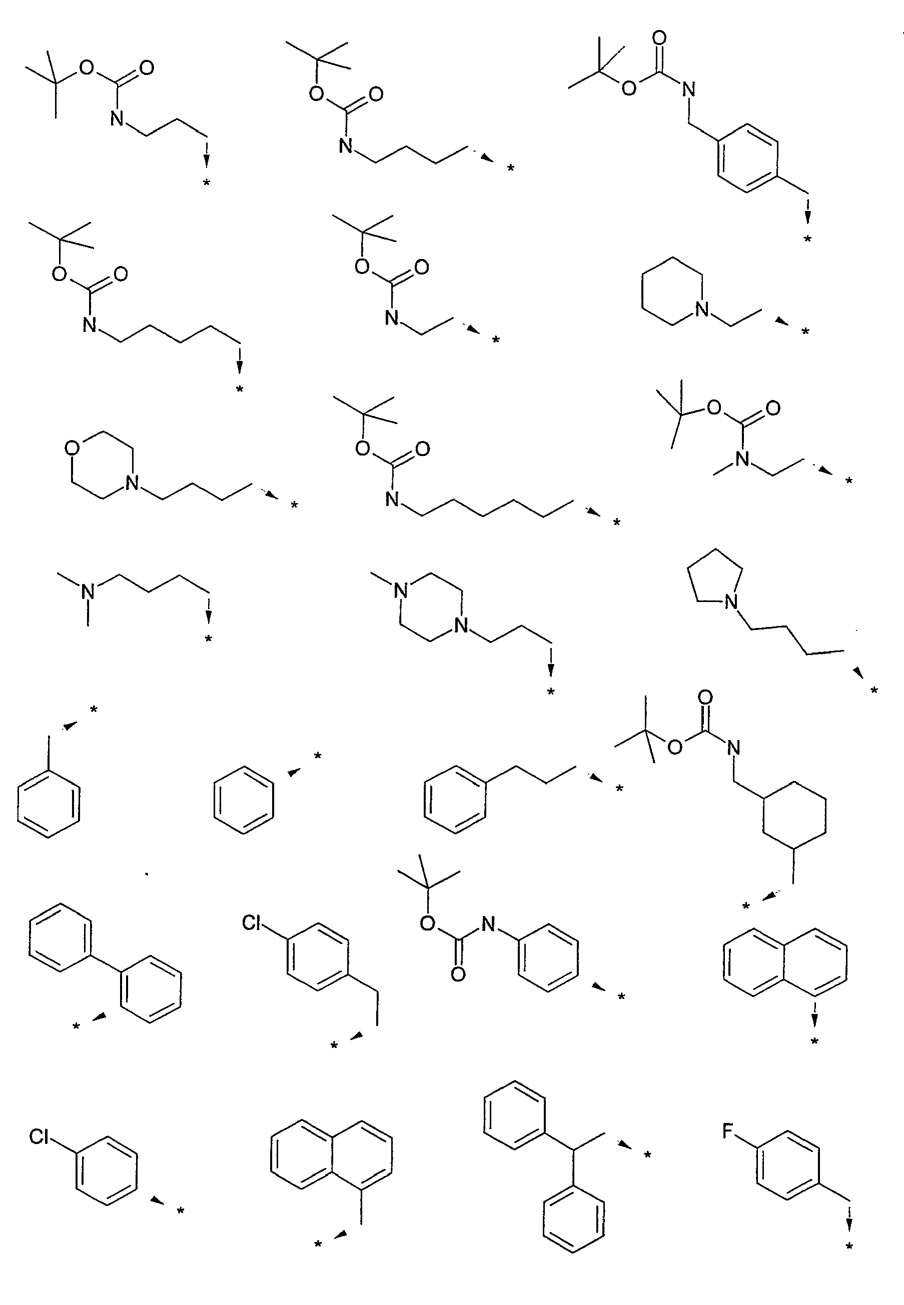

R₂ :

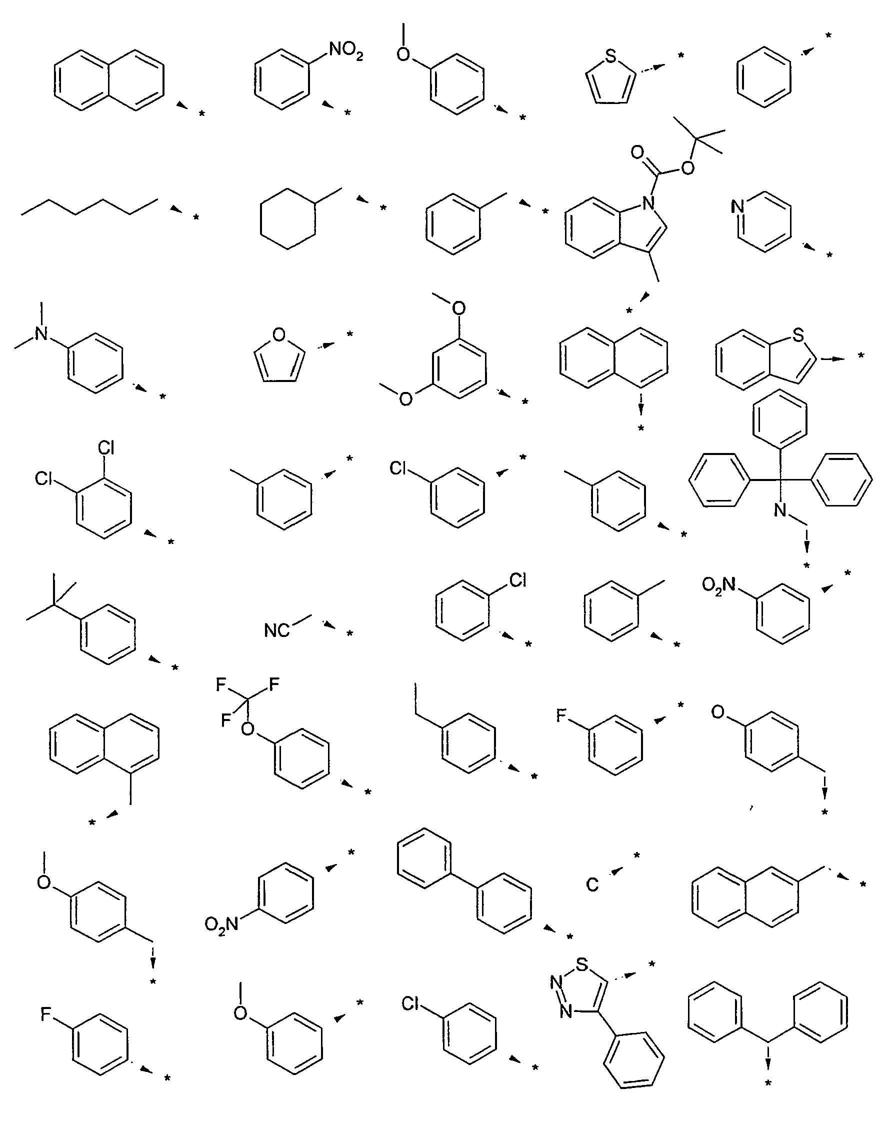

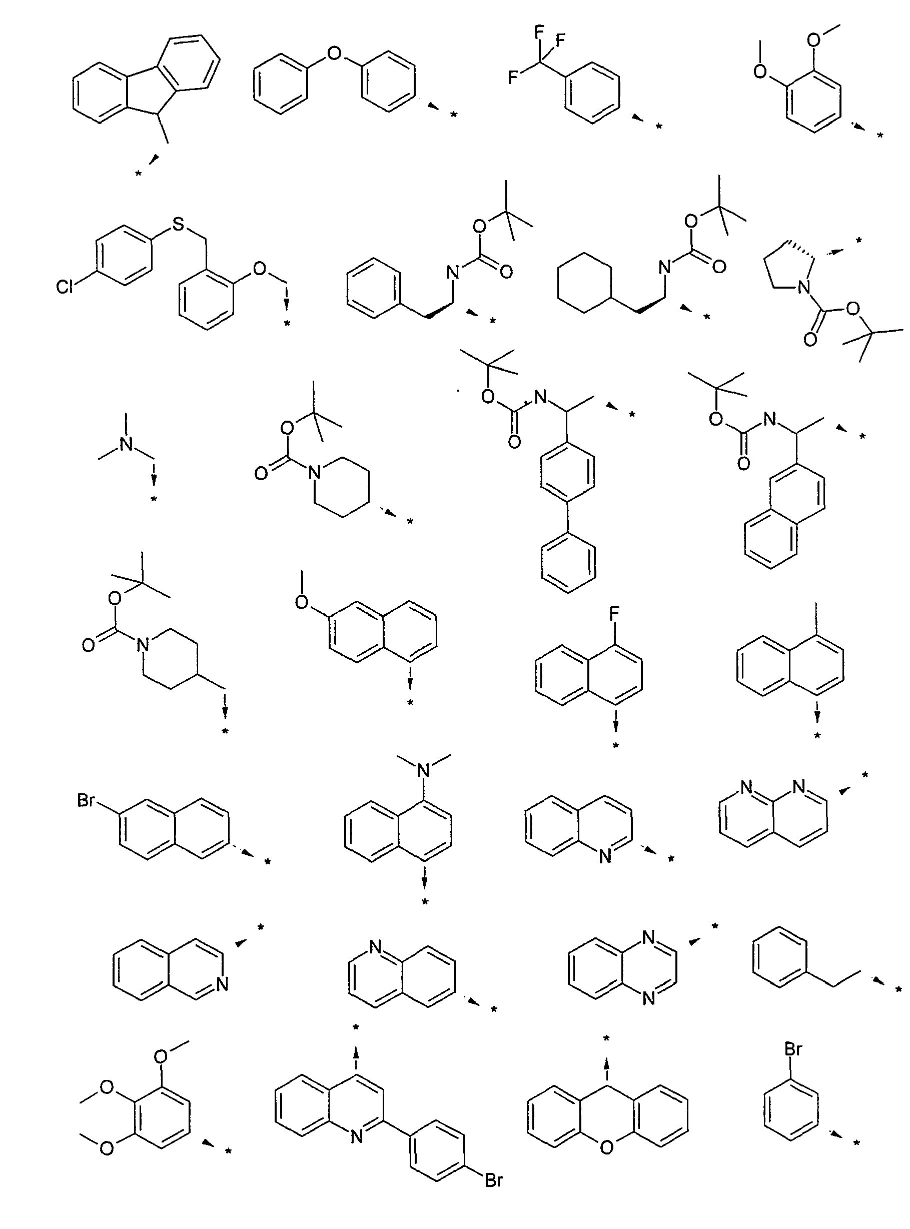

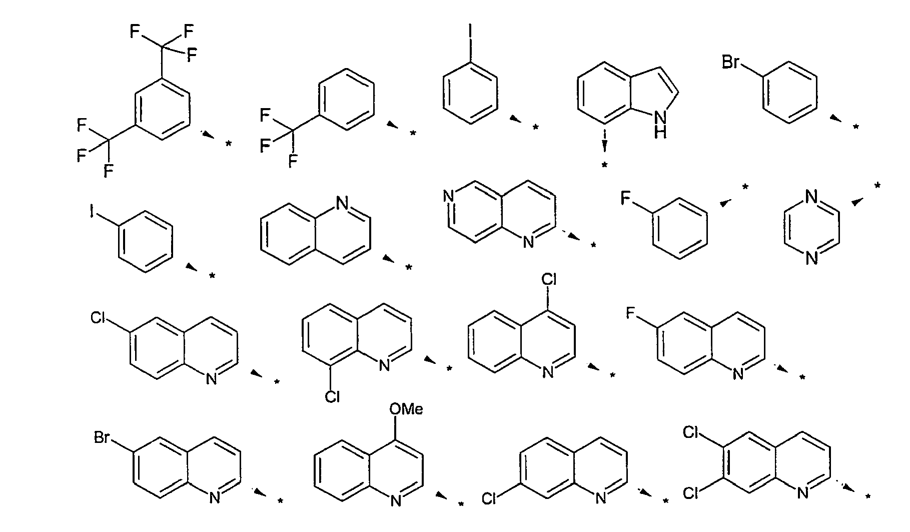

### 4. Préparation des triazoles (6) :

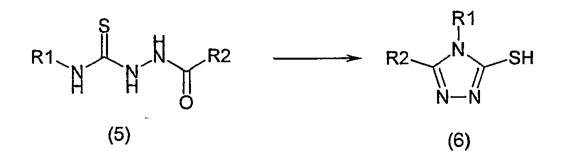

Après dissolution de l'hydrazinethiocarbamide de formule générale (5) dans un solvant de type dioxanne ou toluène, l'étape de cyclisation a lieu dans un solvant protique tel que l'éthanol ou le méthanol en présence d'une solution de soude (1M à 4M) ou de potasse (1M à 4M). La réaction est maintenue à 85° C pendant une durée variant de 4 heures à 18 heures puis après évaporation des solvants, le thiolate obtenu est transformé en thiol (6) par exemple à l'aide d'une résine échangeuse d'ion telle que la résine Amberlite IRN 77 (cation H+) (Prolabo). La résine est filtrée et le filtrat concentré. Une purification sur colonne de silice peut être réalisée.

### Préparation 7 : 5-benzyl-4-phényl-4H-1,2,4-triazole-3-thiol (C₁₅H₁₃N₃S, M = 267,35)

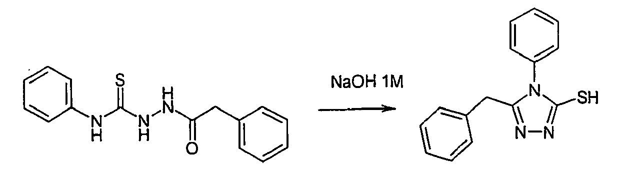

Au N-phényl-2-(phénylacétyl)hydrazinecarbothioamide (3,7 g ; 13 mmol) dissous dans un mélange de dioxanne (30 ml) et de méthanol (10 ml) est ajoutée une solution aqueuse normale d'hydroxyde de sodium (20 ml). La solution est agitée et chauffée à 85° C pendant 4 heures. Les solvants sont évaporés et le résidu est solubilisé dans du méthanol (25 ml). Une résine échangeuse d'ions préalablement rincée au méthanol (Amberlite IRN 77, 50 g, Prolabo) est ajoutée à la solution, agitée pendant 15 minutes puis filtrée. Le filtrat est évaporé puis séché sous vide (3,4 g; rendement = 98 %).

RMN ¹H (DMSO-d₆, 400 MHz) δ : 13,78 (s large, 1H, SH) ; 7,48-7,46 (m, 3H, arom.) ; 7,23-7,17 (m, 5H, arom.) ; 6,92-6,90 (m, 2H, arom.) ; 3,85 (s, 2H, CH₂). SM/CL : m/z = 268,23 (M + H) tr = 5,72 min (condition 2).

Les triazoles de formule générale (6) ont été préparés avec les mêmes groupements R₁ et R₂ que ceux décrits pour la préparation des hydrazinecarbothioamides (5) ci-dessus.

### 5. Préparation des intermédiaires bromés (8) :

### 5.1 Préparation des bromures benzyliques (8a) :

### 5.1.1 Cas général :

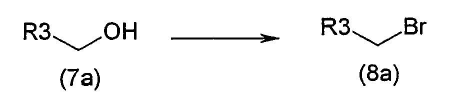

Les bromures benzyliques de formule générale (8a) peuvent être obtenus à partir des alcools correspondants (7a) selon les procédures décrites dans la littérature, par exemple par traitement par de l'acide bromhydrique aqueux au reflux (Kinoshita, T. ; Okunaka, T. ; Ohwada, H. ; Furukawa, S. J. Heterocycl. Chem. 1991, 28 (8), 1901-1909) ou par un halogénure d'acide inorganique tel que PBr₃ ou SOBr₂ (Nagle, A. S. ; Salvatore, R. N. ; Chong, B.-D. ; Jung, K. W. Tetrahedron Lett 2000, 41 (17), 3011-3014) ou encore par un mélange de N-bromosuccinimide ou de CBr₄ et de triphénylphosphine dans un solvant aprotique tel que le tétrahydrofuranne ou le dichlorométhane (Amici, R. ; Pevarello, P. ; Colombo, M. ; Varasi, M. Synthesis 1996, (10), 1177-1179, Campbell, J.A. ; Rapoport, H. J. Org. Chem. 1996, 61 (18), 6313-6325).

### Préparation 8 : 5-(bromométhyl)-1,3-benzodioxole (C₈H₇BrO₂, M = 215,05)

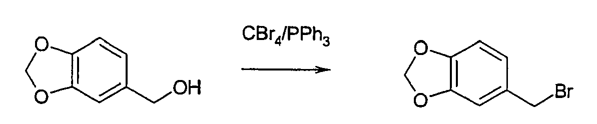

Au 5-(méthanol)-1,3-benzodioxole (1,5 g ; 10 mmol) dissous dans du dicholorométhane (30 ml), est ajouté du tétrabromure de carbone (3,8 g ; 11,5 mmol) ; le mélange est refroidi à 0° C. La triphénylphosphine (3,0 g; 11,5 mmol) est ajoutée par partie, la solution est agitée pendant deux heures à température ambiante. Le solvant est évaporé et le solide obtenu est purifié par chromatographie sur colonne de silice (éluant : heptane / acétate d'éthyle : 3 / 1). Les fractions sont évaporées et le solide obtenu est séché sous vide (2,1 g ; rendement = 97 %).

RMN ¹H (DMSO-d₆, 400 MHz) δ : 7,02-6,98 (m, 1H, arom.) ; 6,96-6,93 (m, 1H, arom) ; 6,88-6,86 (m, 1H, arom) ; 6,02 (s, 2H, CH₂) ; 4,66 (s, 2H, CH₂).

Un bromure benzylique de formule R₃Br a été synthétisé avec le groupement R₃ suivant :

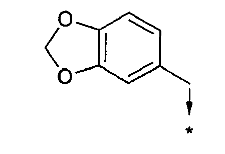

### 5.1.2 Cas particulier des bromures méthylindoliques (8a) :

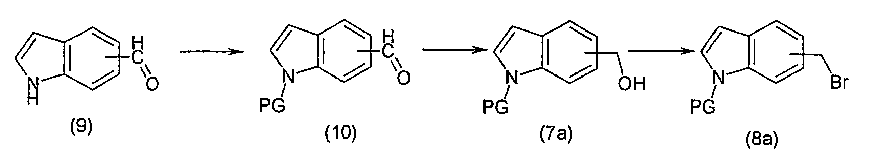

Dans le cas où les intermédiaires bromés de formule générale (8a) sont de type méthylindolique, ils peuvent être obtenus en 3 étapes à partir des indole-carbaldéhydes correspondants, tout d'abord par protection de l'indole, puis par réduction de la fonction aldéhyde suivie finalement d'une bromation de la fonction alcool ainsi obtenue.

### 5.1.2.1 Protection de l'indole :

Un groupement protecteur adéquat tel que, par exemple, un groupement de type carbamate (par exemple, le groupement *tert*-butoxycarbonyle) est introduit sur l'indole par les méthodes classiques connues de l'homme de l'art (P. J. Kocienski, Protecting Groups, 192 (Georg Thiem Verlag Stuttgart, 1994)), par exemple à l'aide de di-*tert-*butyl dicarbonate dans l'acétonitrile ou le diméthylformamide à température ambiante en présence d'un catalyseur tel que la diméthyaminopyridine.

### Préparation 9 : tert-butyl 6-formyl-1H-indole-1-carboxylate (C₁₄H₁₅NO₃, M=245,28)

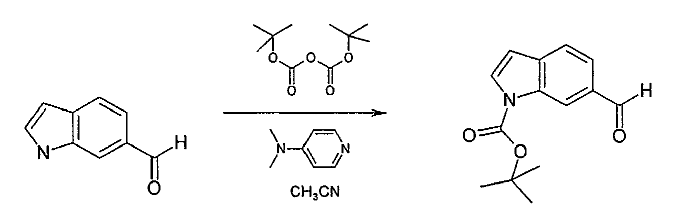

Au 1*H*-indole-6-carbaldéhyde (0,5 g; 3,44 mmol) dissous dans l'acétonitrile (15 ml) sont ajoutés le di-*tert*-butyl dicarbonate (0,827g; 3,8 mmol) et la 4-*N-*diméthylaminopyridine (0,19 mmol ; 21mg). Le mélange est agité à température ambiante pendant 16 heures. L'acétonitrile est évaporé ; le résidu dissous dans l'acétate d'éthyle (30 ml) est lavé deux fois à l'eau distillée (20 ml) puis par une solution aqueuse saturée en chlorure de sodium (20 ml). La phase organique est séchée sur sulfate de magnésium, évaporée et séchée sous vide. Le produit attendu est obtenu sous forme de solide blanc (0,514g ; rendement = 61 %).

RMN ¹H (DMSO-d₆, 400 MHz) δ : 10,06 (s, 1H, CHO) ; 8,60 (s, 1H, arom.); 7,92-7,91 (d, J = 3,7Hz, 1H, arom.), 7,81-7,75 (m, 2H, arom.) ; 6,84-6,83 (d, J=3,7Hz, 1H, arom.) ; 1,65 (s, 9H, t-Bu). SM/CL : m/z = 268,23 (M + H) tr = 5,72 min (condition 1).

### 5.1.2.2 Préparation des alcools (7a) :

Les alcools de formule générale (7a) peuvent être obtenus par réduction des aldéhydes de formule générale (10) par les méthodes classiques connues de l'homme de l'art telles que, par exemple, par action du système : NiCl₂.6H₂O-Zn dans un mélange eau / DMF à température ambiante (Baruah, R. N. Tetrahedron Lett. 1992, 33 (37), 5417-5418) ou bien à l'aide de NaBH₄ dans l'éthanol à température ambiante (Cho, Y. J. ; Lee, S. H. ; Bae, J. W. ; Pyun, H. J. ; Yoon, C. M. Tetrahedron Lett. 2000, 41 (20), 3915-3917) ou encore à l'aide de Bu₃SnH dans un solvant protique tel que, par exemple, le méthanol (Kamiura, K. ; Wada, M. Tetrahedron Lett. 1999, 40 (51), 9059-9062).

### Préparation 10 : tert-butyl 6-(hydroxyméthyl)-1H-indole-1-carboxylate (C₁₄H₁₇NO₃; M = 247,30)

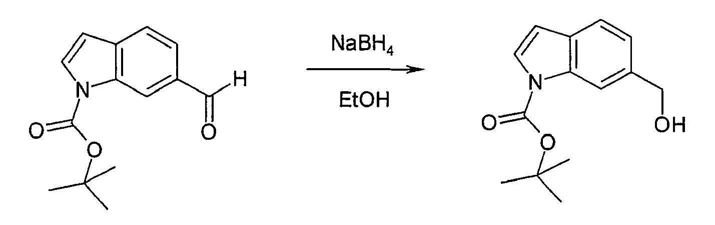

Le *tert*-butyl 6-formyl-1*H*-indole-1-carboxylate (0,514 g : 2,1 mmol) est dissous dans l'éthanol (5 ml) puis le borohydrure de sodium (0,159 g; 4,2 mmol) est ajouté lentement et la solution agitée à température ambiante pendant 2 heures. Le solvant est évaporé, et le résidu redissous dans l'éther éthylique (20 ml) est lavé avec une solution d'hydroxyde de sodium (1N ; 10 ml) puis avec une solution saturée en chlorure de sodium (10 ml). La phase organique est séchée sur sulfate de magnésium, puis évaporée et séchée sous vide. L'alcool est obtenu sous forme de solide blanc (0,48 g, rendement = 93 %).

RMN ¹H (DMSO-d₆, 400 MHz) δ : 8,09 (s, 1H, arom.) ; 7,61-7,60 (d, J = 3,6 Hz, 1H, arom.) ; 7,54-7,52 (d, J = 8 Hz, 1H, arom.) ; 7,18-7,16 (d, J = 8 Hz, 1H, arom.) ; 6,66-6,65 (d, J = 3,6 Hz, 1H, arom.) ; 5,22-5,19 (t, J = 5,7 Hz, 1H, OH) ; 4,60-4,59 (d, J = 5,7 Hz, 2H, CH₂) ; 1,62 (s, 9H, t-Bu). SM/CL : m/z = fragmenté ; tr = 10,28 min (condition 1).

### 5.1.2.3 Préparation des bromures méthylindoliques (8a) :

Les bromures méthylindoliques de formule générale (8a) sont obtenus à partir des alcools de formule générale (7a) selon les procédés de bromation généraux décrits ci-dessus pour la préparation des bromures benzyliques.

Un bromure méthylindolique de formule R₃Br a été synthétisé avec le groupement R₃ suivant :

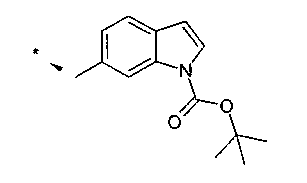

### 5.2 Préparation des bromures éthylindoliques (8b) :

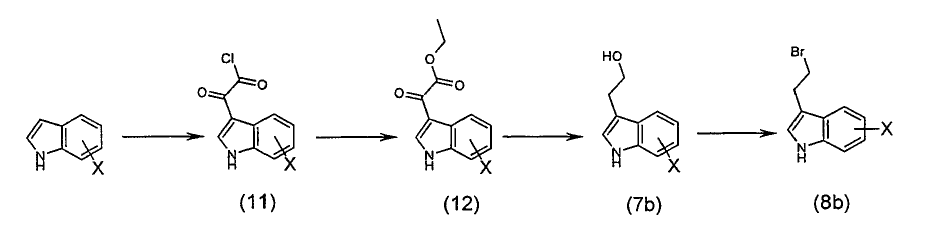

Dans le cas où les intermédiaires bromés de formule générale (8) sont de type éthylindolique, ils peuvent être obtenus en 4 étapes à partir des indoles correspondants, tout d'abord par conversion en chlorure d'α-cétoacide (11) suivie d'une transformation en α-cétoester (12) puis d'une réduction en alcool (7b), pour finalement préparer l'intermédiaire bromé (8b).

### 5.2.1 Obtention des chlorures d'α-cétoacides (11) :

Les chlorures d'α-cétoacides (11) peuvent être obtenus par action de chlorure d'oxalyle dans un solvant aprotique apolaire tel que, par exemple, le diéthyléther, à température ambiante (Woodward, R. B. ; Bader, F. E. ; Bickel, H. ; Frey, A. J. ; Kierstead, R. W. Tetrahedron 1952 2, 1).

### Préparation 11 : chlorure de (6-méthoxy-1H-indol-3-yl)(oxo)acétyle_(C₁₁H₈ClNO₃, M = 237,64).

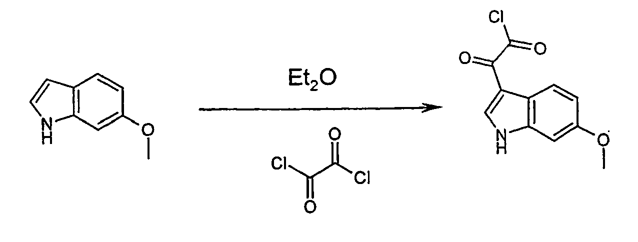

Le 5-méthoxyindole (1g; 6,8 mmol) dissous dans l'éther éthylique (25 ml) est refroidi à 0° C. Le chlorure d'oxalyle (8,8 mmol ; 0,77 ml) est ajouté goutte à goutte sous argon et le mélange est agité à température ambiante sous atmosphère d'argon pendant trois heures. Le produit attendu est obtenu sous forme d'une poudre jaune après filtration et lavage à l'éther éthylique.(1,44 g, rendement = 89 %).

RMN ¹H (DMSO-d₆, 400 MHz) δ : 12,19 (s, 1H, NH) ; 8,27 (s, 1H, arom.); 8,01-7,99 (d, J = 8,7 Hz, 1H, arom.) ; 7,02 (s, 1H, arom.) ; 6,90-6,87 (d, J = 8,7 Hz, 1H, arom.) ; 3,79 (s, 1H, OCH₃).

Des chlorures d'α-cétoacides de formule R_{3'}C(O)C(O)Cl ont étés préparés avec les groupements R₃, indoliques suivants :

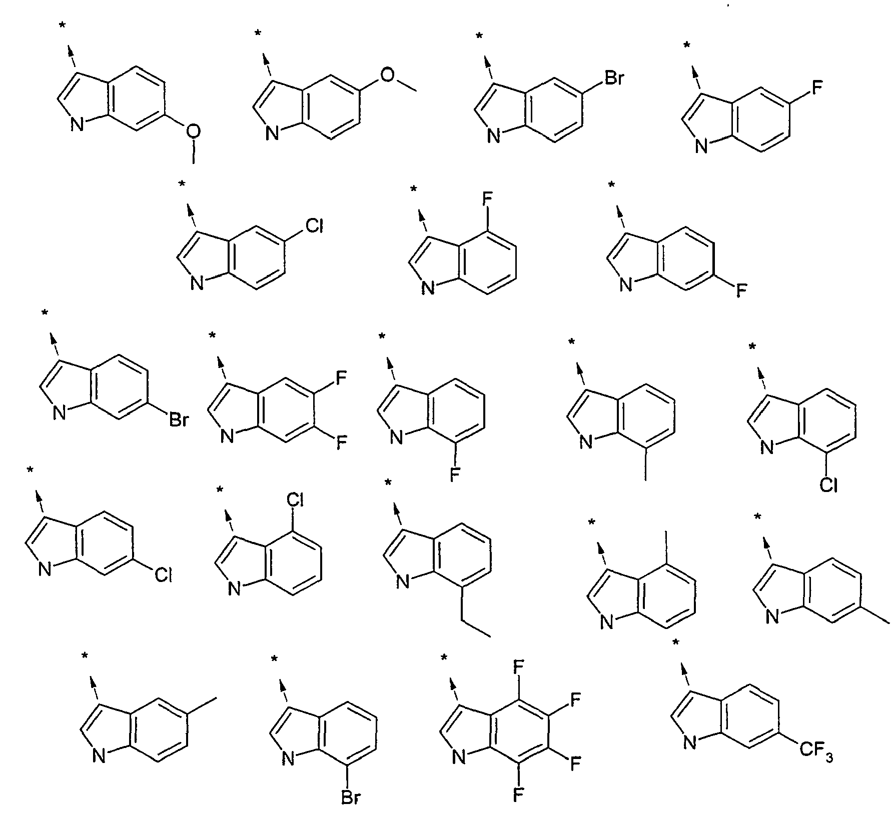

### 5.2.2 Estérification des chlorures d'α-cétoacides (11) en α-cétoesters (12) :

Les α-cétoesters indoliques (12) sont obtenus par les méthodes d'estérification classiques connues de l'homme de l'art, telles que, par exemple, le traitement du chlorure d'α-cétoester correspondant par un alcool (tel que méthanol ou éthanol) en présence d'une base organique telle que, par exemple, la triéthylamine ou la diisopropyléthylamine.

### Préparation 12 : (6-méthoxy-1H-indol-3-yl)(oxo)acétate d'éthyle (C₁₃H₁₃NO₄, M = 247,25)

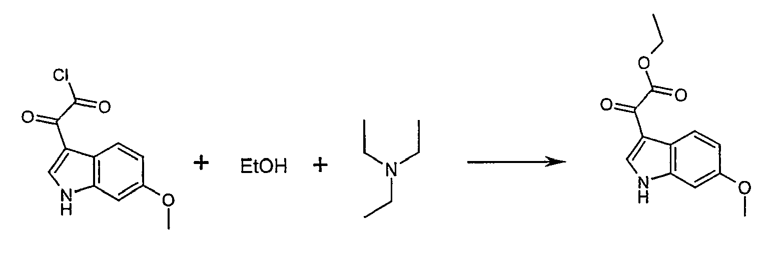

Le chlorure de (6-méthoxy-1*H*-indol-3-yl)(oxo)acétyle (1,44 g ; 6,06 mmol) dissous dans l'éthanol (15 ml) est refroidi à 0° C puis la triéthylamine (1,04 ml ; 7,5 mmol) est ajoutée goutte à goutte. Le mélange est chauffé à reflux pendant 2 heures. Le précipité est filtré, lavé à l'éthanol (5 ml) et à l'éther éthylique (5 ml) puis séché sous vide. Le produit attendu est obtenu sous forme d'une poudre jaune (1,36 g ; rendement = 91 %).

RMN ¹H (DMSO-d₆, 400 MHz) δ : 12,16 (s, 1H, NH) ; 8,28 (s, 1H, arom.) ; 8,00-7,98 (d, J = 8,6 Hz, 1H, arom.) ; 7,02 (s, 1H, arom.) ; 6,91-6,88 (d, J = 8,6 Hz, 1H, arom.) ; 4,37-4,31 (q, J = 7 Hz, 2H, OCH₂) ; 3,79 (s, 3H, OCH₃) ; 1,34-1,31 (t, J = 7Hz, 3H, CH₃).

Des α-cétoesters de formule R_{3'}C(O)C(O)OEt ont étés préparés avec les groupements R_{3'} indoliques suivants :

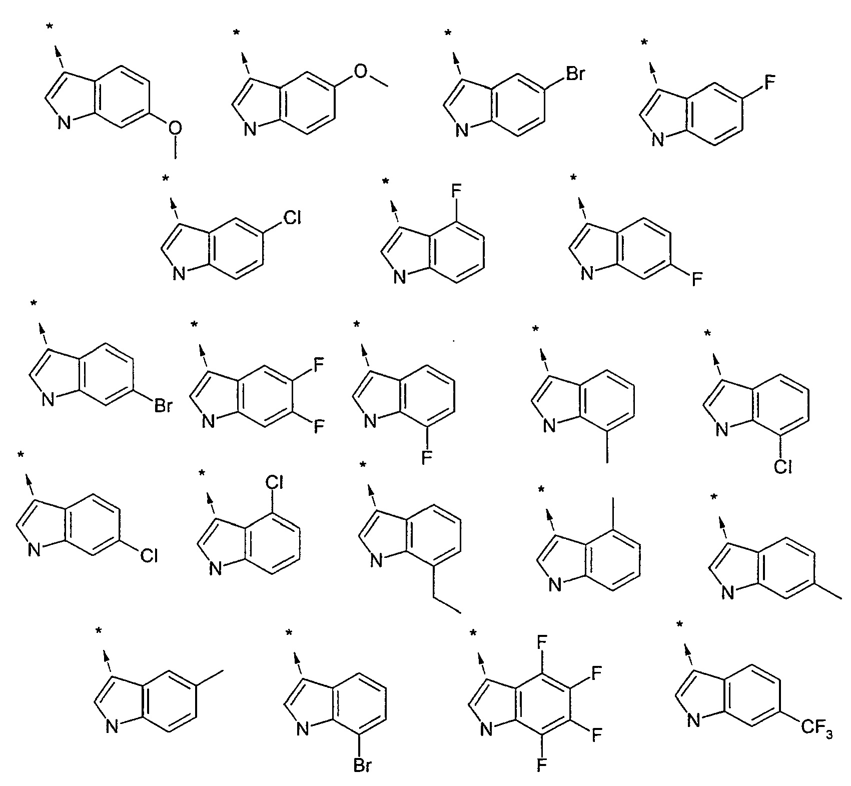

### 5.2.3 Réduction des α-cétoesters (12) en alcools éthylindoliques (7b) :

Les α-cétoesters de formule générale (12) peuvent être réduits en éthylalcools de formule générale (7b) par traitement par exemple par de l'hydrure de lithium et aluminium dans un solvant aprotique tel que le tétrahydrofuranne au reflux (Feldman, P. L. ; Rapoport, H. Synthesis 1986 (9), 735-737).

### Préparation 13 : 2-(6-méthoxy-1H-indol-3-yl)éthanol (C₁₁H₁₃NO₂, M = 191,23).

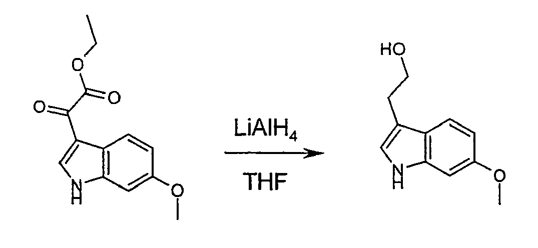

Le (6-méthoxy-1*H*-indol-3-yl)(oxo)acétate d'éthyle (1,36 g ; 5,5 mmol) dissous dans le tétrahydrofuranne (15 ml) est refroidi à 0° C. L'hydrure de lithium et aluminium en solution dans le tétrahydrofuranne (1M ; 16,5 ml ; 16,5 mmol) est alors ajouté lentement. Le mélange réactionnel est porté à reflux, et agité pendant 2 heures. L'excès d'hydrure de lithium et aluminium est neutralisé par l'ajout d'acétate d'éthyle (1 ml) et d'eau distillée (1 ml). La réaction est filtrée à chaud et le solide est lavé avec du méthanol (10 ml). Le filtrat évaporé est resolubilisé dans de l'acétate d'éthyle (25 ml), lavé avec une solution aqueuse d'acide chlorhydrique (0,1M; 15 ml) puis avec une solution saturé en chlorure de sodium (15 ml). La phase organique est séchée sur sulfate de sodium, évaporée puis séchée sous vide. Le produit attendu est obtenu sous forme d'huile jaune clair (0,815 g, rendement = 78 %).

RMN ¹H (DMSO-d₆, 400 MHz) δ :10,54 (s, 1H, NH) ; 7,36-7,34 (d, J = 8,6 Hz, 1H, arom.) ; 6,96 (s, 1H, arom.) ; 6,82 (s, 1H, arom.) ; 6,63-6,60 (d, J = 8,6 Hz, 1H, arom.) ; 4,57-5,54 (t, J = 5,4 Hz, 1H, OH) ; 3,74 (s, 3H, OCH₃) 3,64-3,59 (m, J = 7,4 Hz et J' = 5,4Hz, 2H, CH₂) ; 2,80-2,76 (t, J = 7,4Hz, 2H, CH₂). SM/LC : m/z = 192,17 (M + H) tr = 8,27 min (condition 2).

Des alcools éthylindoliques de formule R_{3'}(CH₂)₂OH ont été préparés avec les groupements R_{3'} indoliques suivants :

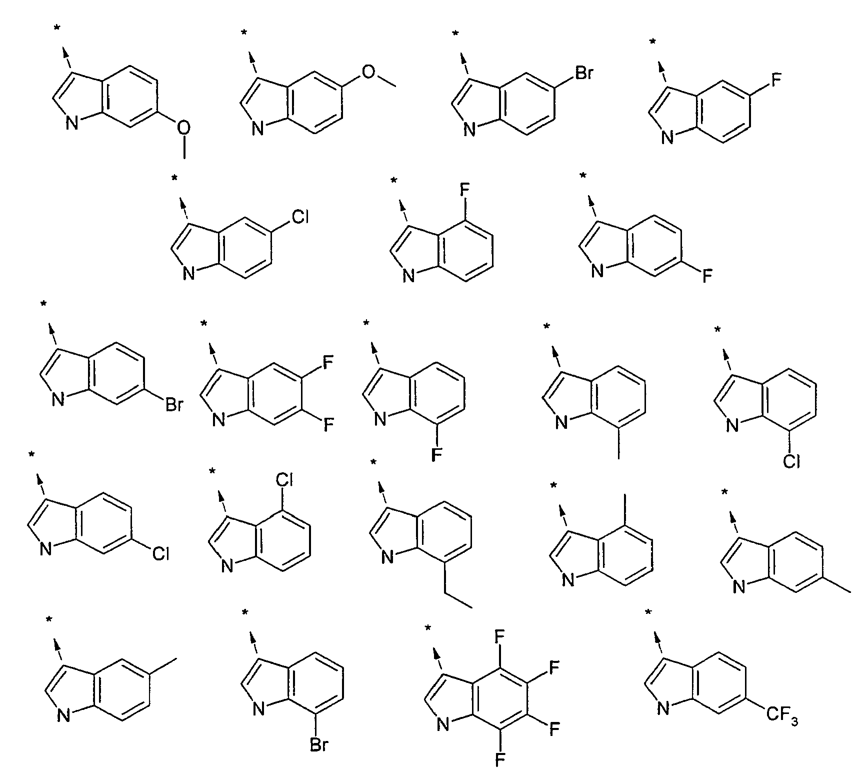

### 5.2.4 Préparation des bromures éthylindoliques (8b) :

Les bromures éthylindoliques de formule générale (8b) peuvent être préparés par bromation des alcools correspondants (7b) selon les méthodes générales décrites plus haut pour l'obtention des bromures benzyliques.

### Préparation 14 : 3-(2-bromoéthyl)-6-méthoxy-1H-indole (C11H12BrNO, M = 254,13)

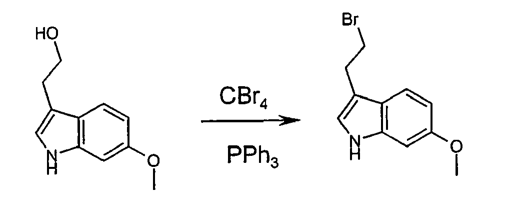

Le 2-(6-méthoxy-1*H*-indol-3-yl)éthanol (0,815 g, 4,3 mmol) et le tétrabromure de carbone (1,6 g ; 5 mmol) dissous dans le dichlorométhane (25 ml) sont refroidis à 0 °C. La triphénylphosphine est ajoutée (1,3 g ; 5 mmol). Le mélange réactionnel est agité à température ambiante pendant 2 heures. Le dichlorométhane est évaporé et le résidu obtenu est purifié sur silice (éluant : heptane / acétate d'éthyle : 3 / 1). Les fractions sont évaporées et le solide obtenu est séché sous vide (0,69 g ; rendement = 63 %).

RMN ¹H (DMSO-d₆, 400 MHz) δ : 10,69 (s, 1H, NH) ; 7,42-7,39 (d, J = 8,6 Hz, 1H, arom.) ; 7,08 (s, 1H, arom.) ; 6,84 (s, 1H, arom.); 6,65-6,62 (d, J = 8,6 Hz, 1H, arom.) ; 3,74 (s, 3H, OCH₃) ; 3,71-3,68 (t, J = 7,6 Hz, 2H, CH₂) ; 3,21-3,17 (t, J = 7,6 Hz, 2H, CH₂). SM/LC : m/z = 254,04 (M + H) tr = 10,56 min (condition 2).

Des bromures indoliques de formule R_{3'}(CH₂)₂Br ont étés préparés avec les groupements R_{3'} indoliques suivants :

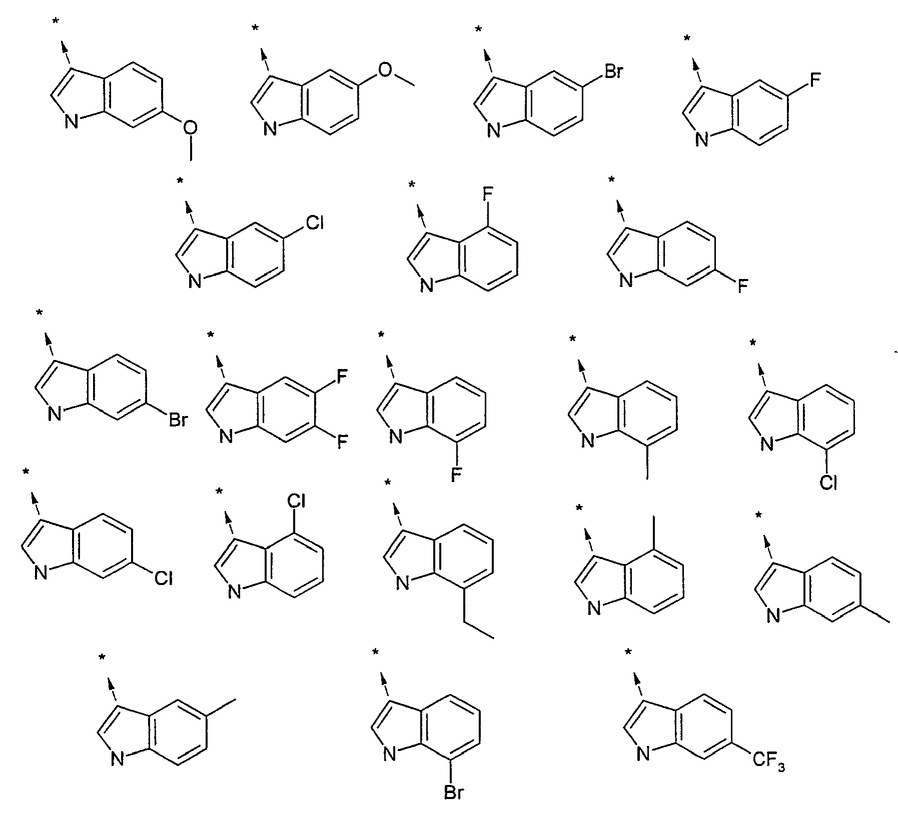

### 5.3. Préparation des dérivés bromés de formule générale (8d) :

Les dérivés bromés de formule générale Br-(CH₂)_{n'}[Q']_{p'}[C(X')(Y')]_{m'}Z' où Q' représente C(O), p' représente 0 ou 1, m' représente 0, Z' représente le groupement indolyle et (CH₂)_{n'} a la signification indiquée ci-dessus, (8d), peuvent être obtenus selon des méthodes connues de l'homme de l'art, par exemple par acylation d'un indole (O. Ottoni et al. Org. Lett., 2001, 3(7), 1005-1007), suivie ou non d'une réduction du groupement carbonyle (E. Wenkert et al. J. Org. Chem. 1986, 51(12), 2343-2351).

### Préparation 15 : 3-bromo-1-(7-methyl-1H indol-3-yl)propan-1-one (C12H12BrNO, M = 266,14).

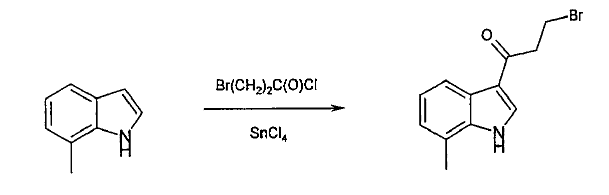

Le 7-méthyl-1*H*-indole (131 mg; 1 mmol) est mis en solution dans 2 ml de dichlorométhane à 0°C. Une solution molaire de tétrachlorure d'étain dans le dichlorométhane (1,2 ml ; 1,2 mmol) est ajoutée à 0°C puis le mélange réactionnel est agité à température ambiante pendant 30 minutes. Le chlorure de 3-bromopropionyle (101 µl; 1 mmol) et le nitrométhane (1,5 ml) sont ensuite ajoutés dans le milieu et la réaction maintenue sous agitation pendant 24 heures. Puis 5 ml d'eau sont ajoutés et le produit est extrait par 3 fois 5 ml d'acétate d'éthyle. Les phases organiques sont réunies, séchées sur sulfate de sodium et les solvants évaporés. Le solide obtenu est séché sous pression réduite (21,5 mg ; rendement = 8 %).

RMN ¹H (DMSO-d₆, 400 MHz) δ : 8,39-8,38 (m, 1H, arom.) ; 8,03-8,01 (d, 1H, arom.) ; 7,09-7,07 (t, 1H, arom.) ; 7,02-7,01 (d, 1H, arom.) ; 3,82-3,79 (t, 2H, CH₂); 3,51-3,48 (t, 2H, CH₂); 2,43 (s, 3H, CH₃). SM/LC: m/z = 266,03 tr = 9,84 min (condition 1).

Les dérivés bromés de formule générale (8d) ont été préparés avec les groupes - (CH₂)_{n'}[Q']_{p'}[C(X')(Y')]_{m'}Z' suivants :

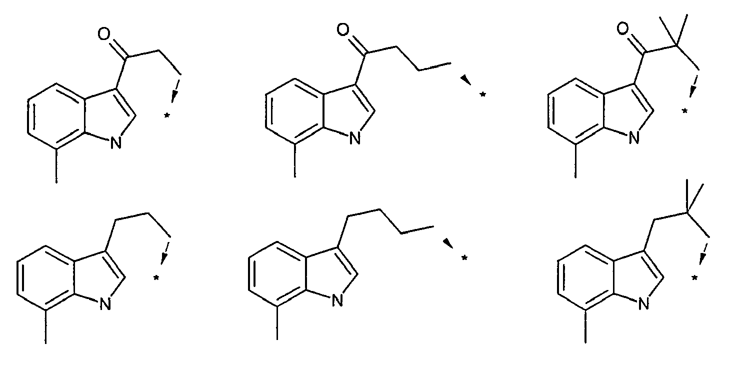

### 6. Préparation des composés de formule générale (I) :

### 6.1. Substitution des thiols (6) par des bromures benzyliques (8a) :

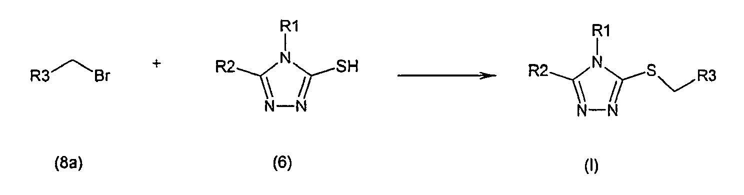

Les thiols de formule générale (6) peuvent être substitués par des bromures benzyliques de formule générale (8a) après activation de l'atome de soufre par une base telle que NaOAc, KOH, K₂CO₃ dans un solvant protique tel que le méthanol ou l'éthanol (Shetgiri, N. P. ; Kokitkar, S. V. Indian J. Chem, Sect B : Org Chem Incl Med Chem 2001, 40 (2), 163-166) ou bien par une base organique telle que la triéthylamine ou la diisopropylamine dans un solvant apolaire tel que l'acétone ou le dichlorométhane ou encore par une base supportée sur résine telle que la résine morpholinométhyl polystyrène (Novabiochem) ou la résine 7-méthyl-1,5,7-triazabicyclo[4,4,0]dec-5-ene polystyrène (Novabiochem) après gonflement de la résine dans un solvant aprotique tel que le dichlorométhane. La réaction se déroule à température ambiante pendant une durée variant de 12 à 36 heures. L'excès de réactif de formule générale (8a) peut être piégé par ajout par exemple d'une résine thiophénol (Argonaut) et agitation pendant 4 à 8 heures. La suspension est filtrée, le filtrat évaporé et purifié par chromatographie sur colonne de silice. Dans le cas où la fonction amine présente sur la molécule est protégée par un groupement de type carbamate (tel que, par exemple, le groupement *tert*-butoxycarbonyle), le résidu est traité par un acide tel que l'acide trifluoroacétique pendant 10 à 30 minutes ou par une solution molaire d'acide chlorhydrique dans l'éther éthylique pendant 16 à 20 heures. Le produit final est alors obtenu sous forme salifiée et dans le cas du trifluoroacétate, le sel est traité par une résine basique de type Amberlite puis resalifié par une solution molaire d'acide chlorhydrique dans un solvant aprotique tel que l'éther éthylique, l'acétate d'éthyle ou le dioxanne.

### Exemple A : chlorhydrate de 2-[3-(benzyl-5-yl)-5-phényl-4H-1,2,4-triazol-4-yl]éthylamine (C₁₇H₁₉N₄SCl, M = 346,88)

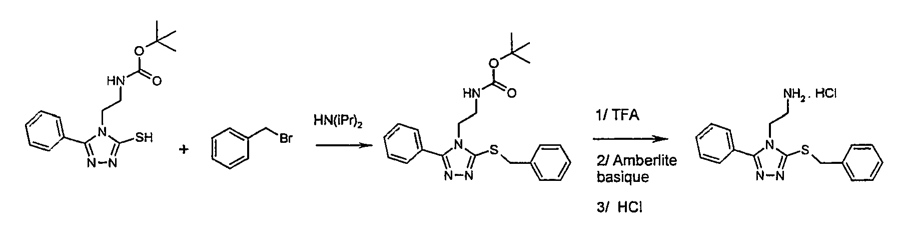

Au *tert*-butyl 2-(3-phényl-5-sulfanyl-4H-1,2,4-triazol-4-yl)éthylcarbamate (320 mg; 1 mmol) dissous dans le tétrahydrofurane (5 ml) est ajoutée la diisopropylamine (0,14 ml ; 1 mmol), puis le bromure de benzyle (0,12 ml ; 1 mmol). La solution est agitée à température ambiante pendant 24 heures, puis le solvant est évaporé. Du dichlorométhane (2 ml) et de l'acide trifluoroacétique (2 ml) sont ajoutés et la solution obtenue est agitée pendant 10 minutes à température ambiante. Les solvants sont évaporés, le composé redissous dans le méthanol est passé sur une résine Amberlite basique afin d'obtenir l'amine sous forme de base libre, puis purifié par chromatographie sur colonne de silice (éluant : acétate d'éthyle / méthanol : 1/1). Les fractions sont évaporées et le chlorhydrate de l'amine est obtenu par traitement par une solution molaire d'acide chlorhydrique dans l'éther éthylique (1 ml ; 1 mmol). Le précipité formé est filtré, lavé à l'éther éthylique puis séché sous vide (80 mg ; rendement = 23 %).

RMN ¹H (DMSO-d₆, 400 MHz) δ : 8,22 (s large, 3H, NH₃⁺) ; 7,66-7,63 (m, 2H, arom.) ; 7,58-7,56 (m, 3H, arom.) 7,41-7,40 (m, 2H, arom.) ; 7,35-7,29 (m, 3H, arom.) ; 4,46 (s, 2H, CH₂) ; 4,16 (t, J = 8,3 Hz, 2H, CH₂) ; 2,88-2,82 (m, J = 2 et 8,3 Hz, 2H, CH₂). SM/LC : m/z = 311,13 (M + H) tr = 6,51 min (condition 1).

Des bromures benzyliques (8a) de formule générale R₃Br ont été utilisés avec les groupes R₃ suivants :

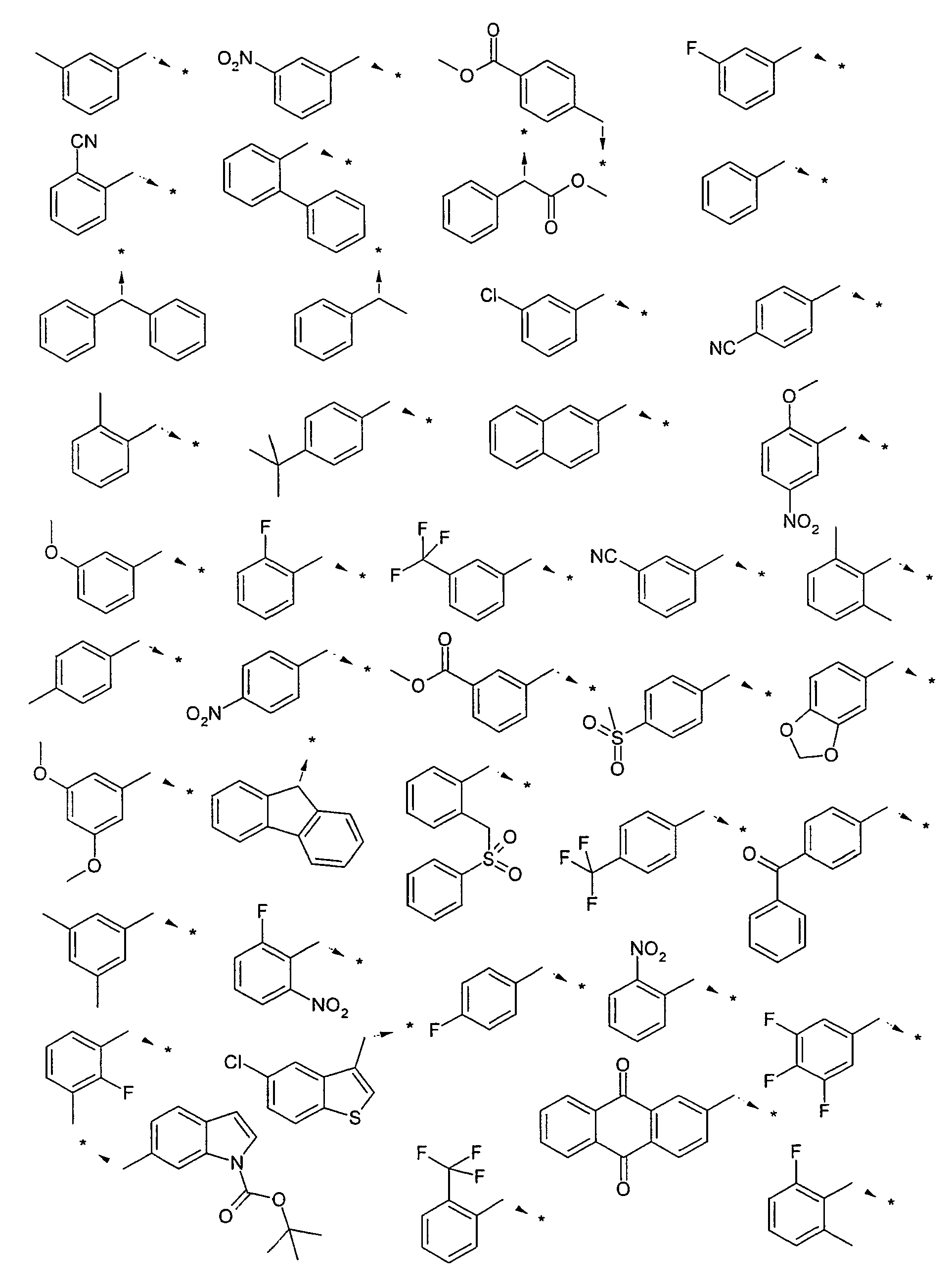

### 6.2. Substitution des thiols (6) par des α-bromocétones (8c) :

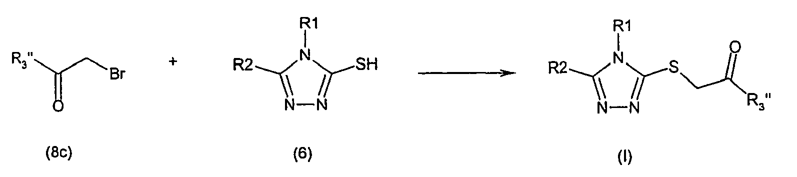

Les thiols de formule générale (6) peuvent être substitués par des α-bromocétones de formule générale (8c) après activation de l'atome de soufre dans les mêmes conditions que celles décrites précédemment. La réaction se déroule à température ambiante pendant une durée variant de 12 à 24 heures. L'excès de réactif de formule générale (8c) peut être piégé par ajout par exemple d'une résine thiophénol (Argonaut) ou d'une résine de type aminométhyl-polystyrène (Novabiochem) et agitation pendant 4 à 8 heures. La suspension est filtrée, le filtrat évaporé et purifié sur colonne de silice. Dans le cas où la fonction amine présente sur la molécule est protégée par un groupement de type carbamate (tel que, par exemple, le groupement *tert*-butoxycarbonyle), le résidu est traité par une solution molaire d'acide chlorhydrique dans l'éther éthylique pendant 16 à 20 heures. Le produit final est alors obtenu, après purification sur colonne de silice si nécessaire, sous forme de chlorhydrate.

### Exemple B : chlorhydrate de 2-{[4-(6-aminohexyl)-5-(2-naphthyl)- 4H-1,2,4-triazol-3-yl]sulfanyl}-1-[4-(diéthylamino)phényl]éthanone (C₃₀H₃₈N₅OSCl, M = 552,19)

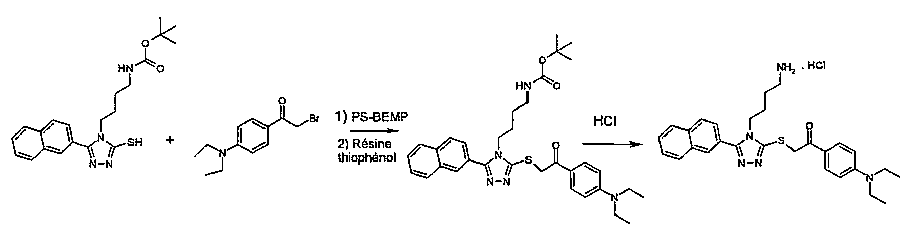

A du *tert*-butyl 6-[3-(2-naphtliyl)-5-sulfanyl-4*H*-1,2,4-triazol-4-yl]hexylcarbamate (30 mg; 0,07 mmol) dissous dans du tétrahydrofuranne (1 ml) est ajoutée la résine 2-*tert*-butylimino-2-diéthylamino-1,3-diméthyl-perhydro-1,3,2-diazophosphorine sur polystyrène. La suspension est agitée à température ambiante pendant 30 minutes puis de la 2-bromo-1-[4-(diéthylamino)phényl]éthanone (22 mg ; 0,08 mmol) est ajoutée au milieu. Le mélange est agité à température ambiante pendant 16 heures. L'excès de 2-bromo-1-[4-(diéthylamino)phényl]éthanone est piégé par ajout d'une résine thiophénol (70 mg, 0,1 mmol, Argonaut) et agitation pendant 6 heures. La suspension est filtrée et le filtrat évaporé. Pour déprotéger la fonction amine, le filtrat est solubilisé dans du méthanol (0,5 mmol) puis une solution molaire d'acide chlorhydrique dans l'éther éthylique est ajoutée (2 ml ; 2 mmol). La solution est agitée pendant 16 heures, puis évaporée. Le solide résultant est séché sous vide (28 mg ; rendement = 63 %). SM/LC : m/z = 516,40 (M + H) tr = 8,60 min (condition 1).

Des bromocétones (8c) de formule générale R₃Br ont étés utilisées avec les groupements R₃ suivants :

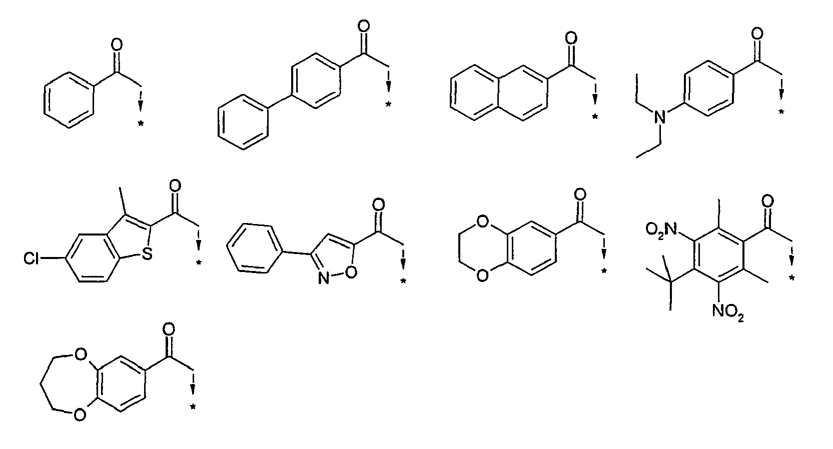

### 6.3. Substitution des thiols (6) par des halogénures aliphatiques, des bromures éthylindoliques (8b) ou des dérivés bromés de formule générale (8d) :

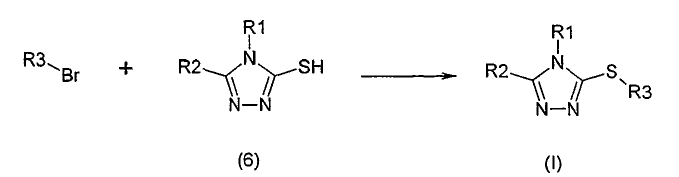

Les thiols de formule générale (6) peuvent être substitués par des halogénures aliphatiques, des bromures éthylindoliques de formule générale (8b) ou des dérivés bromés de formule générale (8d) après activation de l'atome de soufre par la résine 2-*tert*-butylimino-2-diethylamino-1,3-diméthyl-perhydro-1,3,2-diaza-phosphorine sur polystyrène (Fluka). La réaction se déroule à température ambiante pendant une durée variant de 3 à 6 heures. La suspension est filtrée, le filtrat évaporé et purifié sur colonne de silice. Dans le cas où la fonction amine présente sur la molécule est protégée par un groupement de type carbamate (tel que, par exemple, le groupement *tert*-butoxycarbonyle), le résidu est traité par une solution molaire d'acide chlorhydrique dans l'éther éthylique pendant 16 à 20 heures. Le produit final est alors obtenu sous forme de chlorhydrate.

### Exemple C : chlorhydrate de 3[3-{[2-(1H-indol-3-yl)éthyl]sulfanyl}-5-(2-naphthyl)-4H-1,2,4-triazol-4-yl]propylamine (C₂₆H₂₈N₅SCl, M = 478,06)

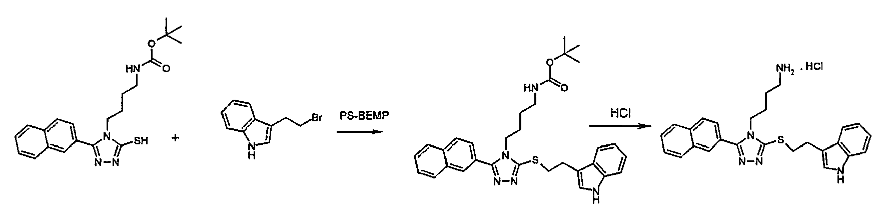

### Etape 1 : tert-butyl-3[3-{[2-(1H-indol-3-yl)éthyl]sulfanyl}-5-(2-naphthyl)-4H-1,2,4-triazol-4-yl]propylcarbamate (C₃₁H₃₅N₅O₂S, M = 541,72)

A 265 mg (0,66 mmol) de *tert*-butyl 4-[3-(2-naphthyl)-5-sulfanyl-4*H*-1,2,4-triazol-4-yl]butylcarbamate dans du tétrahydrofuranne anhydre (15 ml) est ajoutée la résine-2-*tert*-butylimino-2-diéthylamino-1,3-diméthyl-perhydro-1,3,2-diazaphosphorine sur polystyrène (0,91 g, 2 mmol, 2,2 mmol/g, Fluka). La suspension est agitée 10 minutes à température ambiante, puis le 3-(2-bromométhyl)indole (149 mg, 0,66 mmol) est additionné. Le mélange réactionnel obtenu est agité à température ambiante pendant 4 heures, puis filtré. Le filtrat évaporé est purifié par flash chromatographie sur colonne de silice (acétate d'éthyle/heptane 2 :1). Les fractions sont recombinées, évaporées et le résidu blanc est séché sous vide (249 mg, rendement = 70 %).

RMN ¹H (DMSO-d₆, 400 MHz) δ : 8,41 (s large, 1H, NH); 8,01 (s, 1H, arom.) ; 7,99-7,96 (m, 1H, arom.) ; 7,92-7,91 (m, 2H, arom.); 7,69-7,65 (m, 2H, arom.) ; 7,60-7,57 (m, 2H, arom.) ; 7,39-7,37 (m, 1H, arom.) ; 7,22-7,20 (m, 1H, arom.) ; 7,14-7,11 (m, 2H, arom.); 4,44 (s large, 1H, NH) ; 3,91 (t, J = 8 Hz, 2H, CH₂); 3,70 (t, J = 5,9 Hz, 2H, CH₂); 3,33 (t, J = 5,9 Hz, 2H, CH₂); 2,99-2,97 (m, 2H, CH₂); 1,61-1,57 (m, 2H, CH₂); 1,42 (s, 9H, (CH₃)₃); 1,36-1,27 (m, 2H, CH₂). SM/CL : m/z = 542,36 (M+H) rt = 11,07 min (condition 1).

### Etape 2 : chlorhydrate de 3[3-{[2-(1H-indol-3-yl)éthyl]sulfanyl}-5-(2-naphthyl)-4H-1,2,4-triazol-4-yl]propylamine (C₂₆H₂₈N₅SCl, M = 478,06)

Le *tert*-butyl-3[3-{[2-(1H-indol-3-yl)éthyl]sulfanyl}-5-(2-naphthyl)-4*H*-1,2,4-triazol-4-yl]propylcarbamate précédemment formé est dissous dans du dichlorométhane (3 ml) et du méthanol (2 ml) anhydres puis une solution molaire d'acide chlorhydrique dans l'éther éthylique (3,1 ml) est ajoutée à la solution. Le mélange est agité pendant 45 minutes puis évaporé et le solide beige obtenu est séché sous vide (188 mg, rendement = 94 %).

RMN ¹H (D₂O, 77° C, 400 MHz) δ : 8,59-8,56 (m, 1H, arom.); 8,51-8,49 (m, 2H, arom.); 8,41 (s, 1H, arom.) ; 8,19-8,16 (m, 2H, arom.) ; 8,04-8,02 (m, 1H, arom.) ; 7,96-7,93 (m, 1H, arom.); 7,87-7,84 (m, 1H, arom.) ; 7,68 (s, 1H, arom.); 7,65-7,63 (m, 1H, arom.) ; 7,58-7,56 (m, 1H, arom.) ; 4,28 (t, J = 8,3 Hz, 2H, CH₂); 4,17 (t, J = 5,5 Hz, 2H, CH₂); 3,72 (t, J = 5,5 Hz, 2H, CH₂); 3,14 (t, J = 8,3 Hz, 2H, CH₂); 1,92-1,85 (m, J = 8,3 Hz et 7 Hz, 2H, CH₂); 1,82-1,74 (m, J = 8,3 Hz et 7 Hz, 2H, CH₂). SM/CL : m/z = 442,26 (M + H), rt = 8,14 min (condition 1).

Des halogénures aliphatiques, des dérivés bromés (8b) ou (8d) de formule générale R₃Br ont étés utilisés avec les groupements R₃ suivants:

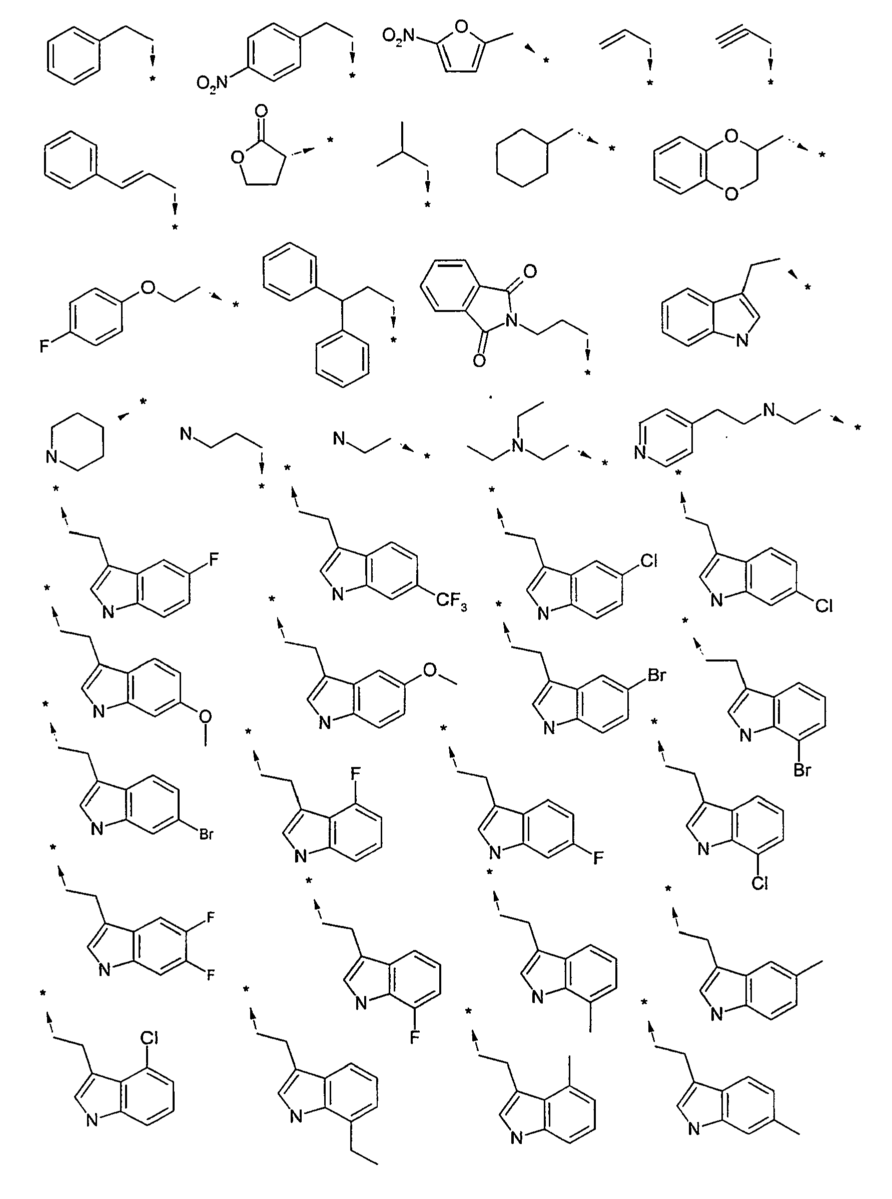

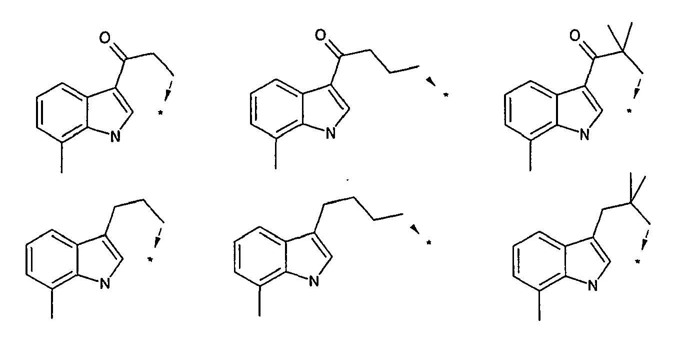

### 6.4. Cas particulier où R₃ comporte une fonction amide :

Les composés de formule générale (I) tels que R₃ est un radical de formule -CH₂-C(O)-NH-(CH₂)ₘ-NXY, où m, X et Y sont tels que définis ci-dessus, peuvent être obtenus en 3 étapes à partir du thiol de formule générale (6).

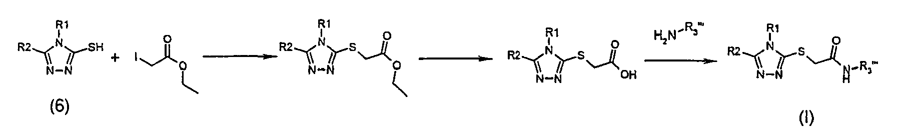

### 6.4.1. Substitution du soufre et hydrolyse de l'ester :

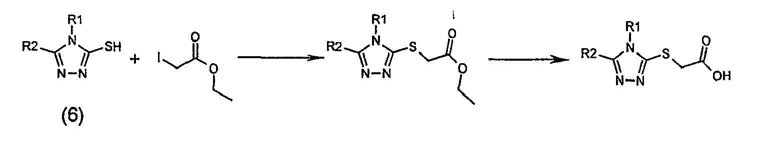

Les thiols de formule générale (6) peuvent être substitués par l'iodoacétate d'éthyle après activation de l'atome de soufre par une base telle que NaH ou par utilisation de la résine 2-*tert*-t-butylimino-2-diethylamino-1,3-diméthyl-perhydro-1,3,2-diazaphosphorine sur polystyrène (Fluka) dans un solvant aprotique tel que le dichlorométhane ou le diméthylformamide. La réaction se déroule à température ambiante pendant une durée variant de 12 à 24 heures, puis le mélange réactionnel est lavé et concentré sous vide. L'ester est ensuite hydrolysé par traitement par une base telle que, par exemple une solution aqueuse de KOH ou d'hydroxyde de lithium en présence d'un solvant aprotique tel que le tétrahydrofuranne à température ambiante pendant une durée variant de 3 à 6 heures (Baldwin, J. E. ; Adlington, R. M. ; Ramcharitar, S. H. J Chem Soc, Chem Commun 1991 (14), 940-942). L'acide correspondant est obtenu après évaporation des solvants, neutralisation par une solution aqueuse d'acide chlorhydrique, extraction par un solvant organique tel que l'acétate d'éthyle et utilisé dans les étapes suivantes sans autre purification.

### Préparation 16 : Acide {[4-(2,2-diphényléthyl)-5-(2-naphthylméthyl)-4H-1,2,4-triazol-3-yl]sulfanyl} acétique (C₂₉H₂₅N₃O₂S, M = 479,61)

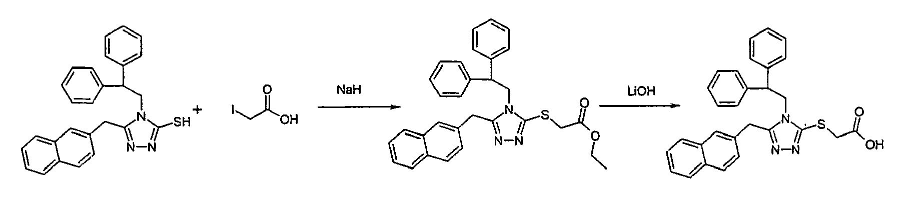

A du 4-(2,2-diphényléthyl)-5-(2-naphthylméthyl)-4H-1,2,4-triazol-3-thiol (4 g ; 9,5 mmol) dissous dans du dichlorométhane (100 ml) est ajouté de l'hydrure de sodium (0,4 g ; 10 mmol) ; la solution est agitée à température ambiante pendant 30 minutes. De l'iodoacétate d'éthyle est ajouté (1,2 ml ; 10 mmol) et le mélange est agité à température ambiante pendant 16 heures. Le mélange réactionnel est lavé avec de l'eau distillée (50 ml) puis avec une solution saturée de chlorure de sodium (50 ml). La phase organique est évaporée. L'acide est obtenu par hydrolyse : au résidu dissous dans du tétrahydrofuranne (80 ml) est ajouté de l'hydroxyde de lithium (1,1 g ; 27 mmol) dissous dans de l'eau distillée (40 ml), et ce mélange est agité à température ambiante pendant 4 heures. Les solvants sont évaporés puis une solution normale d'acide chlorhydrique est ajoutée jusqu'à ce que le pH soit légèrement acide. Cette solution est extraite deux fois avec de l'acétate d'éthyle (50 ml), les phases organiques sont combinées, séchées sur du sulfate de sodium, filtrées, évaporées et le solide obtenu est séché sous vide (2 g, rendement = 44 %) avant d'être utilisé dans l'étape suivante.

RMN ¹H (DMSO-d₆, 400 MHz) δ : 12,92 (s large, 1H, C(O)-OH) ; 7,92-7,90 (m, 1H, arom.); 7,83-7,81 (m, 1H, arom.); 7,55-7,37 (m, 4H, arom.); 7,34-7,23 (m, 10H, arom.) ; 7,11-7,09 (m, 1H, arom.) ; 4,64 (d, J = 9 Hz, 2H, CH₂) ; 4,38 (t, J = 9 Hz, 1H, CH) ; 3,96 (s, 2H, CH₂) ; 3,92 (s, 2H, CH₂). SM/CL : m/z = 480,28 (M + H), tr = 10.75 min (condition 1).

### 6.4.2. Couplage peptidique :

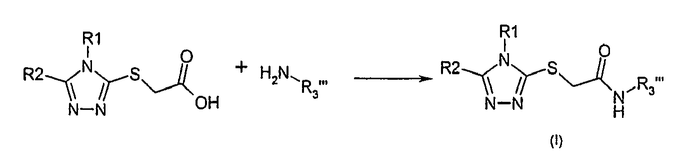

Les composés de formule générale (I) tels que R₃ est un radical de formule -CH₂-C(O)-NH-(CH₂)ₘ-NXY, où m, X et Y sont tels que définis ci-dessus, peuvent être obtenus par les méthodes classiques de la synthèse peptidique (M. Bodansky, The Practice of Peptide Synthesis, 145 (Springer-Verlag, 1984)), par exemple dans le tétrahydrofuranne, le dichlorométhane ou le diméthylformamide en présence d'un réactif de couplage tels que le cyclohexylcarbodiimide (DCC), le 1,1'-carbonyldiimidazole (CDI) (J. Med. Chem. 1992, 35 (23), 4464-4472), le chlorhydrate de 1-(3-diméthylaminopropyl)-3-éthylcarbodiimide (EDC ou WSCI) (John Jones, the chemical synthesis of peptides, 54 (Clarendon Press, Oxford, 1991)) ou le benzotriazol-1-yl-oxy-tris-pyrrolidino-phosphonium hexafluorophosphate (PyBOP) (Coste, J. ; Le-Nguyen, D. ; Castro, B. ; Tetrahedron Lett 1990, 31, 205). Le composé de formule générale (I) est obtenu après purification sur colonne de silice.

### Exemple D : 2-{[4-(2,2-diphényléthyl)-5-(2-naphthylméthyl)-4H-1,2,4-triazol-3-yl]sulfanyl}-N-[3-(4-méthyl-1-pipérazinyl)propyl]acétamide (C₃₇H₄₂N₆OS, M = 618,85)

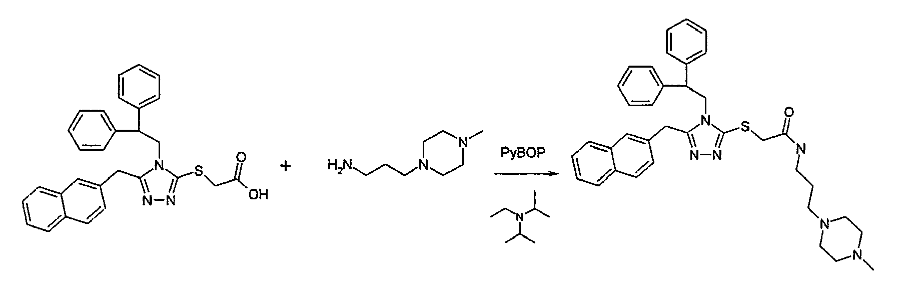

A de l'acide {[4-(2,2-diphényléthyl)-5-(2-naphthylméthyl)-4H-1,2,4-triazol-3-yl]sulfanyl}acétique (48 mg ; 0,1 mmol) dissous dans du dichlorométhane (5 ml) est ajouté du benzotriazol-1-yl-oxy-tris-pyrrolidino-phosphonium hexafluorophosphate (52 mg ; 0,1 mmol). La solution est agitée à température ambiante pendant 30 minutes puis sont ajoutées de la diisopropyl-éthyl-amine (38 µl ; 0,22 mmol) et de la 3-(4-méthyl-1-pipérazinyl)propylamine (20 µl ; 0,12 mmol). Le mélange est agité sous argon à température ambiante pendant 16 heures. Les solvants sont évaporés et le résidu est purifié par chromatographie sur colonne de silice (éluant : dichlorométhane / méthanol 95/5). Après évaporation des fractions, le solide obtenu est séché sous vide (7 mg, rendement = 11 %). SM/CL : m/z = 619,41 (M + H), tr = 8,37 min (condition 1).

Les groupements de type R₃''' suivants ont été utilisés :

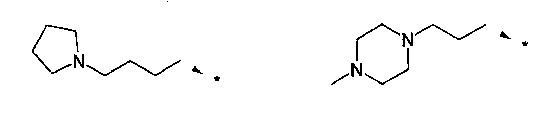

L'invention a également pour objet un procédé de préparation, en phase liquide, des composés de formule I selon l'invention, **caractérisé en ce qu**'il comprend la réaction des isothiocyanates de formule R₁-NCS sur des hydrazides de formule R₂-C(O)-NH-NH₂ dans lesquels R₁ et R₂ ont la signification indiquée ci-dessus, pour obtenir les composés de formule (5) 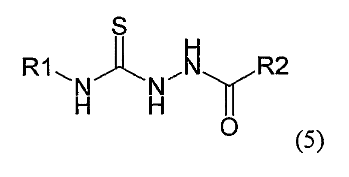 composés de formule (5) qui peuvent être soumis à un traitement basique pour obtenir les composés correspondants de formule (6) 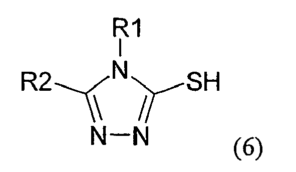 composés de formule (6) que l'on fait réagir avec
A) soit un composé de formule Br-(CH₂)_{n'}[Q']_{p'}[C(X')(Y')]_{m'}Z' où n' = 1, p' = m' = 0 et Z' a la signification indiquée ci-dessus pour obtenir, après déprotection de la fonction amine présente sur la molécule, le composé de formule (I) correspondant,
B) soit un composé de formule Br-(CH₂)_{n'}[Q']_{p'}[C(X')(Y')]_{m'}Z' où n' = 1, Q' = -C(O)-, m' = 0 et Z' a la signification indiquée ci-dessus pour obtenir, après déprotection de la fonction amine présente sur la molécule, le composé de formule (I) correspondant,
C) soit un composé de formule Br-(CH₂)_{n'}[Q']_{p'}[C(X')(Y')]_{m'}Z' où Q', X', Y', Z', n', p' et m' ont la signification indiquée ci-dessus pour obtenir, après déprotection de la fonction amine présente sur la molécule, le composé de formule (I) correspondant.

Les composés I de la présente invention possèdent d'intéressantes propriétés pharmacologiques. C'est ainsi que l'on a découvert que les composés I de la présente invention ont une haute affinité pour un (ou plusieurs) des récepteurs de la somatostatine. Ils peuvent être utilisés comme agonistes ou antagonistes non-peptidiques de la somatostatine de manière sélective ou non.

Les composés de la présente invention peuvent ainsi être utilisés dans différentes applications thérapeutiques. Ils peuvent avantageusement être utilisés pour traiter les états pathologiques ou les maladies tels que présentés ci-dessus et dans lesquels un (ou plusieurs) des récepteurs de la somatostatine est (sont) impliqué(s).

On trouvera ci-après, dans la partie expérimentale, une illustration des propriétés pharmacologiques des composés de l'invention.

Les composés de l'invention sont également des analogues de l'urotensine II et sont donc particulièrement intéressants pour traiter les états pathologiques ou les maladies dans lesquels l'urotensine II est impliquée.

La présente demande a également pour objet des compositions pharmaceutiques contenant, à titre de principe actif, au moins un des produits de formule I telle que définie ci-dessus ainsi que les sels d'addition avec les acides minéraux ou organiques pharmaceutiquement acceptables desdits produits de formule I, en association avec un support pharmaceutiquement acceptable.

Les composés de formule I dans laquelle soit R₁ représente (CH₂)₂-W et W représente morpholino ou pipérazinyl, R₂ phényl, m-chlorophényl ou 4-pyridyl, et R₃ l'atome d'hydrogène, soit R₁ représente (CH₂)₂-W et W représente pyrrolidinyl, R₂ p-chlorophényl et R₃ l'atome d'hydrogène, ont été décrits dans Phosphorus.Sulfur and Silicon, 2000, vol.164 pp.67-81, mais uniquement comme intermédiaires de synthèse et aucune activité thérapeutique n'a été envisagée pour ces composés. D'autres dérivés triazole ont été décrits dans la littérature mais soit dans des domaines techniques différents de celui de la présente demande soit sans indication d'activité thérapeutique (J. Heterocycl. Chem., 30, 1, 1993, 169-172 ; J. Pharm. Sci., 72, 1, 1983, 45-50 ; J. Med. Chem., 13, 1970, 672 ; Chem. Pharm. Bull., 23, 1975, 955-959 ; J. Heterocycl. Chem., 17, 1980, 1087-1088 ; J. Heterocycl. Chem., 27, 6, 1990, 1689-1696 ; Angew. Chem., 93, 9, 1981, 835-836 ; J. Chem. Soc., 1952, 4811-4817 ; JP10104784 ; JP3163443 ; JP3091737 ; JP2278253 ; JP1093739 ; EP249239 ; DE 3500628).

La présente invention a donc également pour objet une composition pharmaceutique pour une utilisation comme médicament et contenant, à titre de principe actif, en association avec un support pharmaceutiquement acceptable, au moins un composés de formule générale 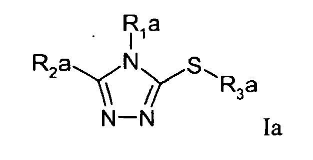 sous forme racémique, d'énantiomère ou toutes combinaisons de ces formes, dans laquelle

R₁ₐ représente un radical de formule -(CH₂)ₙ-[Q]ₚ-(CH₂)ₘ-NXY dans laquelle
X et Y représentent, indépendamment, l'atome d'hydrogène, (C₁-C₆)alkyle ;
p et n représentent 0, et m représente un entier de 2 à 6,
et les deux autres radicaux R₂ₐ et R₃ₐ représentent, indépendamment, un radical de formule -(CH₂)_{n'}[Q']_{p'}[C(X')(Y')]_{m'}Z' dans laquelle
Q' représente -O-, -S-, -C(O)-, -NH-, -CH=CH- ou -C≡C- ;
X', Y' et Z' représentent, indépendamment, un atome d'hydrogène, (C₁-C₆)alkyle, (C₁-C₆)alkoxy, (C₁-C₆)alkoxy-carbonyle, cyano, amino, (C₁-C₆)alkylamino, di((C₁-C₆)alkyl)amino, (C₃-C₇)cycloalkyle, hétérocycloalkyle, aryle ou hétéroaryle, ou bien un radical de formule 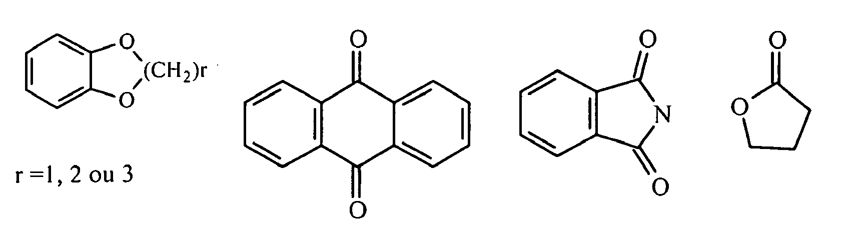 les radicaux (C₃-C₇)cycloalkyle, hétérocycloalkyle, aryle et hétéroaryle étant éventuellement substitués par un ou plusieurs substituants identiques ou différents choisis parmi : -(CH₂)_{q'}-X"-Y", hydroxy, halo, nitro, cyano, amino, (C₁-C₆)alkylamino et di((C₁-C₆)alkyl)amino ;
X" représente -O-, -S-, -C(O)-, -C(O)-O-, -SO₂- ou une liaison covalente ;
Y" représente un radical (C₁-C₆)alkyle éventuellement substitué par un ou plusieurs radicaux halo identiques ou différents ; ou aryle ou hétéroaryle éventuellement substitué par un ou plusieurs substituants identiques ou différents choisis parmi : (C₁-C₆)alkoxy, hydroxy, halo, nitro cyano, amino, (C₁-C₆)alkylamino et di((C₁-C₆)alkyl)amino ;
p' représente 0 ou 1, et n', m' et q' représentent, indépendamment, un entier de 0 à 6 ;
ou leurs sels d'addition avec les acides minéraux ou organiques pharmaceutiquement acceptables, en association avec un support pharmaceutiquement acceptable.

Une composition pharmaceutique selon l'invention peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale :

D'autres composés selon l'invention obtenus selon les procédures des exemples A, B, C et D précédemment décrites, sont rassemblés dans le tableau ci-dessous.

Les composés sont caractérisés par leur temps de rétention (tr), exprimé en minute, déterminé par chromatographie liquide (LC) et leur pic moléculaire (M + H)⁺ déterminé par spectrométrie de masse (SM). Pour la spectrométrie de masse, un spectromètre de masse simple quadripôle (Micromass, modèle Platform) équipé d'une source électrospray est utilisé avec une résolution de 0,8 Da à 50 % de vallée.

Les conditions pour les exemples présentés, sont les suivantes :
Eluant : A : Eau + 0,02 % acide trifluoroacétique ; B : Acétonitrile

**Condition 1 (C1) :**

| T (min) | A (%) | B (%) |
|---|---|---|
| 0 | 95 | 5 |
| 8,5 | 10 | 90 |
| 10,5 | 10 | 90 |
| 10,6 | 95 | 5 |
| 15 | 95 | 5 |

Débit : 1,0 ml/min
Injection : 10 µl
Température ambiante
Longueur d'onde (% UV) : 220 nm
Colonne : Uptisphere HDO 3 µm 75 * 4,6 mm i.d.

**Condition 2 (C2) :**

| T (min) | A (%) | B (%) |
|---|---|---|
| 0 | 100 | 0 |
| 6 | 20 | 80 |
| 8 | 20 | 80 |
| 8,1 | 100 | 0 |
| 10 | 100 | 0 |

Débit: 1,0 ml/min
Injection : 5 µl
Température ambiante
Longueur d'onde (% UV) : 220 nm
Colonne : Uptisphere ODS 3 µm 50 * 4,6 mm i.d

Les conditions suivant les exemples, sont les suivantes :

| Exemples | Conditions | Exemples | Conditions | Exemples | Conditions |
|---|---|---|---|---|---|
| 1 à 15 | 1 | 163 à 164 | 1 | 374 à 466 | 1 |
| 16 à 30 | 2 | 165 à 191 | 2 | 467 à 489 | 2 |
| 31 à 45 | 1 | 192 à 210 | 1 | 490 | 1 |
| 46 à 59 | 2 | 211 à 213 | 2 | 491 à 495 | 2 |
| 60 | 1 | 214 | 1 | 496 à 533 | 1 |
| 61 à 81 | 2 | 215 à 234 | 2 | 534 à 537 | 2 |
| 82 à 98 | 1 | 235 à 236 | 1 | 538 à 551 | 1 |
| 99 à 145 | 2 | 237 à 260 | 2 | 552 | 2 |
| 146 à 151 | 1 | 261 | 1 | 553 à 582 | 1 |
| 152 à 153 | 2 | 262 à 269 | 2 | 583 à 638 | 1 |
| 155 | 1 | 270 à 368 | 1 | 639 à 708 | 1 |
| 155 à 162 | 2 | 369 à 373 | 2 | | |

Ces exemples sont présentés pour illustrer les procédures ci-dessus et ne doivent en aucun cas être considérés comme une limite à la portée de l'invention.

Dans chaque illustration des radicaux R₁, R₂ et R₃, les radicaux X₁, X₂ et X₃ représentent, respectivement, la partie restante du composé de formule générale (I).

| | R1 | R2 | R3 | TR | MH+ |
|---|---|---|---|---|---|
| 1 | 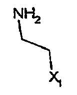 | 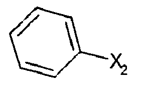 | 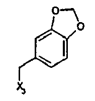 | 7,46 | 355,23 |
| 2 | 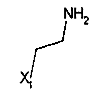 | 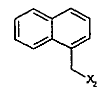 | 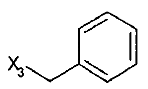 | 7,39 | 375,27 |
| 3 | 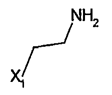 | 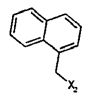 | 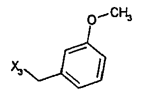 | 7,11 | 405,33 |
| 4 | 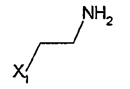 | 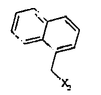 | 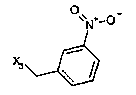 | 7,17 | 420,33 |
| 5 | 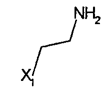 | 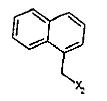 | 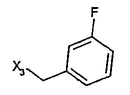 | 7,29 | 393,21 |
| 6 | 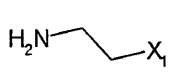 | 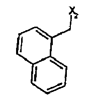 | 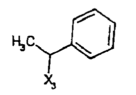 | 7,26 | 389,36 |
| 7 | 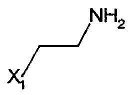 | 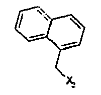 | 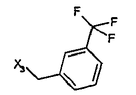 | 7,70 | 443,21 |
| 8 | 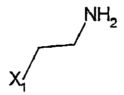 | 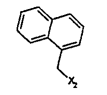 | 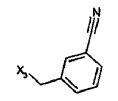 | 7,02 | 400,33 |
| 9 | 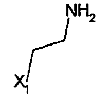 | 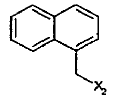 | 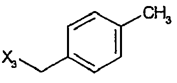 | 7,30 | 389,29 |
| 10 | 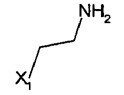 | 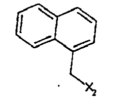 | 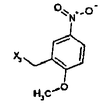 | 7,22 | 450,35 |
| 11 | 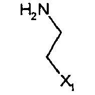 | 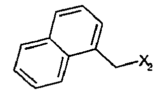 | 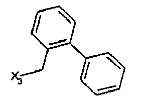 | 7,72 | 451,36 |
| 12 | 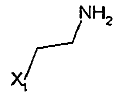 | 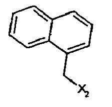 | 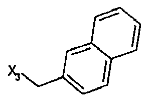 | 7,50 | 425,35 |
| 13 | 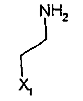 | 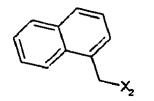 | 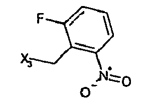 | 7,14 | 438,32 |
| 14 | 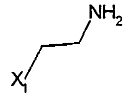 | 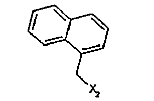 | 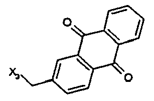 | 7,56 | 505,32 |
| 15 | 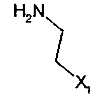 | 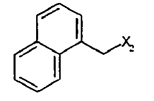 | 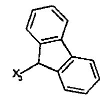 | 7,75 | 449,37 |
| 16 | 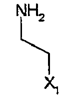 | 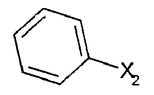 | 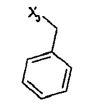 | 3,71 | 311,24 |
| 17 | 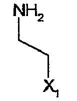 | 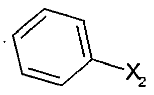 | 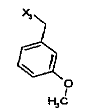 | 3,84 | 341,21 |
| 18 | 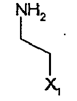 | 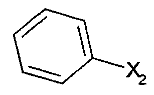 | 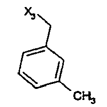 | 3,97 | 325,24 |
| 19 | 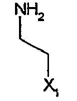 | 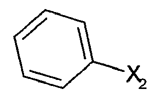 | 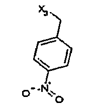 | 3,86 | 356,20 |
| 20 | 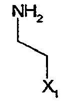 | 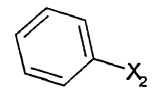 | 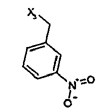 | 3,82 | 356,20 |
| 21 | 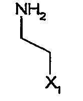 | 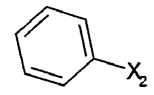 | 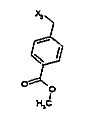 | 3,82 | 369,21 |
| 22 | 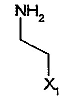 | 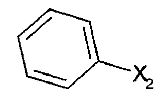 | 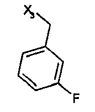 | 3,82 | 329,20 |
| 23 | 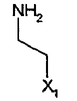 | 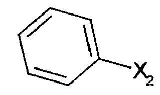 | 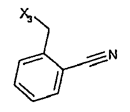 | 3,62 | 336,22 |
| 24 | 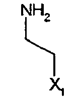 | 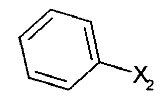 | 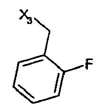 | 3,79 | 329,17 |
| 25 | 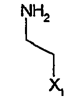 | 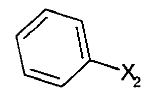 | 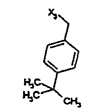 | 4,60 | 367,20 |
| 26 | 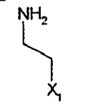 | 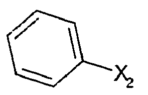 | 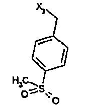 | 3,37 | 389,16 |
| 27 | 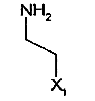 | 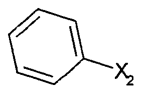 | 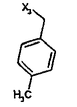 | 3,99 | 325,25 |
| 28 | 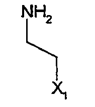 | 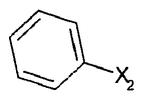 | 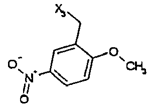 | 3,90 | 386,10 |
| 29 | 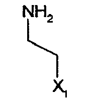 | 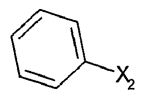 | 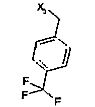 | 4,36 | 379,18 |
| 30 | 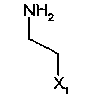 | 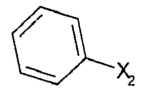 | 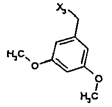 | 3,90 | 371,23 |
| 31 | 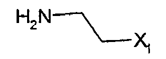 | 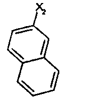 | 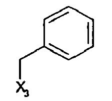 | 7,31 | 361,28 |
| 32 | 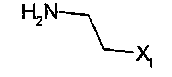 | 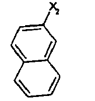 | 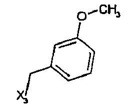 | 7,03 | 391,31 |
| 33 | 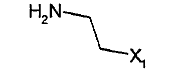 | 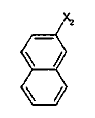 | 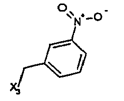 | 7,09 | 406,32 |
| 34 | 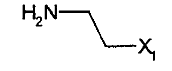 | 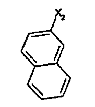 | 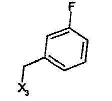 | 7,21 | 379,21 |
| 35 | 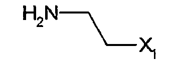 | 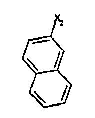 | 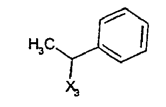 | 7,11 | 375,38 |
| 36 | 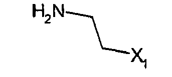 | 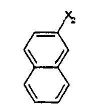 | 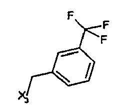 | 7,61 | 429,18 |
| 37 | 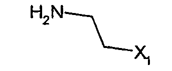 | 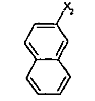 | 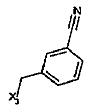 | 6,92 | 386,32 |
| 38 | 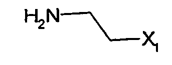 | 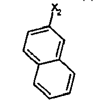 | 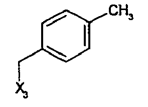 | 7,27 | 375,30 |
| 39 | 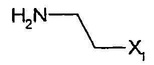 | 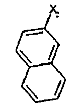 | 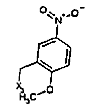 | 7,13 | 436,32 |
| 40 | 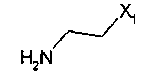 | 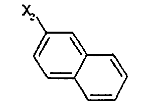 | 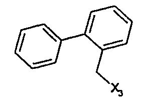 | 7,64 | 437,33 |
| 41 | 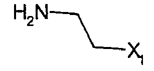 | 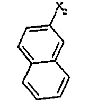 | 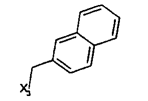 | 7,41 | 411,33 |
| 42 | 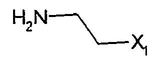 | 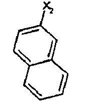 | 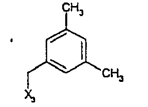 | 7,45 | 389,37 |
| 43 | 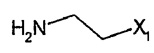 | 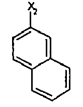 | 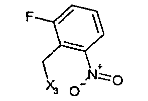 | 7,04 | 424,30 |
| 44 | 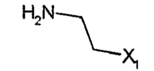 | 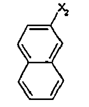 | 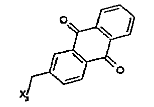 | 7,48 | 491,29 |
| 45 | 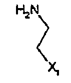 | 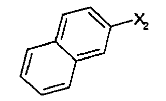 | 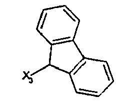 | 7,73 | 435,35 |
| 46 | 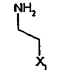 | 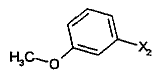 | 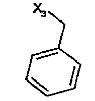 | 3,87 | 341,25 |
| 47 | 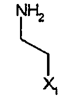 | 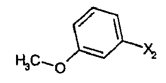 | 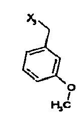 | 3,95 | 371,28 |
| 48 | 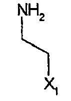 | 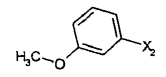 | 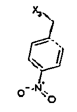 | 4,02 | 386,18 |
| 49 | 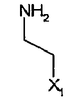 | 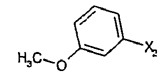 | 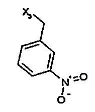 | 3,98 | 386,18 |
| 50 | 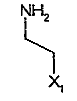 | 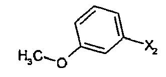 | 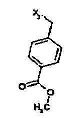 | 3,96 | 399,20 |
| 51 | 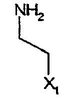 | 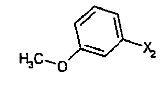 | 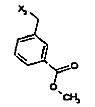 | 3,96 | 399,20 |
| 52 | 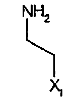 | 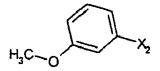 | 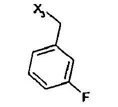 | 3,98 | 359,21 |
| 53 | 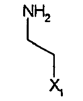 | 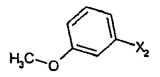 | 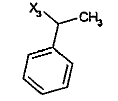 | 4,02 | 355,24 |
| 54 | 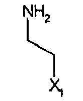 | 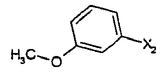 | 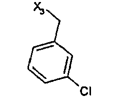 | 4,20 | 375,18 |
| 55 | 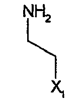 | 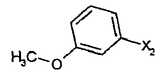 | 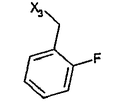 | 3,90 | 359,20 |
| 56 | 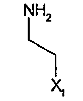 | 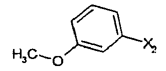 | 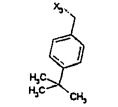 | 4,83 | 397,26 |
| 57 | 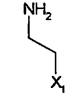 | 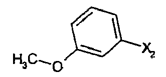 | 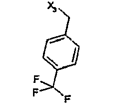 | 4,49 | 409,17 |
| 58 | 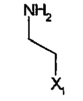 | 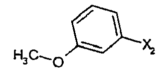 | 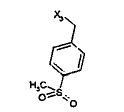 | 3,54 | 419,23 |
| 59 | 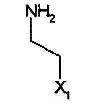 | 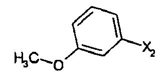 | 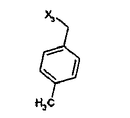 | 4,14 | 355,24 |
| 60 | 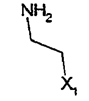 | 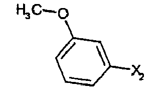 | 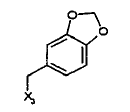 | 7,57 | 385,23 |
| 61 | 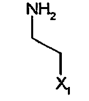 | 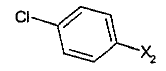 | 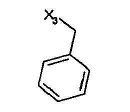 | 4,09 | 345,08 |
| 62 | 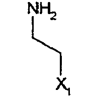 | 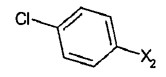 | 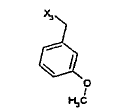 | 4,16 | 375,05 |
| 63 | 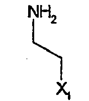 | 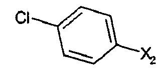 | 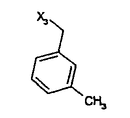 | 4,31 | 359,06 |
| 64 | 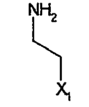 | 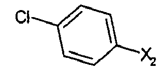 | 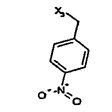 | 4,23 | 390,03 |
| 65 | 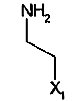 | 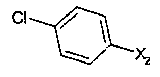 | 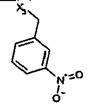 | 4,19 | 390,04 |
| 66 | 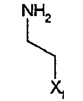 | 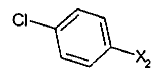 | 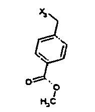 | 4,20 | 403,19 |
| 67 | 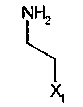 | 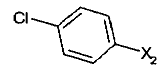 | 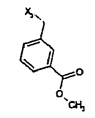 | 4,17 | 403,02 |
| 68 | 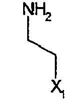 | 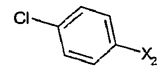 | 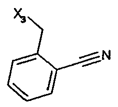 | 4,02 | 370,06 |
| 69 | 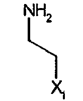 | 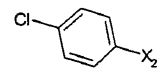 | 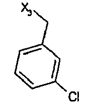 | 4,43 | 379,16 |
| 70 | 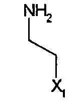 | 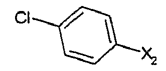 | 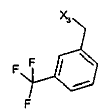 | 4,62 | 413,00 |
| 71 | 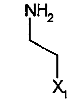 | 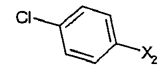 | 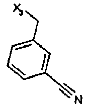 | 4,03 | 370,06 |
| 72 | 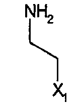 | 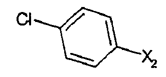 | 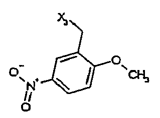 | 4,25 | 420,05 |
| 73 | 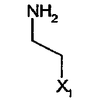 | 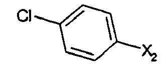 | 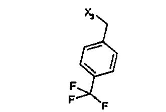 | 4,68 | 413,00 |
| 74 | 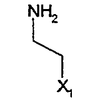 | 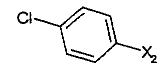 | 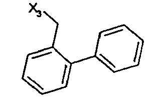 | 4,83 | 421,07 |
| 75 | 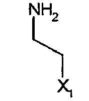 | 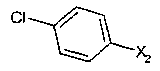 | 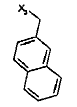 | 4,59 | 395,04 |
| 76 | 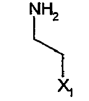 | 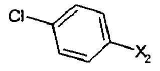 | 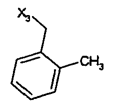 | 4,26 | 359,06 |
| 77 | 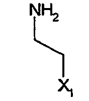 | 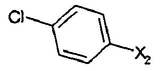 | 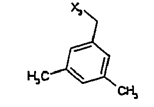 | 4,55 | 373,07 |
| 78 | 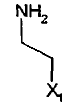 | 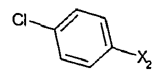 | 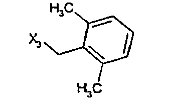 | 4,40 | 377,10 |
| 79 | 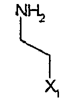 | 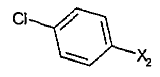 | 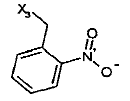 | 4,12 | 390,04 |
| 80 | 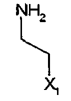 | 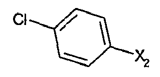 | 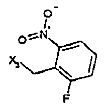 | 4,08 | 407,99 |
| 81 | 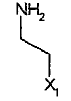 | 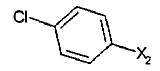 | 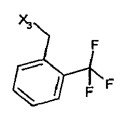 | 4,54 | 413,17 |
| 82 | 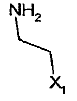 | 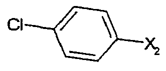 | 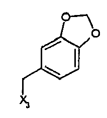 | 7,74 | 389,16 |
| 83 | 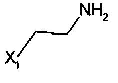 | 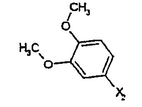 | 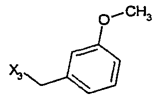 | 6,55 | 401,32 |
| 84 | 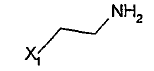 | 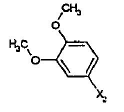 | 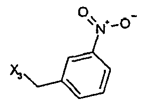 | 6,62 | 416,31 |
| 85 | 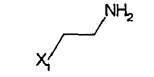 | 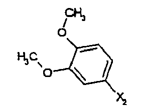 | 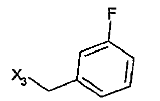 | 6,70 | 389,20 |
| 86 | 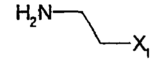 | 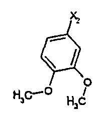 | 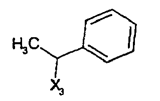 | 6,64 | 385,37 |
| 87 | 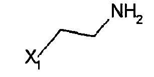 | 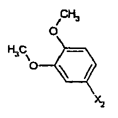 | 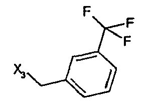 | 7,11 | 439,21 |
| 88 | 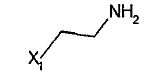 | 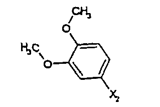 | 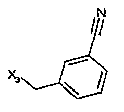 | 6,47 | 396,30 |
| 89 | 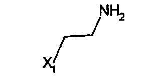 | 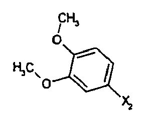 | 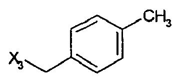 | 6,79 | 385,29 |
| 90 | 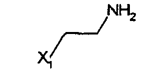 | 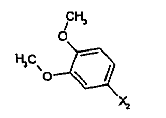 | 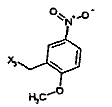 | 6,68 | 446,32 |
| 91 | 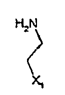 | 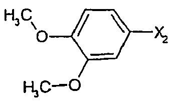 | 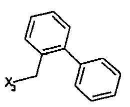 | 7,21 | 447,33 |
| 92 | 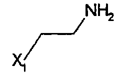 | 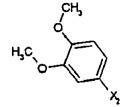 | 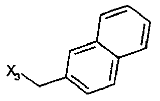 | 6,96 | 421,32 |
| 93 | 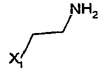 | 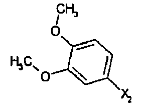 | 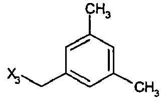 | 6,97 | 399,37 |
| 94 | 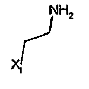 | 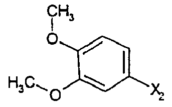 | 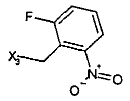 | 6,57 | 434,30 |
| 95 | 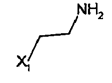 | 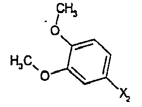 | 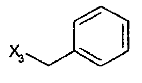 | 6,73 | 371,28 |
| 96 | 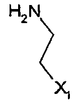 | 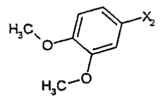 | 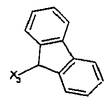 | 7,23 | 445,36 |
| 97 | 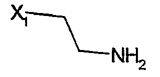 | 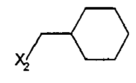 | 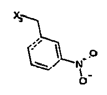 | 6,99 | 376,33 |
| 98 | 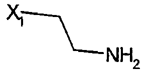 | 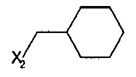 | 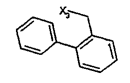 | 7,65 | 407,36 |
| 99 | 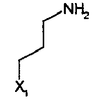 | 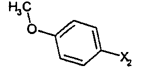 | 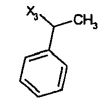 | 4,18 | 369,31 |
| 100 | 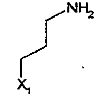 | 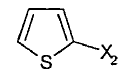 | 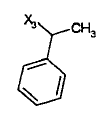 | 4,07 | 345,27 |
| 101 | 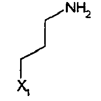 | 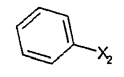 | 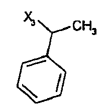 | 4,08 | 339,31 |
| 102 | 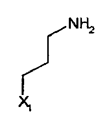 | 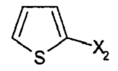 | 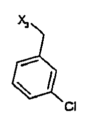 | 4,21 | 365,21 |
| 103 | 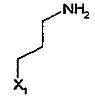 | 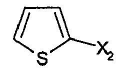 | 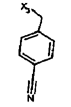 | 3,82 | 356,23 |
| 104 | 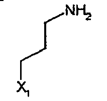 | 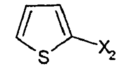 | 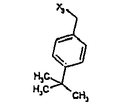 | 4,87 | 387,26 |
| 105 | 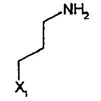 | 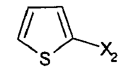 | 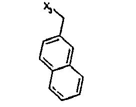 | 4,45 | 381,23 |
| 106 | 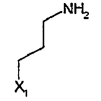 | 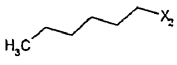 | 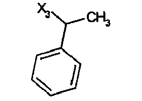 | 4,61 | 347,36 |
| 107 | 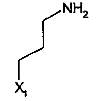 | 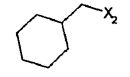 | 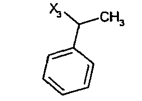 | 4,55 | 359,34 |
| 108 | 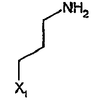 | 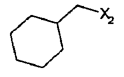 | 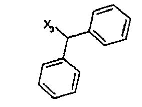 | 5,01 | 421,33 |
| 109 | 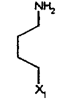 | 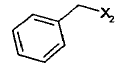 | 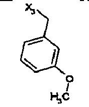 | 3,54 | 383,19 |
| 110 | 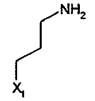 | 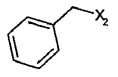 | 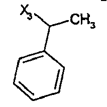 | 4,23 | 353,31 |
| 111 | 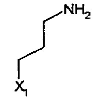 | 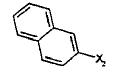 | 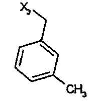 | 4,65 | 389,27 |
| 112 | 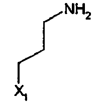 | 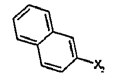 | 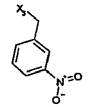 | 4,51 | 420,24 |
| 113 | 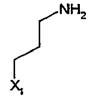 | 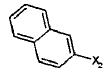 | 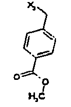 | 4,47 | 433,25 |
| 114 | 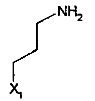 | 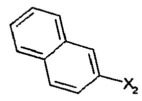 | 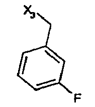 | 4,53 | 393,24 |
| 115 | 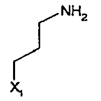 | 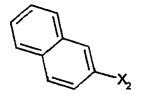 | 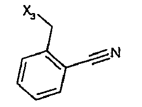 | 4,33 | 400,25 |
| 116 | 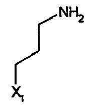 | 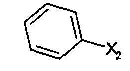 | 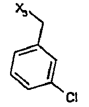 | 4,22 | 359,22 |
| 117 | 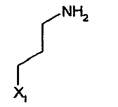 | 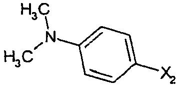 | 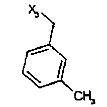 | 4,34 | 382,30 |
| 118 | 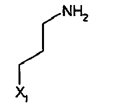 | 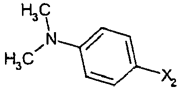 | 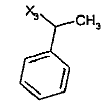 | 4,27 | 382,30 |
| 119 | 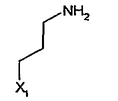 | 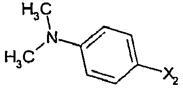 | 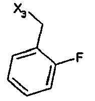 | 4,14 | 386,29 |
| 120 | 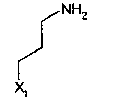 | 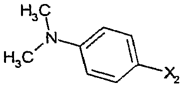 | 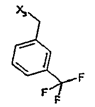 | 4,66 | 436,29 |
| 121 | 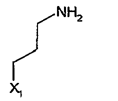 | 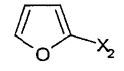 | 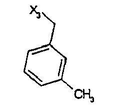 | 4,13 | 329,29 |
| 122 | 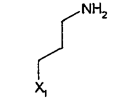 | 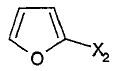 | 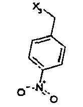 | 3,96 | 360,27 |
| 123 | 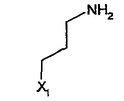 | 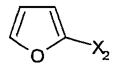 | 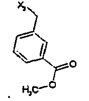 | 3,94 | 373,26 |
| 124 | 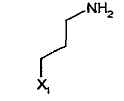 | 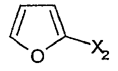 | 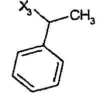 | 4,04 | 329,29 |
| 125 | 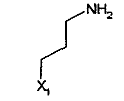 | 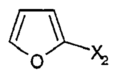 | 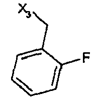 | 3,91 | 333,27 |
| 126 | 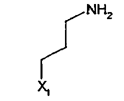 | 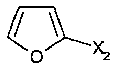 | 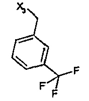 | 4,47 | 383,24 |
| 127 | 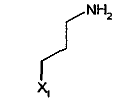 | 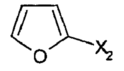 | 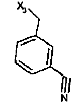 | 3,78 | 340,29 |
| 128 | 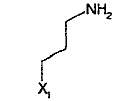 | 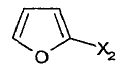 | 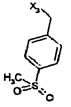 | 3,47 | 393,23 |
| 129 | 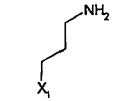 | 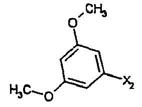 | 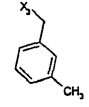 | 4,45 | 399,31 |
| 130 | 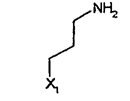 | 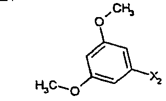 | 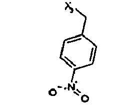 | 4,35 | 430,30 |
| 131 | 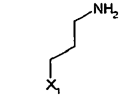 | 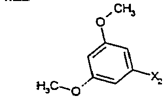 | 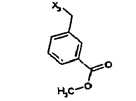 | 4,29 | 443,32 |
| 132 | 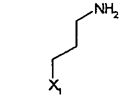 | 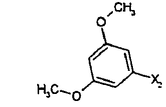 | 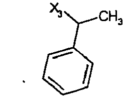 | 4,37 | 399,31 |
| 133 | 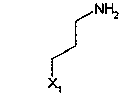 | 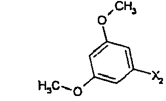 | 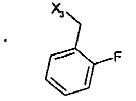 | 4,26 | 403,27 |
| 134 | 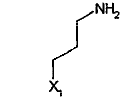 | 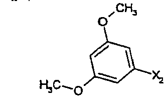 | 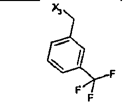 | 4,76 | 453,29 |
| 135 | 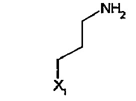 | 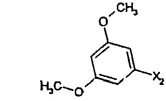 | 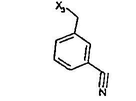 | 4,16 | 410,28 |
| 136 | 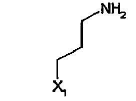 | 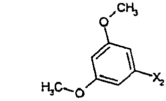 | 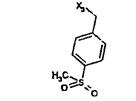 | 3,90 | 463,28 |
| 137 | 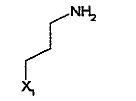 | 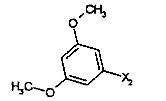 | 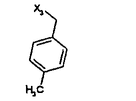 | 4,48 | 399,31 |
| 138 | 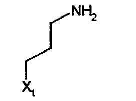 | 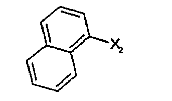 | 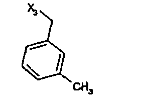 | 4,57 | 389,31 |
| 139 | 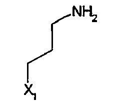 | 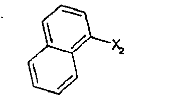 | 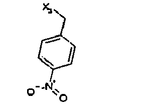 | 4,46 | 420,28 |
| 140 | 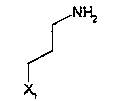 | 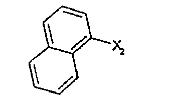 | 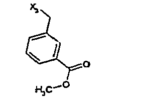 | 4,40 | 433,29 |
| 141 | 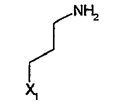 | 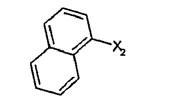 | 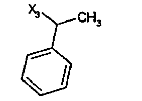 | 4,51 | 389,31 |
| 142 | 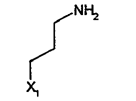 | 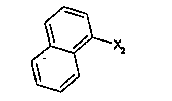 | 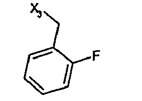 | 4,39 | 393,28 |
| 143 | 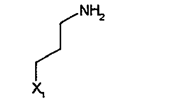 | 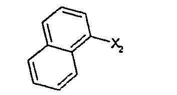 | 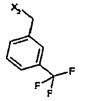 | 4,87 | 443,30 |
| 144 | 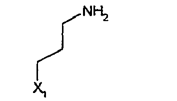 | 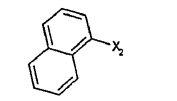 | 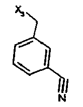 | 4,28 | 400,28 |
| 145 | 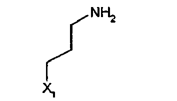 | 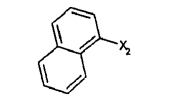 | 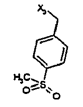 | 4,01 | 453,26 |
| 146 | 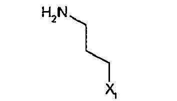 | 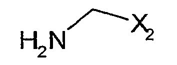 | 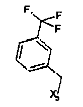 | 6,11 | 346,28 |
| 147 | 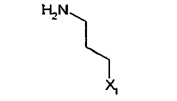 | 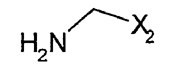 | 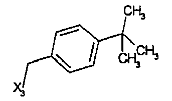 | 6,50 | 334,35 |
| 148 | 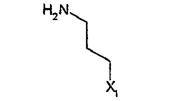 | 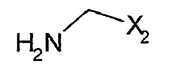 | 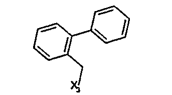 | 6,37 | 354,30 |
| 149 | 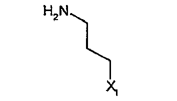 | 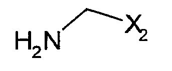 | 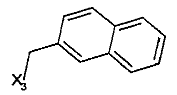 | 6,17 | 328,30 |
| 150 | 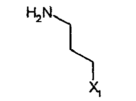 | 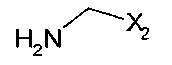 | 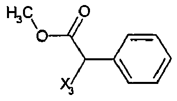 | 5,73 | 336,28 |
| 151 | 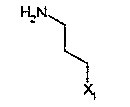 | 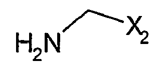 | 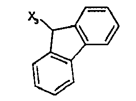 | 6,30 | 352,30 |
| 152 | 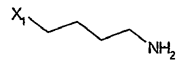 | 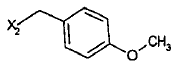 | 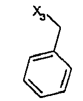 | 3,52 | 383,19 |
| 153 | 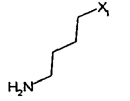 | 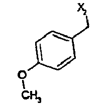 | 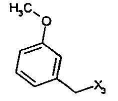 | 3,58 | 413,18 |
| 154 | 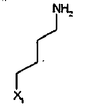 | 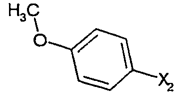 | 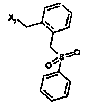 | 7,99 | 523.30 |
| 155 | 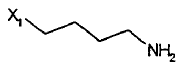 | 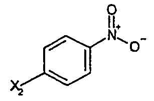 | 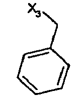 | 3,60 | 384,15 |
| 156 | 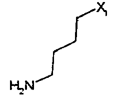 | 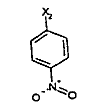 | 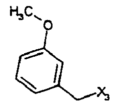 | 3,66 | 414,14 |
| 157 | 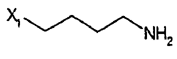 | 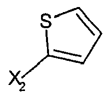 | 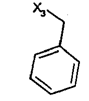 | 3,35 | 345,16 |
| 158 | 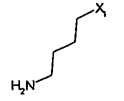 | 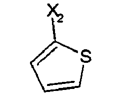 | 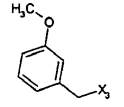 | 3,40 | 375,14 |
| 159 | 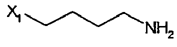 | 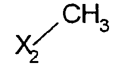 | 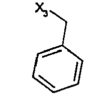 | 2,74 | 277,19 |
| 160 | 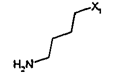 | 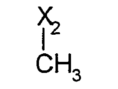 | 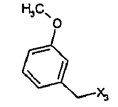 | 2,84 | 307,18 |
| 161 | 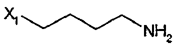 | 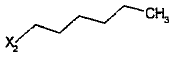 | 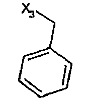 | 3,93 | 347,26 |
| 162 | 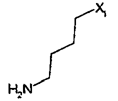 | 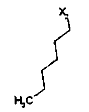 | 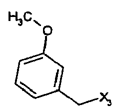 | 3,94 | 377,25 |
| 163 | 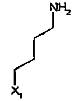 | 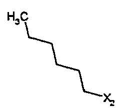 | 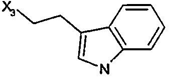 | 8,04 | 400,37 |
| 164 | 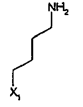 | 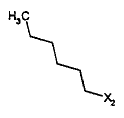 | 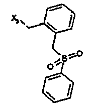 | 8,25 | 501,33 |
| 165 | 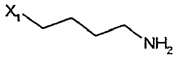 | 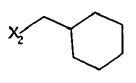 | 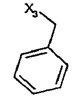 | 3,85 | 359,25 |
| 166 | 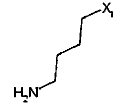 | 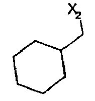 | 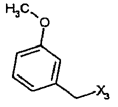 | 3,87 | 389,25 |
| 167 | 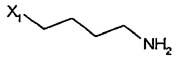 | 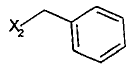 | 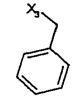 | 3,50 | 353,21 |
| 168 | 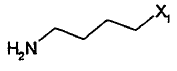 | 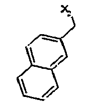 | 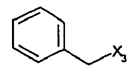 | 3,94 | 403,18 |
| 169 | 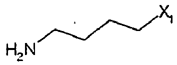 | 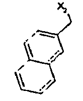 | 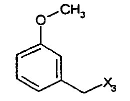 | 3,98 | 433,21 |
| 170 | 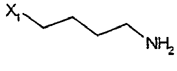 | 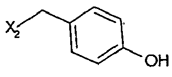 | 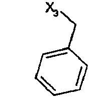 | 3,04 | 369,20 |
| 171 | 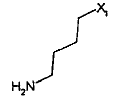 | 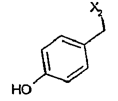 | 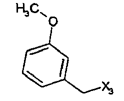 | 3,12 | 399,18 |
| 172 | 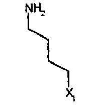 | 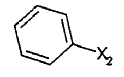 | 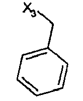 | 3,90 | 339,20 |
| 173 | 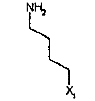 | 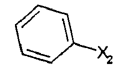 | 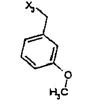 | 4,07 | 369,16 |
| 174 | 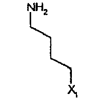 | 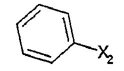 | 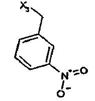 | 4,08 | 384,10 |
| 175 | 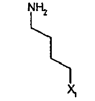 | 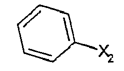 | 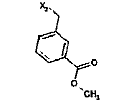 | 4,08 | 397,11 |
| 176 | 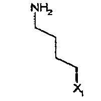 | 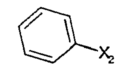 | 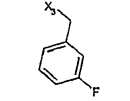 | 4,12 | 357,14 |
| 177 | 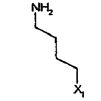 | 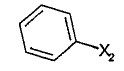 | 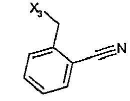 | 3,90 | 364,13 |
| 178 | 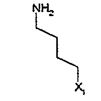 | 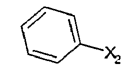 | 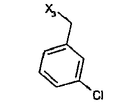 | 4,32 | 373,08 |
| 179 | 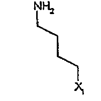 | 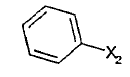 | 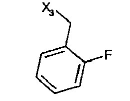 | 4,06 | 357,15 |
| 180 | 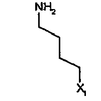 | 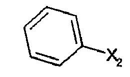 | 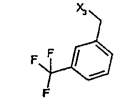 | 4,54 | 407,25 |
| 181 | 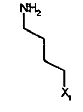 | 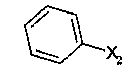 | 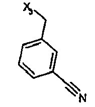 | 3,94 | 364,30 |
| 182 | 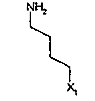 | 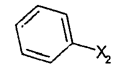 | 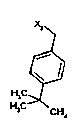 | 4,94 | 395,17 |
| 183 | 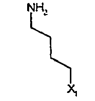 | 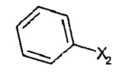 | 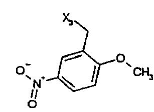 | 4,14 | 414,09 |
| 184 | 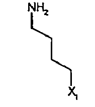 | 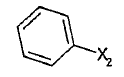 | 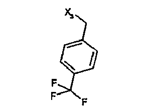 | 4,58 | 407,08 |
| 185 | 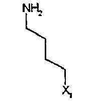 | 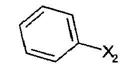 | 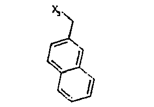 | 4,51 | 389,12 |
| 186 | 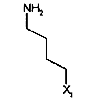 | 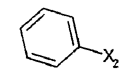 | 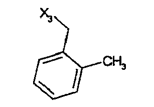 | 4,22 | 353,17 |
| 187 | 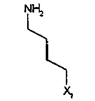 | 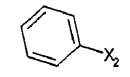 | 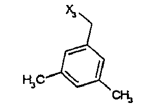 | 4,51 | 367,17 |
| 188 | 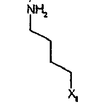 | 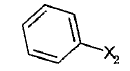 | 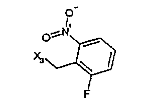 | 4,00 | 402,06 |
| 189 | 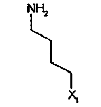 | 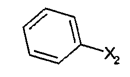 | 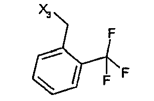 | 4,47 | 407,25 |
| 190 | 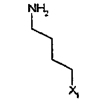 | 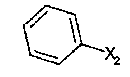 | 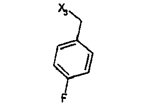 | 4,14 | 357,29 |
| 191 | 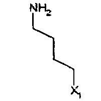 | 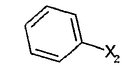 | 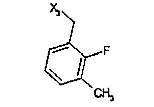 | 4,31 | 371,14 |
| 192 | 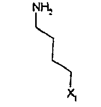 | 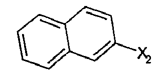 | 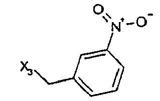 | 7,96 | 433,90 |
| 193 | 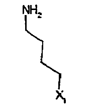 | 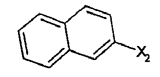 | 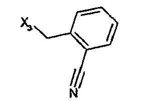 | 7,80 | 413,93 |
| 194 | 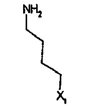 | 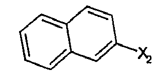 | 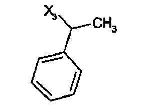 | 8,01 | 402,95 |
| 195 | 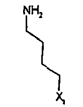 | 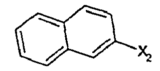 | 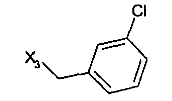 | 8,15 | 422,88 |
| 196 | 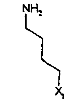 | 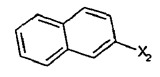 | 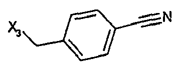 | 7,83 | 413,93 |
| 197 | 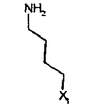 | 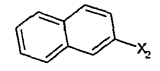 | 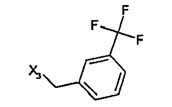 | 8,28 | 456,90 |
| 198 | 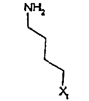 | 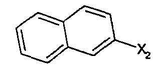 | 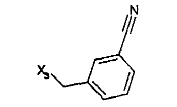 | 7,83 | 413,92 |
| 199 | 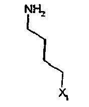 | 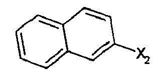 | 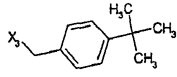 | 8,57 | 445,02 |
| 200 | 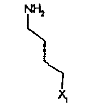 | 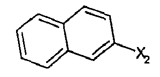 | 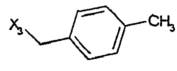 | 8,03 | 403,02 |
| 201 | 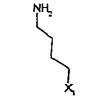 | 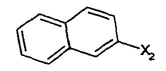 | 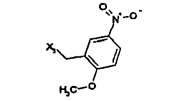 | 7,99 | 463,90 |
| 202 | 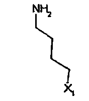 | 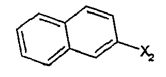 | 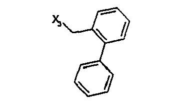 | 8,51 | 464,93 |
| 203 | 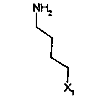 | 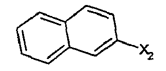 | 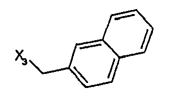 | 8,25 | 438,94 |
| 204 | 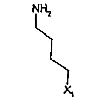 | 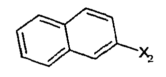 | 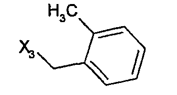 | 8,04 | 402,96 |
| 205 | 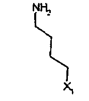 | 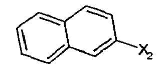 | 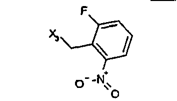 | 7,89 | 451,88 |
| 206 | 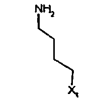 | 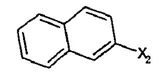 | 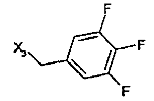 | 8,13 | 442,95 |
| 207 | 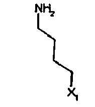 | 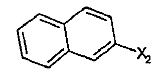 | 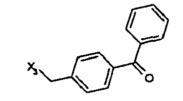 | 8,23 | 492,96 |
| 208 | 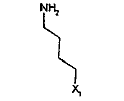 | 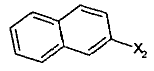 | 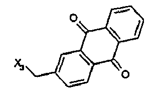 | 8,33 | 518,86 |
| 209 | 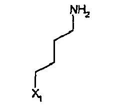 | 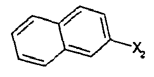 | 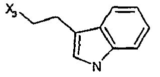 | 8,20 | 442,20 |
| 210 | 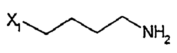 | 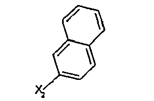 | 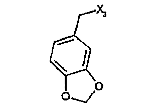 | 8,03 | 433,25 |
| 211 | 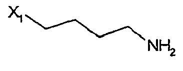 | 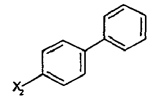 | 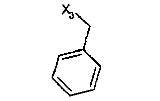 | 4,22 | 415,18 |
| 212 | 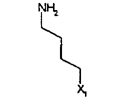 | 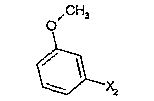 | 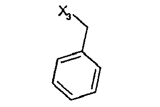 | 4,16 | 369,24 |
| 213 | 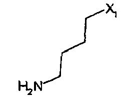 | 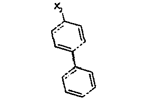 | 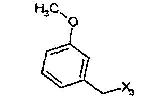 | 4,24 | 445,21 |
| 214 | 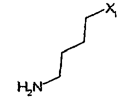 | 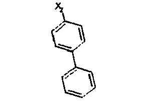 | 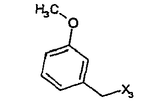 | 8,19 | 444,94 |
| 215 | 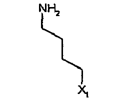 | 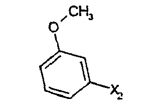 | 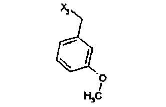 | 4,21 | 399,23 |
| 216 | 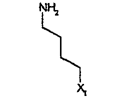 | 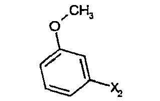 | 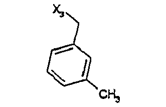 | 4,38 | 383,23 |
| 217 | 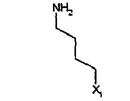 | 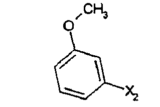 | 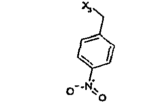 | 4,25 | 414,20 |
| 218 | 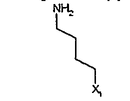 | 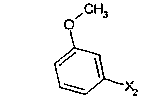 | 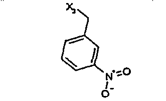 | 4,20 | 414,19 |
| 219 | 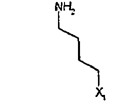 | 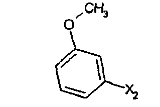 | 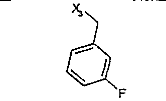 | 4,24 | 387,20 |
| 220 | 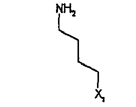 | 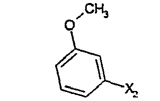 | 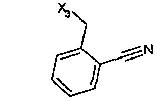 | 4,05 | 394,22 |
| 221 | 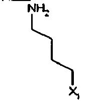 | 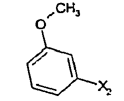 | 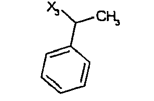 | 4,31 | 383,23 |
| 222 | 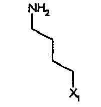 | 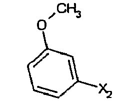 | 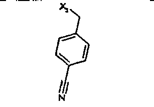 | 4,06 | 394,22 |
| 223 | 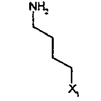 | 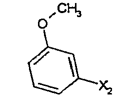 | 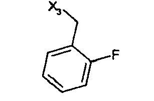 | 4,19 | 387,20 |
| 224 | 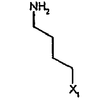 | 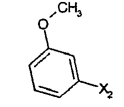 | 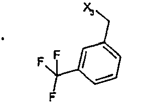 | 4,62 | 437,21 |
| 225 | 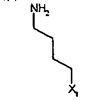 | 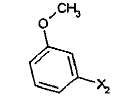 | 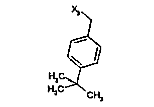 | 5,01 | 425,28 |
| 226 | 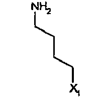 | 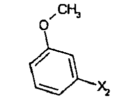 | 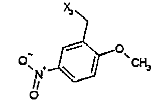 | 4,28 | 444,24 |
| 227 | 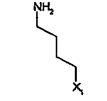 | 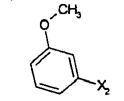 | 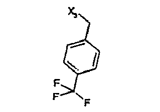 | 4,68 | 437,21 |
| 228 | 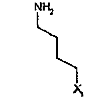 | 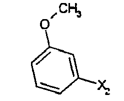 | 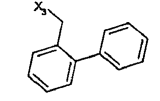 | 4,84 | 445,27 |
| 229 | 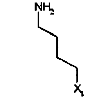 | 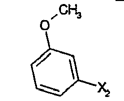 | 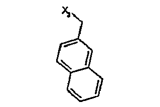 | 4,60 | 419,25 |
| 230 | 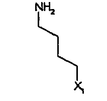 | 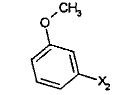 | 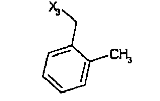 | 4,32 | 383,23 |
| 231 | 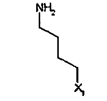 | 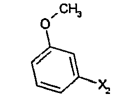 | 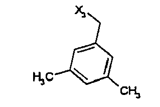 | 4,60 | 397,24 |
| 232 | 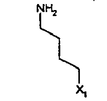 | 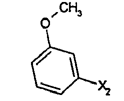 | 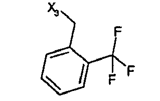 | 4,56 | 437,21 |
| 233 | 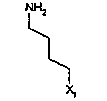 | 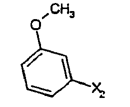 | 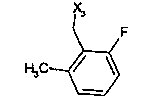 | 4,42 | 401,21 |
| 234 | 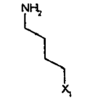 | 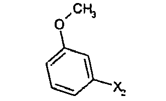 | 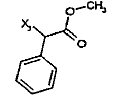 | 4,28 | 427,22 |
| 235 | 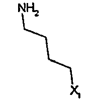 | 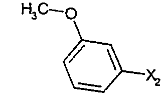 | 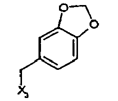 | 7,73 | 413,24 |
| 236 | 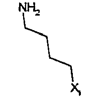 | 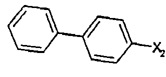 | 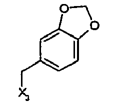 | 8,26 | 459,27 |
| 237 | 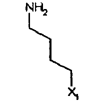 | 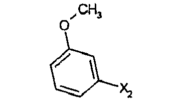 | 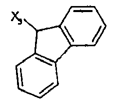 | 4,84 | 443,26 |
| 238 | 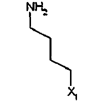 | 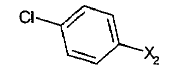 | 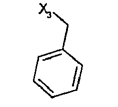 | 4,34 | 373,18 |
| 239 | 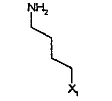 | 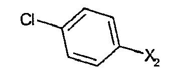 | 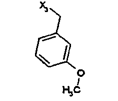 | 4,39 | 403,16 |
| 240 | 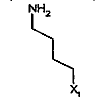 | 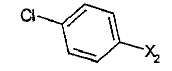 | 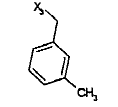 | 4,56 | 387,17 |
| 241 | 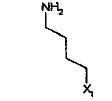 | 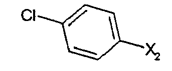 | 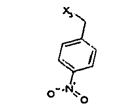 | 4,44 | 418,14 |
| 242 | 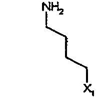 | 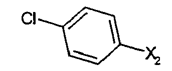 | 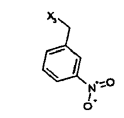 | 4,40 | 418,14 |
| 243 | 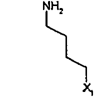 | 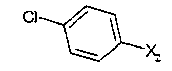 | 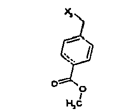 | 4,37 | 431,17 |
| 244 | 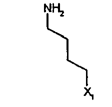 | 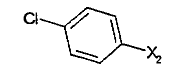 | 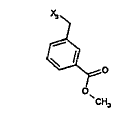 | 4,38 | 431,18 |
| 245 | 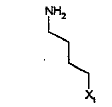 | 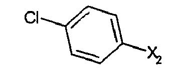 | 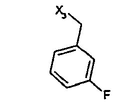 | 4,44 | 391,17 |
| 246 | 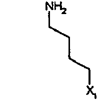 | 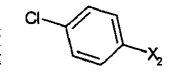 | 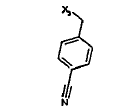 | 4,25 | 398,15 |
| 247 | 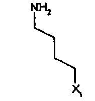 | 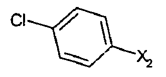 | 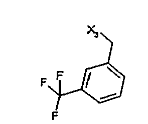 | 4,80 | 441,14 |
| 248 | 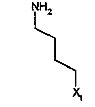 | 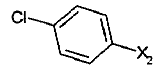 | 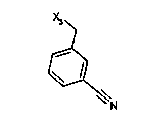 | 4,26 | 398,14 |
| 249 | 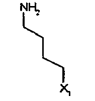 | 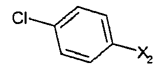 | 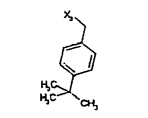 | 5,17 | 429,24 |
| 250 | 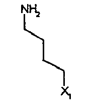 | 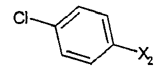 | 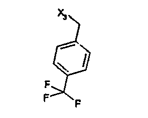 | 4,86 | 441,14 |
| 251 | 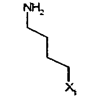 | 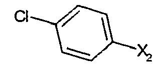 | 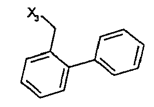 | 4,99 | 449,22 |
| 252 | 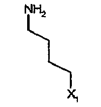 | 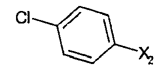 | 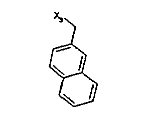 | 4,77 | 423,18 |
| 253 | 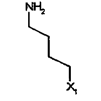 | 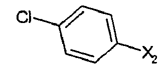 | 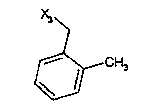 | 4,50 | 387,18 |
| 254 | 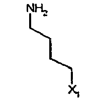 | 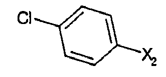 | 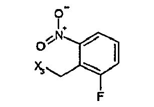 | 4,32 | 436,14 |
| 255 | 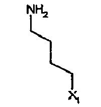 | 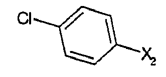 | 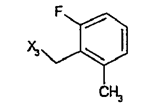 | 4,59 | 405,17 |
| 256 | 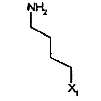 | 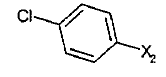 | 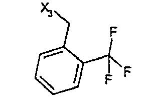 | 4,72 | 441,15 |
| 257 | 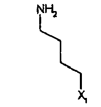 | 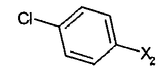 | 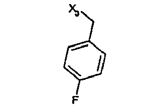 | 4,44 | 391,17 |
| 258 | 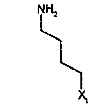 | 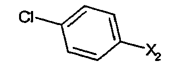 | 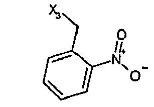 | 4,36 | 418,15 |
| 259 | 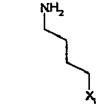 | 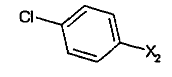 | 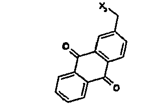 | 4,86 | 503,21 |
| 260 | 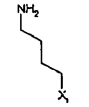 | 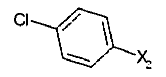 | 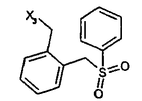 | 4,50 | 448,20 |
| 261 | 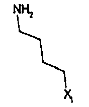 | 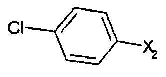 | 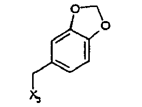 | 7,90 | 417,19 |
| 262 | 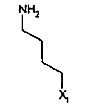 | 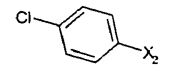 | 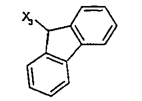 | 5,00 | 447,19 |
| 263 | 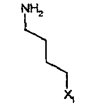 | 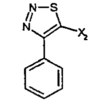 | 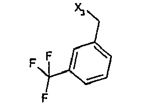 | 4,93 | 491,19 |
| 264 | 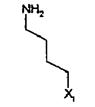 | 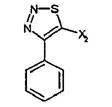 | 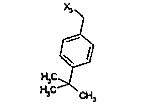 | 5,29 | 479,27 |
| 265 | 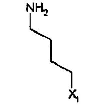 | 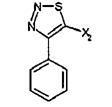 | 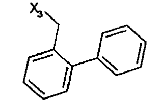 | 5,13 | 499,24 |
| 266 | 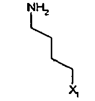 | 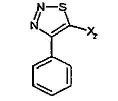 | 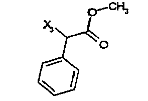 | 4,62 | 481,21 |
| 267 | 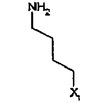 | 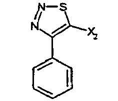 | 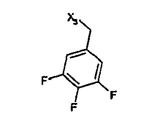 | 4,83 | 477,18 |
| 268 | 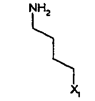 | 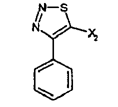 | 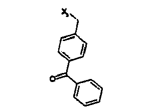 | 4,93 | 527,24 |
| 269 | 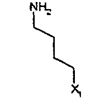 | 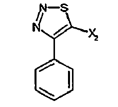 | 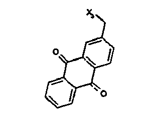 | 4,97 | 553,19 |
| 270 | 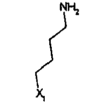 | 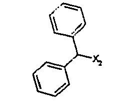 | 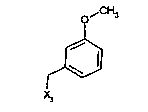 | 8,10 | 458,96 |
| 271 | 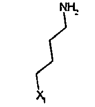 | 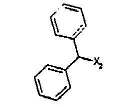 | 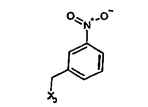 | 8,13 | 473,89 |
| 272 | 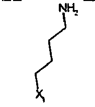 | 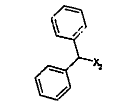 | 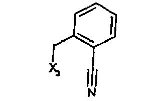 | 7,98 | 453,96 |
| 273 | 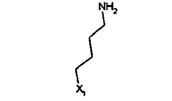 | 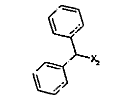 | 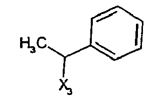 | 8,19 | 442,97 |
| 274 | 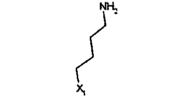 | 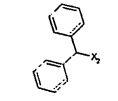 | 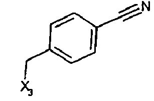 | 7,97 | 453,95 |
| 275 | 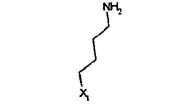 | 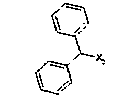 | 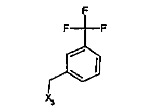 | 8,49 | 496,91 |
| 276 | 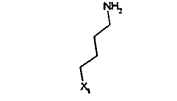 | 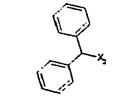 | 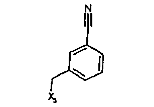 | 7,98 | 453,94 |
| 277 | 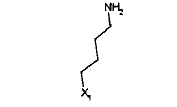 | 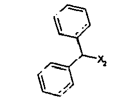 | 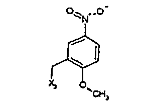 | 8,17 | 503,91 |
| 278 | 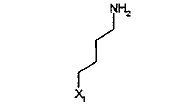 | 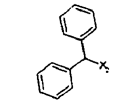 | 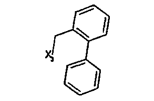 | 8,70 | 504,95 |
| 279 | 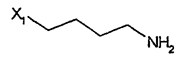 | 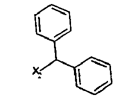 | 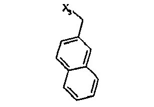 | 8,43 | 478,96 |
| 280 | 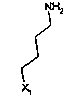 | 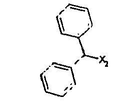 | 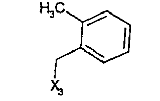 | 8,18 | 442,97 |
| 281 | 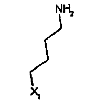 | 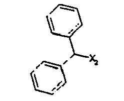 | 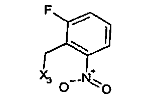 | 8,07 | 491,89 |
| 282 | 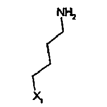 | 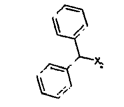 | 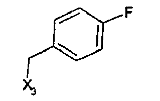 | 8,14 | 446,92 |
| 283 | 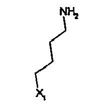 | 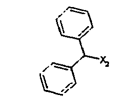 | 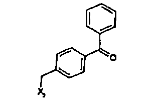 | 8,46 | 532,87 |
| 284 | 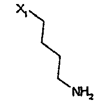 | 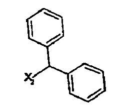 | 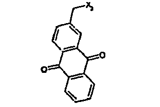 | 8,51 | 558,83 |
| 285 | 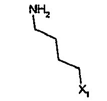 | 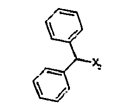 | 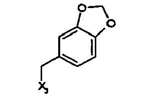 | 8,17 | 473,28 |
| 286 | 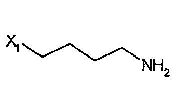 | 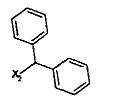 | 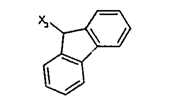 | 8,71 | 502,91 |
| 287 | 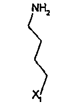 | 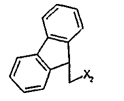 | 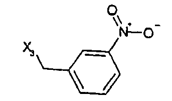 | 8,33 | 485,89 |
| 288 | 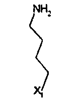 | 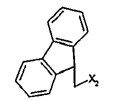 | 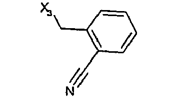 | 8,21 | 465,93 |
| 289 | 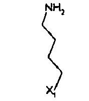 | 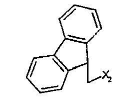 | 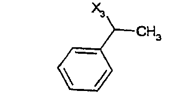 | 8,41 | 454,95 |
| 290 | 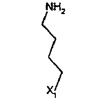 | 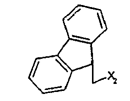 | 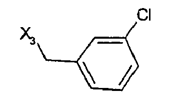 | 8,55 | 474,88 |
| 291 | 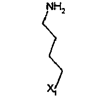 | 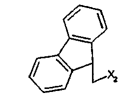 | 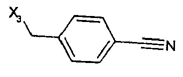 | 8,17 | 465,93 |
| 292 | 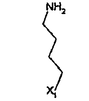 | 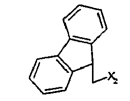 | 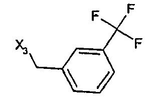 | 8,69 | 511,31 |
| 293 | 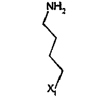 | 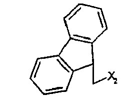 | 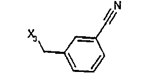 | 8,19 | 465,93 |
| 294 | 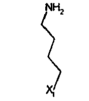 | 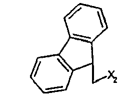 | 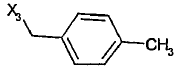 | 8,50 | 455,00 |
| 295 | 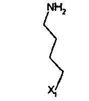 | 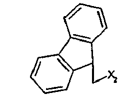 | 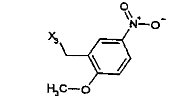 | 8,36 | 515,87 |
| 296 | 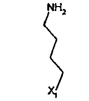 | 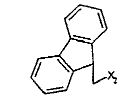 | 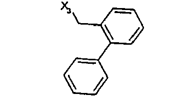 | 8,92 | 516,91 |
| 297 | 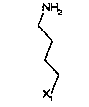 | 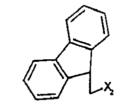 | 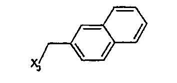 | 8,67 | 490,91 |
| 298 | 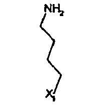 | 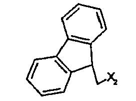 | 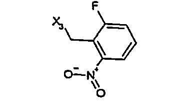 | 8,31 | 503,87 |
| 299 | 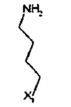 | 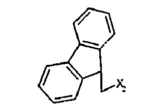 | 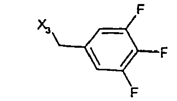 | 8,59 | 494,88 |
| 300 | 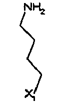 | 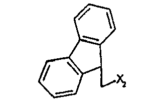 | 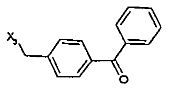 | 8,65 | 544,86 |
| 301 | 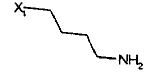 | 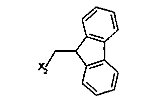 | 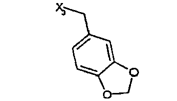 | 8,37 | 485,29 |
| 302 | 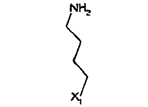 | 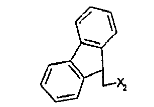 | 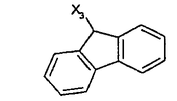 | 8,92 | 514,89 |
| 303 | 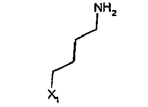 | 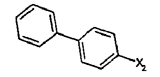 | 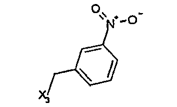 | 8,23 | 459,91 |
| 304 | 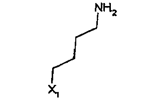 | 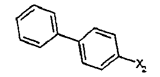 | 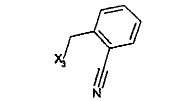 | 8,07 | 439,94 |
| 305 | 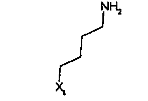 | 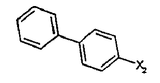 | 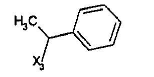 | 8,30 | 428,97 |
| 306 | 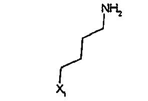 | 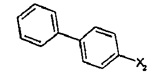 | 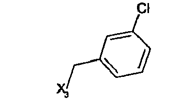 | 8,41 | 448,90 |
| 307 | 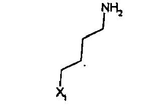 | 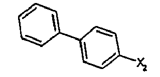 | 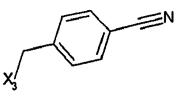 | 8,08 | 439,94 |
| 308 | 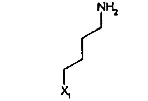 | 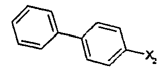 | 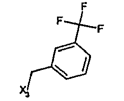 | 8,54 | 482,90 |
| 309 | 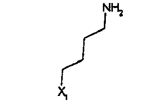 | 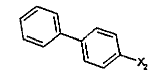 | 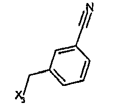 | 8,09 | 439,94 |
| 310 | 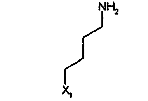 | 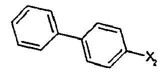 | 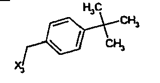 | 8,94 | 470,96 |
| 311 | 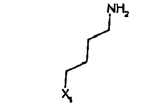 | 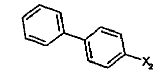 | 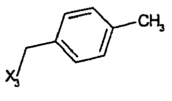 | 8,36 | 428,97 |
| 312 | 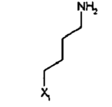 | 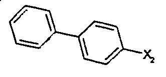 | 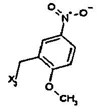 | 8,24 | 489,91 |
| 313 | 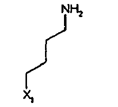 | 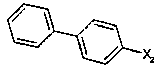 | 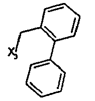 | 8,77 | 490,93 |
| 314 | 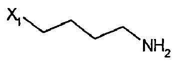 | 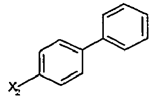 | 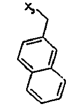 | 8,54 | 464,94 |
| 315 | 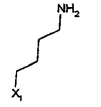 | 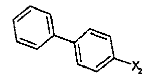 | 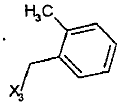 | 8,31 | 428,98 |
| 316 | 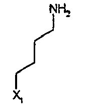 | 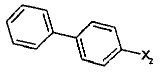 | 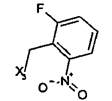 | 8,18 | 477,89 |
| 317 | 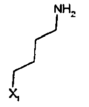 | 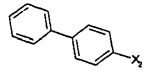 | 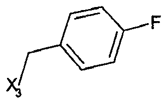 | 8,22 | 432,95 |
| 318 | 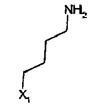 | 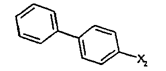 | 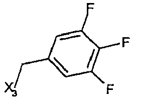 | 8,46 | 468,89 |
| 319 | 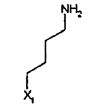 | 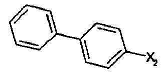 | 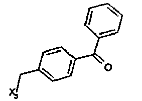 | 8,59 | 518,90 |
| 320 | 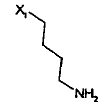 | 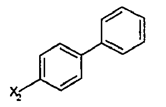 | 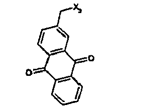 | 8,60 | 544,85 |
| 321 | 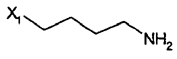 | 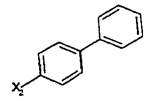 | 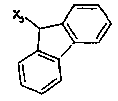 | 8,79 | 488,96 |
| 322 | 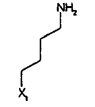 | 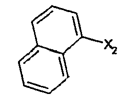 | 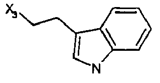 | 8,07 | 442,30 |
| 323 | 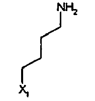 | 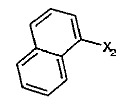 | 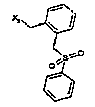 | 8,19 | 543,29 |
| 324 | 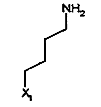 | 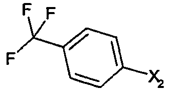 | 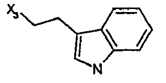 | 8,20 | 460,29 |
| 325 | 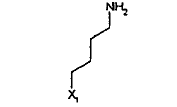 | 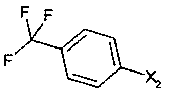 | 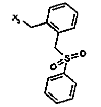 | 8,34 | 561,26 |
| 326 | 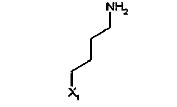 | 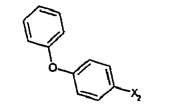 | 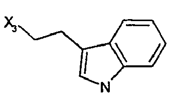 | 8,40 | 484,34 |
| 327 | 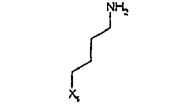 | 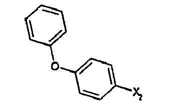 | 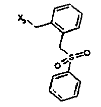 | 8,54 | 585,30 |
| 328 | 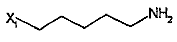 | 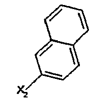 | 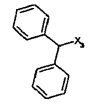 | 8,64 | 479,36 |
| 329 | 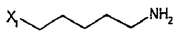 | 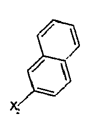 | 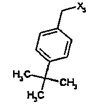 | 8,81 | 459,36 |
| 330 | 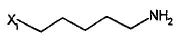 | 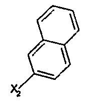 | 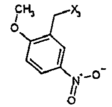 | 8,18 | 478,30 |
| 331 | 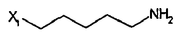 | 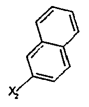 | 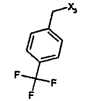 | 8,51 | 471,28 |
| 332 | 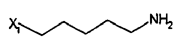 | 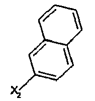 | 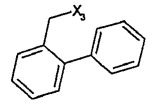 | 8,65 | 479,37 |
| 333 | 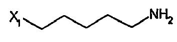 | 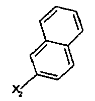 | 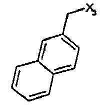 | 8,41 | 453,32 |
| 334 | 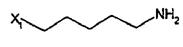 | 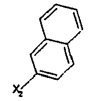 | 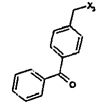 | 8,48 | 507,34 |
| 335 | 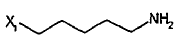 | 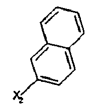 | 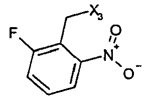 | 8,12 | 466,29 |
| 336 | 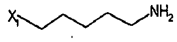 | 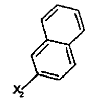 | 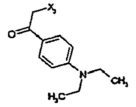 | 8,50 | 502,40 |
| 337 | 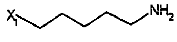 | 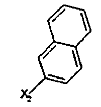 | 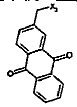 | 8,51 | 533,30 |
| 338 | 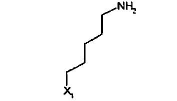 | 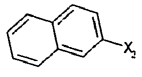 | 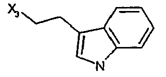 | 8,30 | 456,30 |
| 339 | 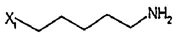 | 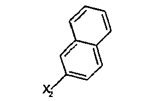 | 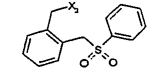 | 8,41 | 557,30 |
| 340 | 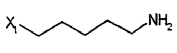 | 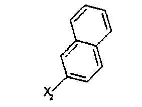 | 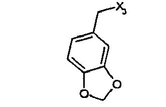 | 8,10 | 447,27 |
| 341 | 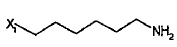 | 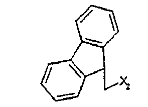 | 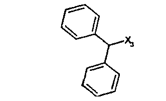 | 8,78 | 493,36 |
| 342 | 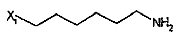 | 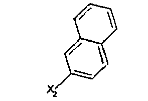 | 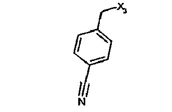 | 8,14 | 442,29 |
| 343 | 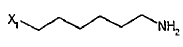 | 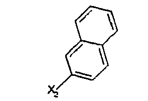 | 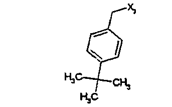 | 8,94 | 473,36 |
| 344 | 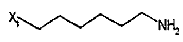 | 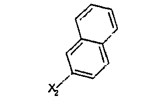 | 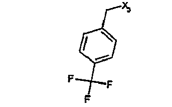 | 8,61 | 485,31 |
| 345 | 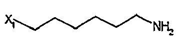 | 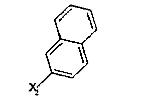 | 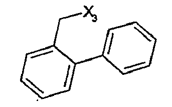 | 8,79 | 493,36 |
| 346 | 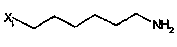 | 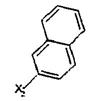 | 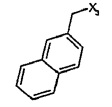 | 8,55 | 467,32 |
| 347 | 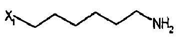 | 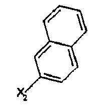 | 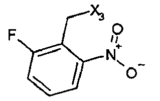 | 8,24 | 480,30 |
| 348 | 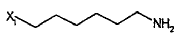 | 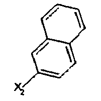 | 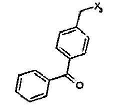 | 8,61 | 521,35 |
| 349 | 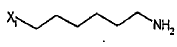 | 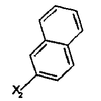 | 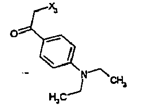 | 8,60 | 516,40 |
| 350 | 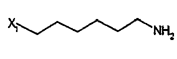 | 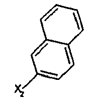 | 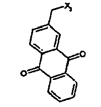 | 8,61 | 547,30 |
| 351 | 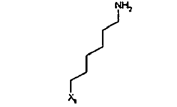 | 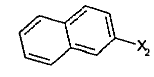 | 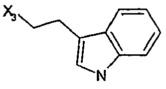 | 8,40 | 470,30 |
| 352 | 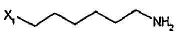 | 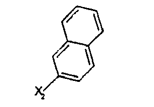 | 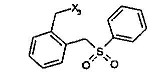 | 8,48 | 571,30 |
| 353 | 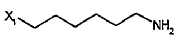 | 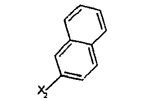 | 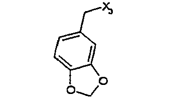 | 8,23 | 461,28 |
| 354 | 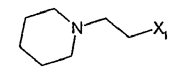 | 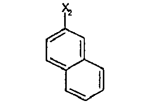 | 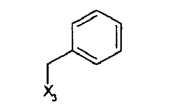 | 7,56 | 429,26 |
| 355 | 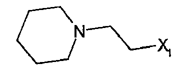 | 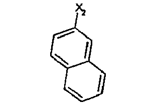 | 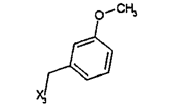 | 7,39 | 459,39 |
| 356 | 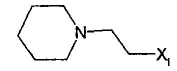 | 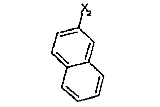 | 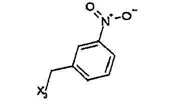 | 7,45 | 474,39 |
| 357 | 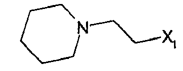 | 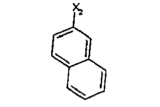 | 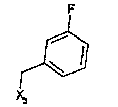 | 7,59 | 447,27 |
| 358 | 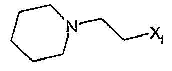 | 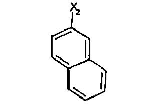 | 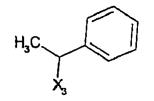 | 7,54 | 443,43 |
| 359 | 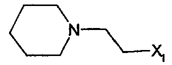 | 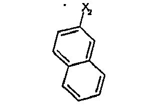 | 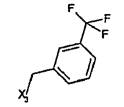 | 8,03 | 497,25 |
| 360 | 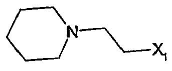 | 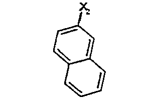 | 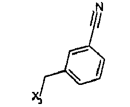 | 7,29 | 454,40 |
| 361 | 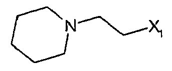 | 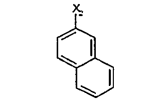 | 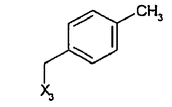 | 7,59 | 443,38 |
| 362 | 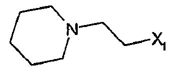 | 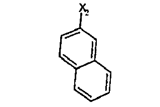 | 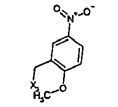 | 7,50 | 504,41 |
| 363 | 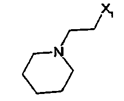 | 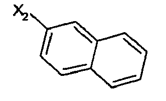 | 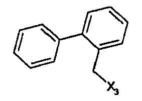 | 8,07 | 505,42 |
| 364 | 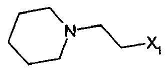 | 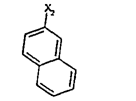 | 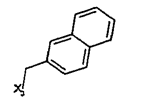 | 7,78 | 479,43 |
| 365 | 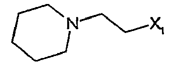 | 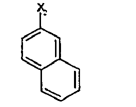 | 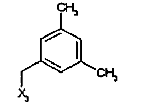 | 7,84 | 457,43 |
| 366 | 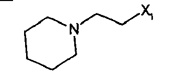 | 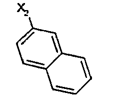 | 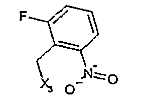 | 7,45 | 492,38 |
| 367 | 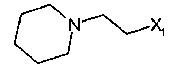 | 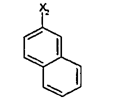 | 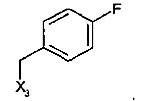 | 7,55 | 447,39 |
| 368 | 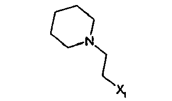 | 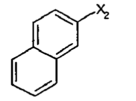 | 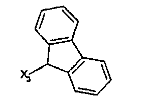 | 8,18 | 503,46 |
| 369 | 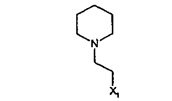 | 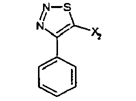 | 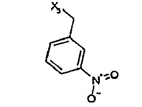 | 4,76 | 508,21 |
| 370 | 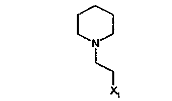 | 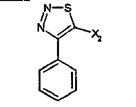 | 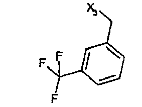 | 5,18 | 531,21 |
| 371 | 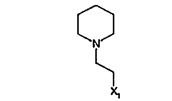 | 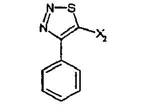 | 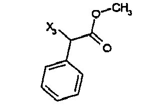 | 4,85 | 521,22 |
| 372 | 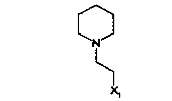 | 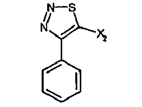 | 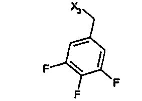 | 5,07 | 517,21 |
| 373 | 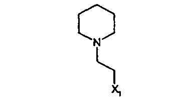 | 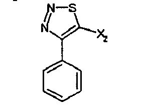 | 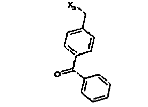 | 5,17 | 567,23 |
| 374 | 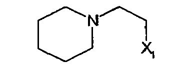 | 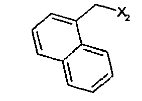 | 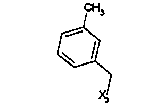 | 7,56 | 457,26 |
| 375 | 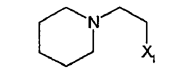 | 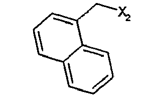 | 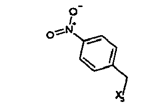 | 10,90 | 488,31 |
| 376 | 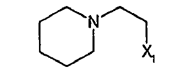 | 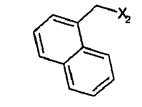 | 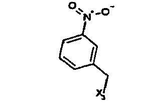 | 7,48 | 488,21 |
| 377 | 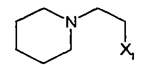 | 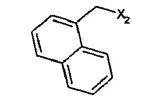 | 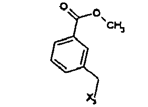 | 7,38 | 501,23 |
| 378 | 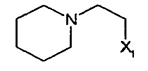 | 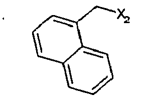 | 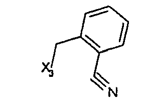 | 12,40 | 465,28 |
| 379 | 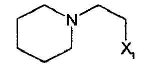 | 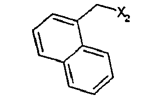 | 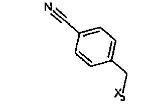 | 7,25 | 468,24 |
| 380 | 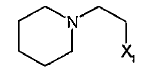 | 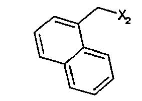 | 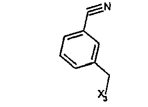 | 7,30 | 468,24 |
| 381 | 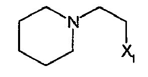 | 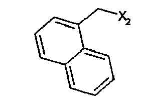 | 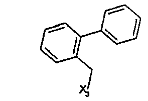 | 8,12 | 519,26 |
| 382 | 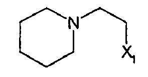 | 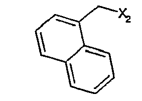 | 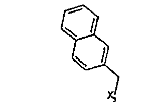 | 7,84 | 493,34 |
| 383 | 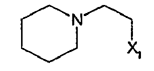 | 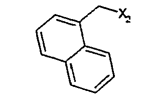 | 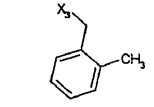 | 7,48 | 457,26 |
| 384 | 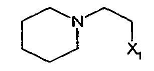 | 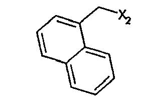 | 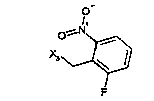 | 7,40 | 506,23 |
| 385 | 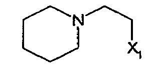 | 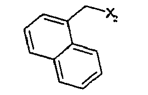 | 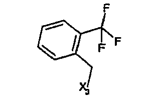 | 11,16 | 511,31 |
| 386 | 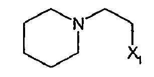 | 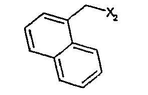 | 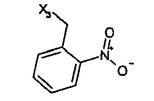 | 7,42 | 488,23 |
| 387 | 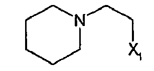 | 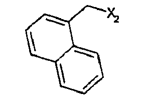 | 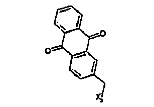 | 7,89 | 573,20 |
| 388 | 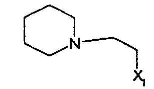 | 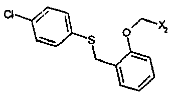 | 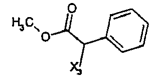 | 8,98 | 623,35 |
| 389 | 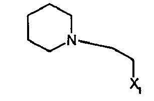 | 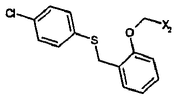 | 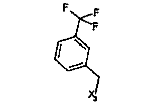 | 9,29 | 633,34 |
| 390 | 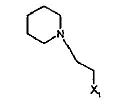 | 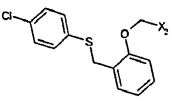 | 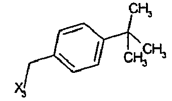 | 9,77 | 621,41 |
| 391 | 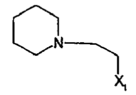 | 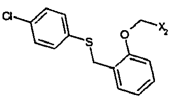 | 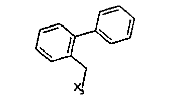 | 9,52 | 641,39 |
| 392 | 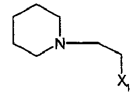 | 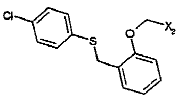 | 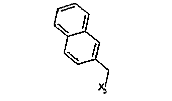 | 9,28 | 615,35 |
| 393 | 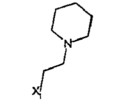 | 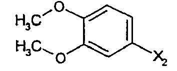 | 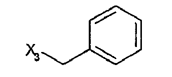 | 6,95 | 439,28 |
| 394 | 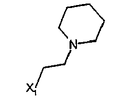 | 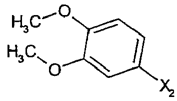 | 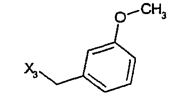 | 6,86 | 469,39 |
| 395 | 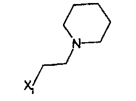 | 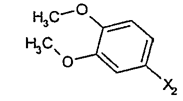 | 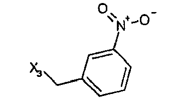 | 6,91 | 484,42 |
| 396 | 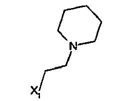 | 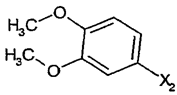 | 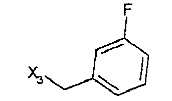 | 7,01 | 457,29 |
| 397 | 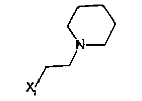 | 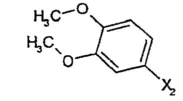 | 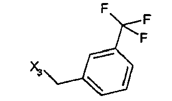 | 7,45 | 507,26 |
| 398 | 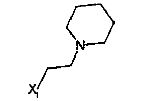 | 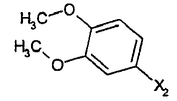 | 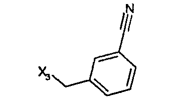 | 6,76 | 464,41 |
| 399 | 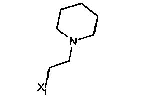 | 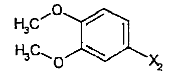 | 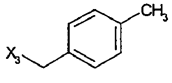 | 7,08 | 453,39 |
| 400 | 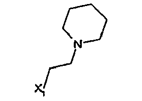 | 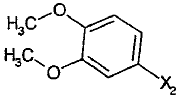 | 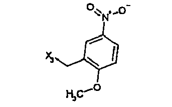 | 7,01 | 514,40 |
| 401 | 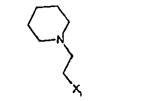 | 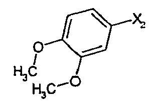 | 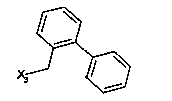 | 7,59 | 515,41 |
| 402 | 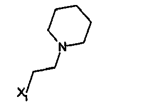 | 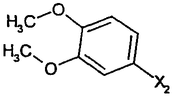 | 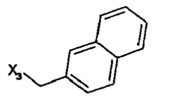 | 7,28 | 489,44 |
| 403 | 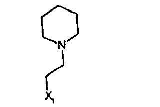 | 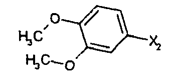 | 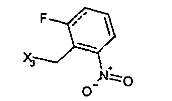 | 6,90 | 502,40 |
| 404 | 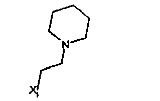 | 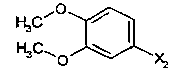 | 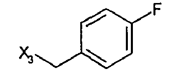 | 6,98 | 457,41 |
| 405 | 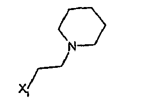 | 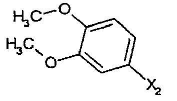 | 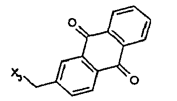 | 7,43 | 569,36 |
| 406 | 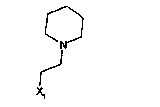 | 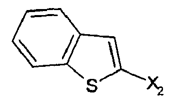 | 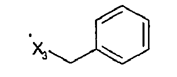 | 7,67 | 435,22 |
| 407 | 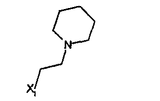 | 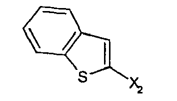 | 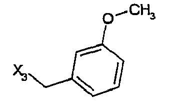 | 7,49 | 465,36 |
| 408 | 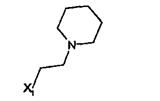 | 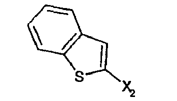 | 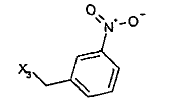 | 7,58 | 480,37 |
| 409 | 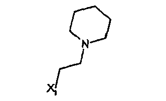 | 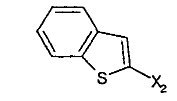 | 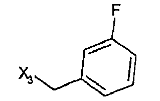 | 7,70 | 453,27 |
| 410 | 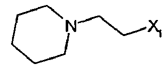 | 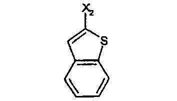 | 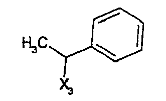 | 7,69 | 449,38 |
| 411 | 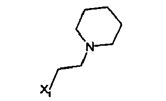 | 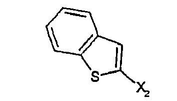 | 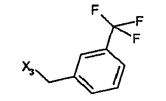 | 8,16 | 503,23 |
| 412 | 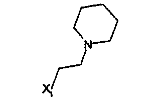 | 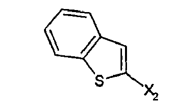 | 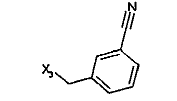 | 7,40 | 460,35 |
| 413 | 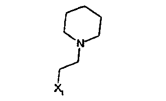 | 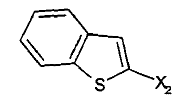 | 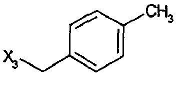 | 7,71 | 449,33 |
| 414 | 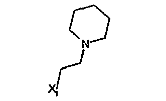 | 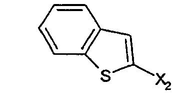 | 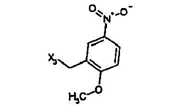 | 7,60 | 510,35 |
| 415 | 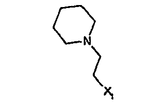 | 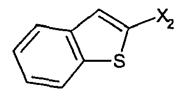 | 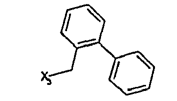 | 8,16 | 511,37 |
| 416 | 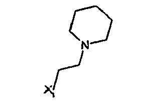 | 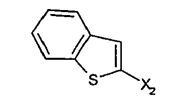 | 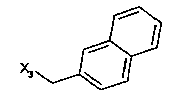 | 7,94 | 485,38 |
| 417 | 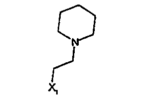 | 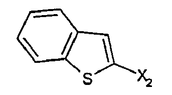 | 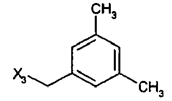 | 7,99 | 463,40 |
| 418 | 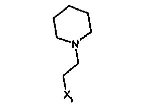 | 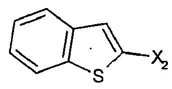 | 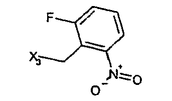 | 7,59 | 498,34 |
| 419 | 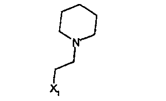 | 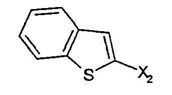 | 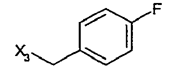 | 7,68 | 453,36 |
| 420 | 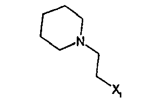 | 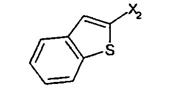 | 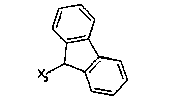 | 8,38 | 509,41 |
| 421 | 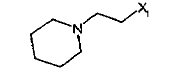 | 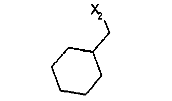 | 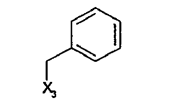 | 7,43 | 399,31 |
| 422 | 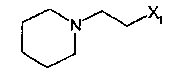 | 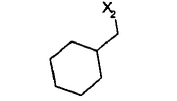 | 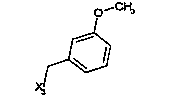 | 7,30 | 429,44 |
| 423 | 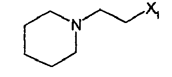 | 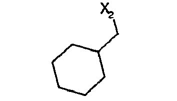 | 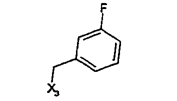 | 7,46 | 417,33 |
| 424 | 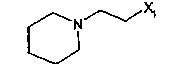 | 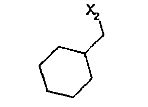 | 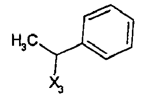 | 7,49 | 413,45 |
| 425 | 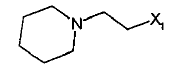 | 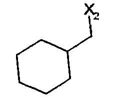 | 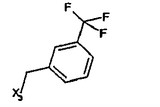 | 7,92 | 467,31 |
| 426 | 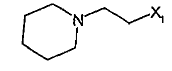 | 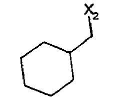 | 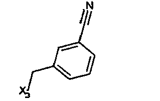 | 7,19 | 424,45 |
| 427 | 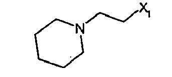 | 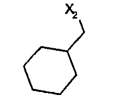 | 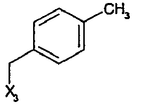 | 7,48 | 413,40 |
| 428 | 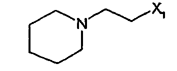 | 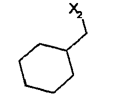 | 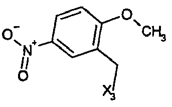 | 7,39 | 474,44 |
| 429 | 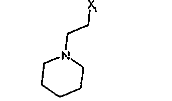 | 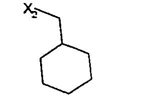 | 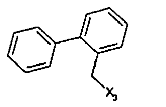 | 8,01 | 475,47 |
| 430 | 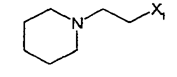 | 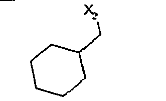 | 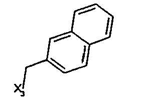 | 7,70 | 449,45 |
| 431 | 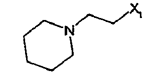 | 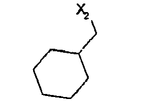 | 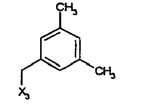 | 7,79 | 427,46 |
| 432 | 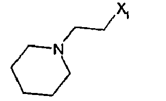 | 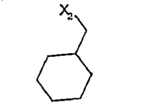 | 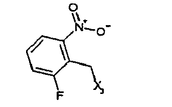 | 7,37 | 462,42 |
| 433 | 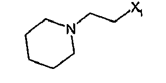 | 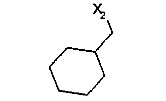 | 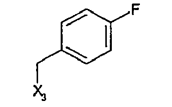 | 7,45 | 417,43 |
| 434 | 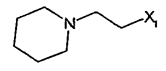 | 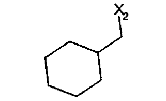 | 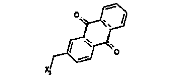 | 7,79 | 529,46 |
| 435 | 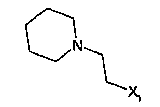 | 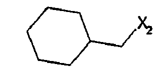 | 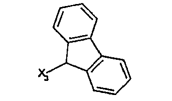 | 8,03 | 473,48 |
| 436 | 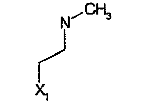 | 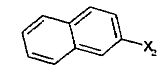 | 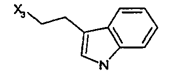 | 8,10 | 428,20 |
| 437 | 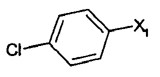 | 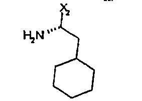 | 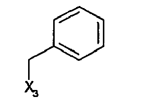 | 8,97 | 427,28 |
| 438 | 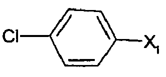 | 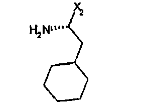 | 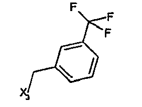 | 9,02 | 495,23 |
| 439 | 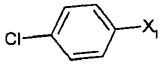 | 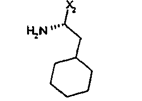 | 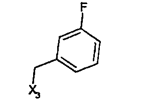 | 8,62 | 445,21 |
| 440 | 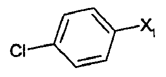 | 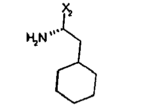 | 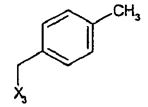 | 8,68 | 441,31 |
| 441 | 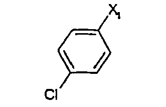 | 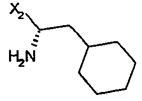 | 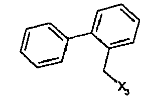 | 9,07 | 503,39 |
| 442 | 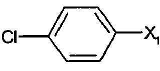 | 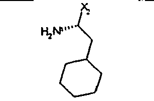 | 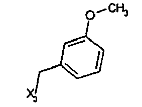 | 8,34 | 457,36 |
| 443 | 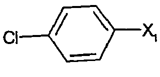 | 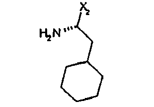 | 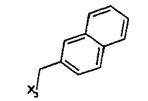 | 8,84 | 477,40 |
| 444 | 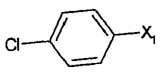 | 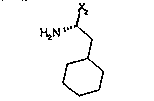 | 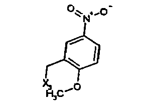 | 8,35 | 502,38 |
| 445 | 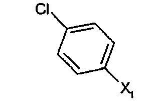 | 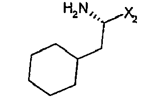 | 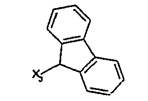 | 9,25 | 501,39 |
| 446 | 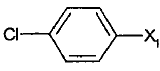 | 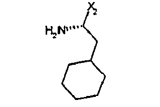 | 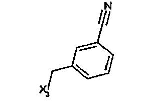 | 8,14 | 452,37 |
| 447 | 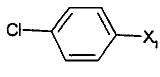 | 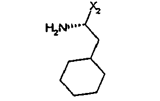 | 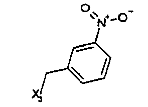 | 8,31 | 472,35 |
| 448 | 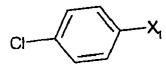 | 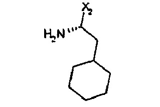 | 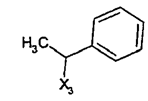 | 8,64 | 441,37 |
| 449 | 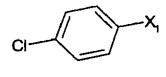 | 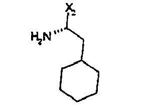 | 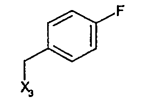 | 8,50 | 445,33 |
| 450 | 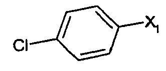 | 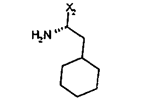 | 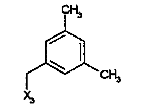 | 9,02 | 455,39 |
| 451 | 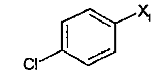 | 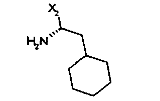 | 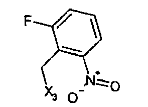 | 8,35 | 490,35 |
| 452 | 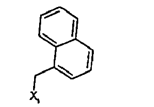 | 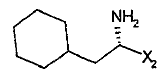 | 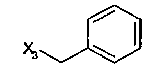 | 9,27 | 457,35 |
| 453 | 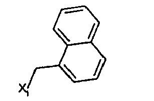 | 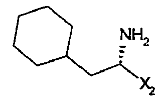 | 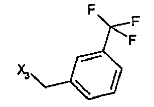 | 9,23 | 525,26 |
| 454 | 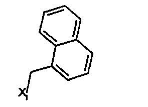 | 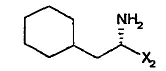 | 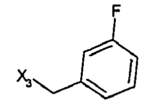 | 8,84 | 475,30 |
| 455 | 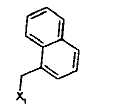 | 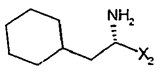 | 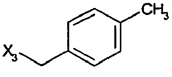 | 8,91 | 471,39 |
| 456 | 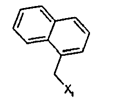 | 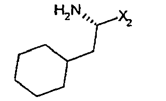 | 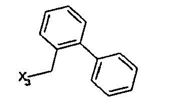 | 9,33 | 533,42 |
| 457 | 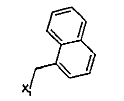 | 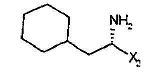 | 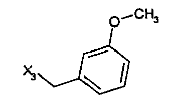 | 8,55 | 487,42 |
| 458 | 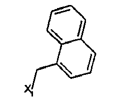 | 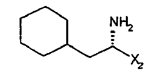 | 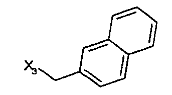 | 9,01 | 507,45 |
| 459 | 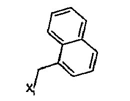 | 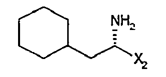 | 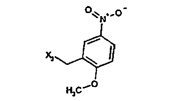 | 8,53 | 532,43 |
| 460 | 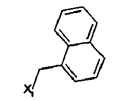 | 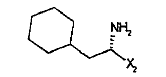 | 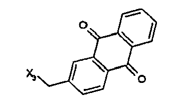 | 8,88 | 587,37 |
| 461 | 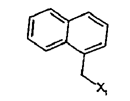 | 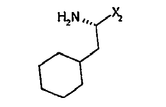 | 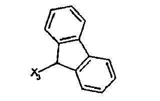 | 9,53 | 531,47 |
| 462 | 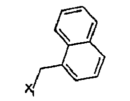 | 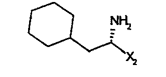 | 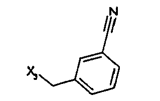 | 8,34 | 482,43 |
| 463 | 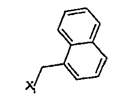 | 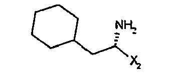 | 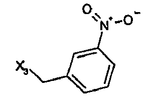 | 8,48 | 502,46 |
| 464 | 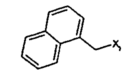 | 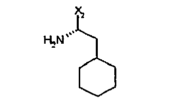 | 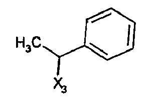 | 8,91 | 471,44 |
| 465 | 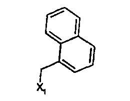 | 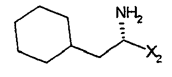 | 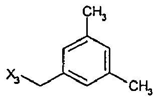 | 9,24 | 485,47 |
| 466 | 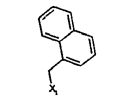 | 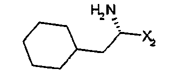 | 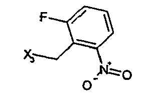 | 8,58 | 520,40 |
| 467 | 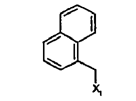 | 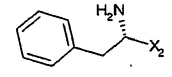 | 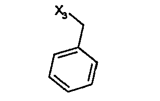 | 5,49 | 451,23 |
| 468 | 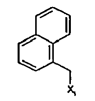 | 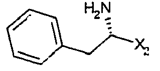 | 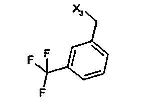 | 5,81 | 519,22 |
| 469 | 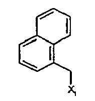 | 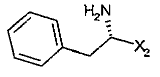 | 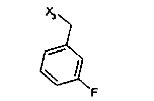 | 5,52 | 469,23 |
| 470 | 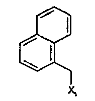 | 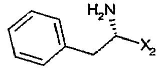 | 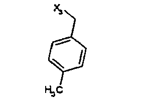 | 5,67 | 465,25 |
| 471 | 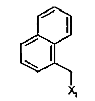 | 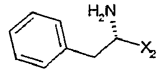 | 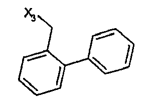 | 6,05 | 527,28 |
| 472 | 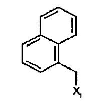 | 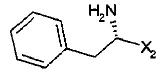 | 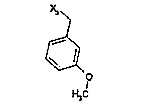 | 5,46 | 481,25 |
| 473 | 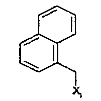 | 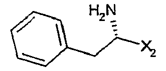 | 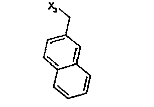 | 5,83 | 501,27 |
| 474 | 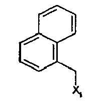 | 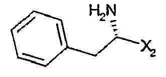 | 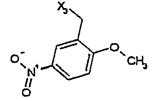 | 5,43 | 526,25 |
| 475 | 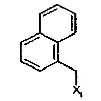 | 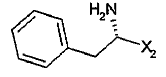 | 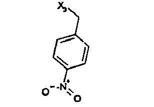 | 5,39 | 496,22 |
| 476 | 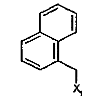 | 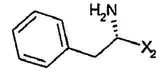 | 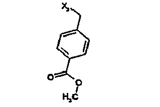 | 5,37 | 509,26 |
| 477 | 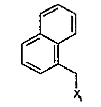 | 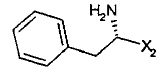 | 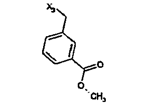 | 5,39 | 509,26 |
| 478 | 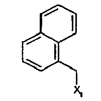 | 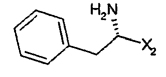 | 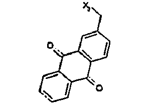 | 5,71 | 581,22 |
| 479 | 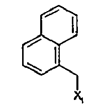 | 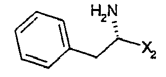 | 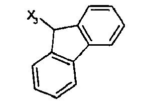 | 6,06 | 525,26 |
| 480 | 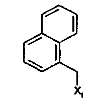 | 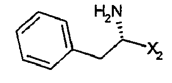 | 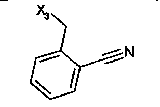 | 5,26 | 476,24 |
| 481 | 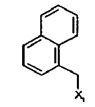 | 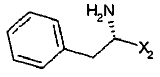 | 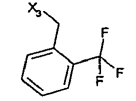 | 5,79 | 519,23 |
| 482 | 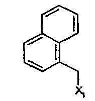 | 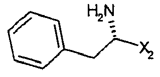 | 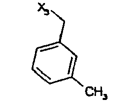 | 5,68 | 465,25 |
| 483 | 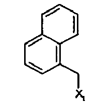 | 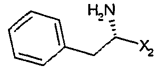 | 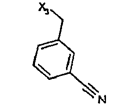 | 5,25 | 476,25 |
| 484 | 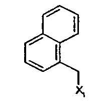 | 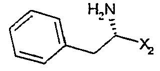 | 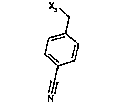 | 5,25 | 476,25 |
| 485 | 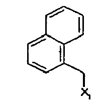 | 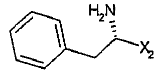 | 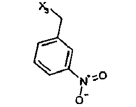 | 5,39 | 496,23 |
| 486 | 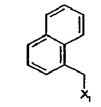 | 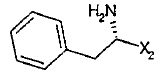 | 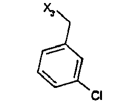 | 5,71 | 485,21 |
| 487 | 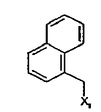 | 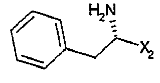 | 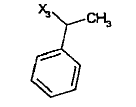 | 5,66 | 465,25 |
| 488 | 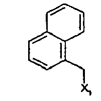 | 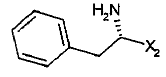 | 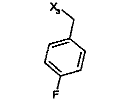 | 5,51 | 469,24 |
| 489 | 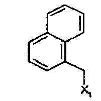 | 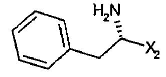 | 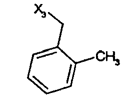 | 5,65 | 465,24 |
| 490 | 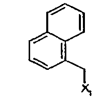 | 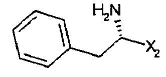 | 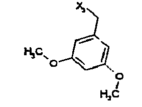 | 11,53 | 511,31 |
| 491 | 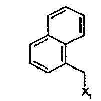 | 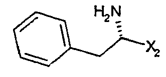 | 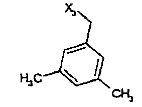 | 5,91 | 479,28 |
| 492 | 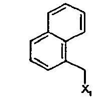 | 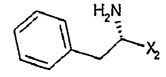 | 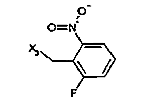 | 5,38 | 514,23 |
| 493 | 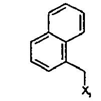 | 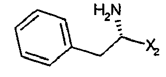 | 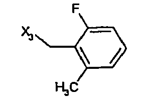 | 5,70 | 483,28 |
| 494 | 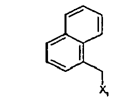 | 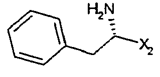 | 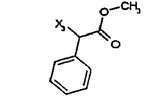 | 5,53 | 509,26 |
| 495 | 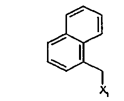 | 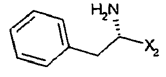 | 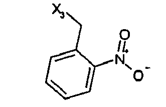 | 5,39 | 496,24 |
| 496 | 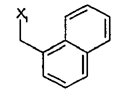 | 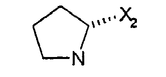 | 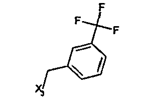 | 9,20 | 469,12 |
| 497 | 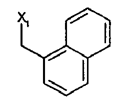 | 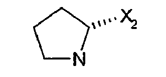 | 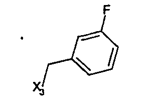 | 8,87 | 419,10 |
| 498 | 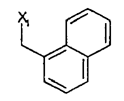 | 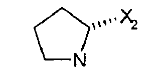 | 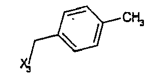 | 9,09 | 415,14 |
| 499 | 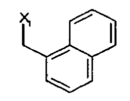 | 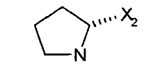 | 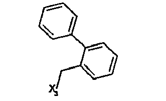 | 9,55 | 477,18 |
| 500 | 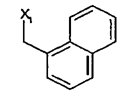 | 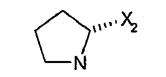 | 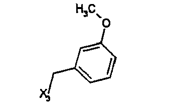 | 8,82 | 431,14 |
| 501 | 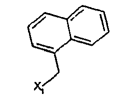 | 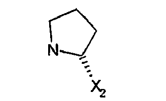 | 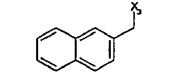 | 9,25 | 451,15 |
| 502 | 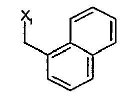 | 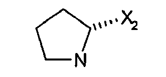 | 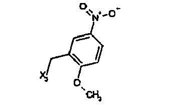 | 8,85 | 476,15 |
| 503 | 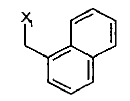 | 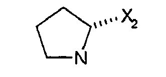 | 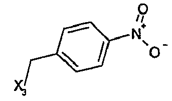 | 8,81 | 446,11 |
| 504 | 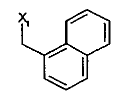 | 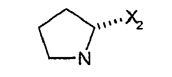 | 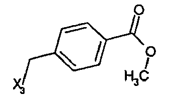 | 8,75 | 459,14 |
| 505 | 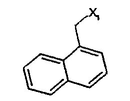 | 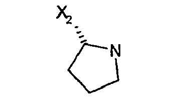 | 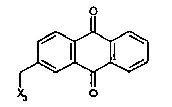 | 9,18 | 531,14 |
| 506 | 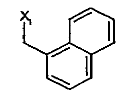 | 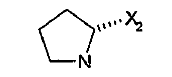 | 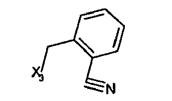 | 8,61 | 426,13 |
| 507 | 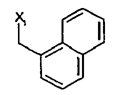 | 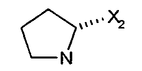 | 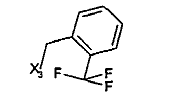 | 9,21 | 469,11 |
| 508 | 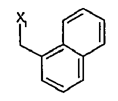 | 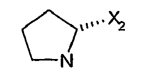 | 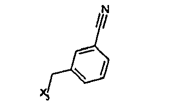 | 8,64 | 426,13 |
| 509 | 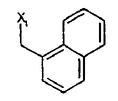 | 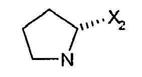 | 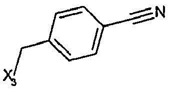 | 8,64 | 426,13 |
| 510 | 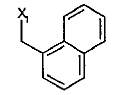 | 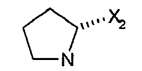 | 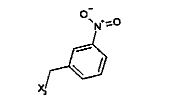 | 8,77 | 446,11 |
| 511 | 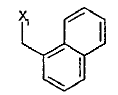 | 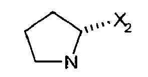 | 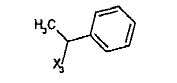 | 9,01 | 415,14 |
| 512 | 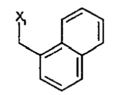 | 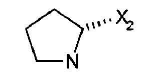 | 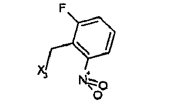 | 8,74 | 464,11 |
| 513 | 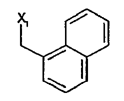 | 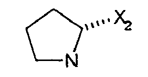 | 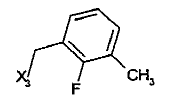 | 9,11 | 433,14 |
| 514 | 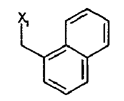 | 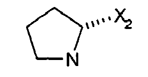 | 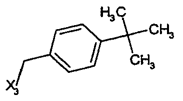 | 9,77 | 457,21 |
| 515 | 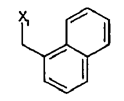 | 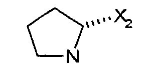 | 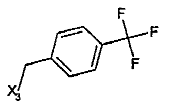 | 9,28 | 469,12 |
| 516 | 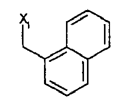 | 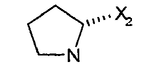 | 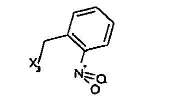 | 8,77 | 446,11 |
| 517 | 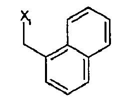 | 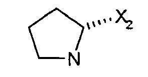 | 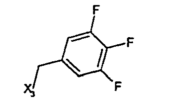 | 9,09 | 455,09 |
| 518 | 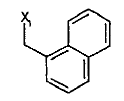 | 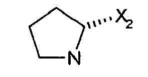 | 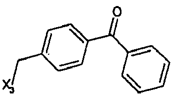 | 9,25 | 505,17 |
| 519 | 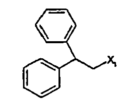 | 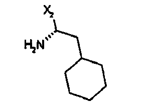 | 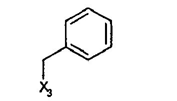 | 9,56 | 497,37 |
| 520 | 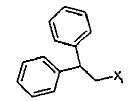 | 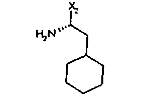 | 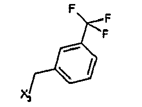 | 9,51 | 565,28 |
| 521 | 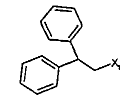 | 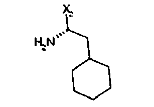 | 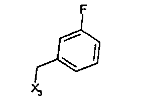 | 9,11 | 515,32 |
| 522 | 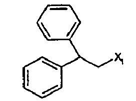 | 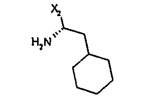 | 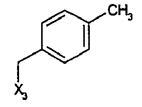 | 9,15 | 511,44 |
| 523 | 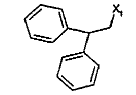 | 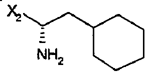 | 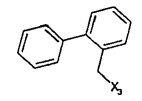 | 9,58 | 573,42 |
| 524 | 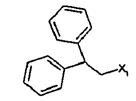 | 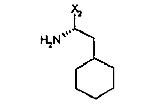 | 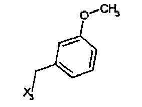 | 8,83 | 527,44 |
| 525 | 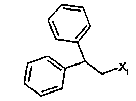 | 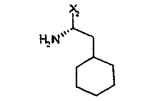 | 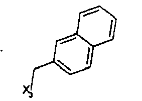 | 9,24 | 547,45 |
| 526 | 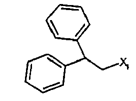 | 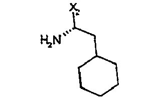 | 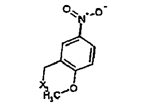 | 8,83 | 572,42 |
| 527 | 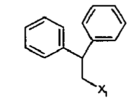 | 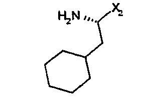 | 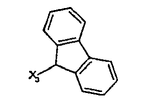 | 9,78 | 571,48 |
| 528 | 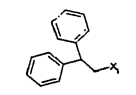 | 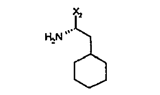 | 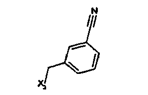 | 8,61 | 522,47 |
| 529 | 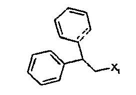 | 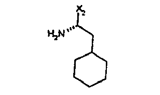 | 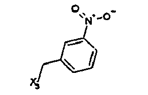 | 8,80 | 542,45 |
| 530 | 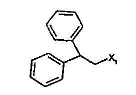 | 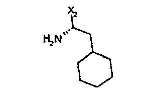 | 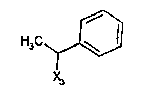 | 9,12 | 511,51 |
| 531 | 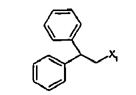 | 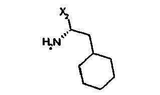 | 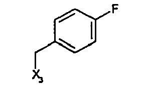 | 9,00 | 515,46 |
| 532 | 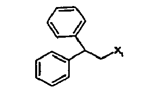 | 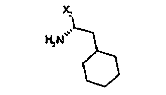 | 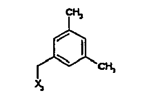 | 9,49 | 525,49 |
| 533 | 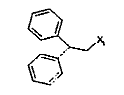 | 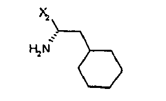 | 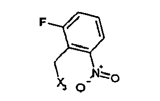 | 8,90 | 560,41 |
| 534 | 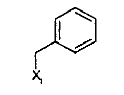 | 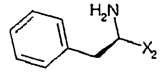 | 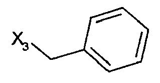 | 4,74 | 401,2 |
| 535 | 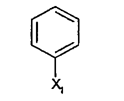 | 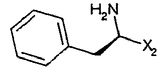 | 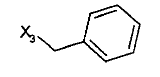 | 4,67 | 387,2 |
| 536 | 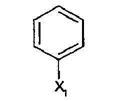 | 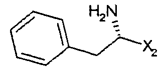 | 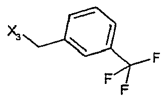 | 5,43 | 455,18 |
| 537 | 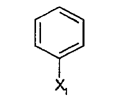 | 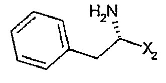 | 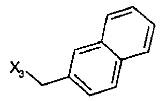 | 5,46 | 437,21 |
| 538 | 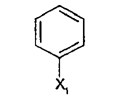 | 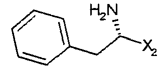 | 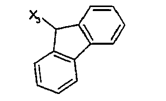 | 8,40 | 461,29 |
| 539 | 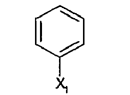 | 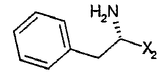 | 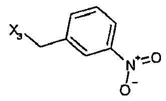 | 7,63 | 432,24 |
| 540 | 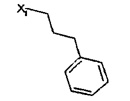 | 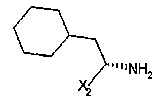 | 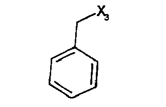 | 8,62 | 435,39 |
| 541 | 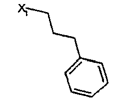 | 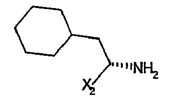 | 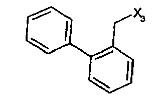 | 9,46 | 511,45 |
| 542 | 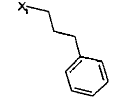 | 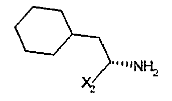 | 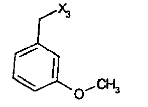 | 8,63 | 465,43 |
| 543 | 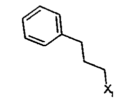 | 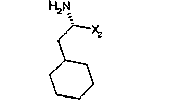 | 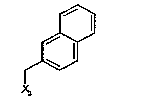 | 9,08 | 485,44 |
| 544 | 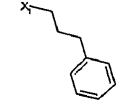 | 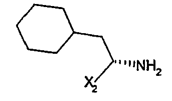 | 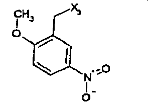 | 8,59 | 510,40 |
| 545 | 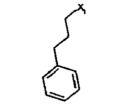 | 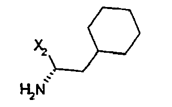 | 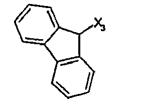 | 9,40 | 509,43 |
| 546 | 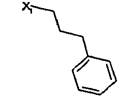 | 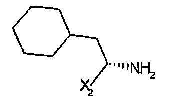 | 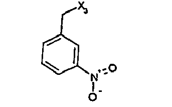 | 8,54 | 480,41 |
| 547 | 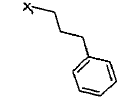 | 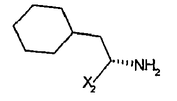 | 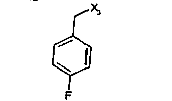 | 8,62 | 453,41 |
| 548 | 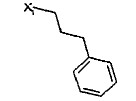 | 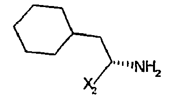 | 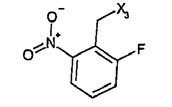 | 8,52 | 498,39 |
| 549 | 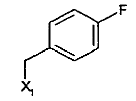 | 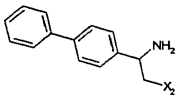 | 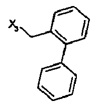 | 9,51 | 570,89 |
| 550 | 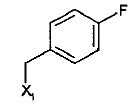 | 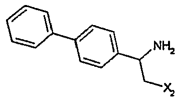 | 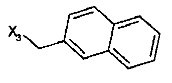 | 9,21 | 544,90 |
| 551 | 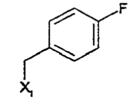 | 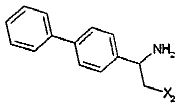 | 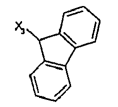 | 9,51 | 568,87 |
| 552 | 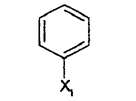 | 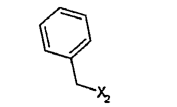 | 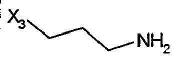 | 3,70 | 325,20 |
| 553 | 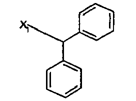 | 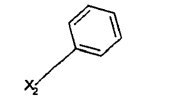 | 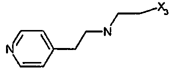 | 8,09 | 520,34 |
| 554 | 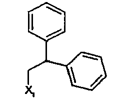 | 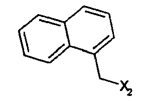 | 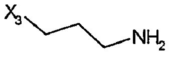 | 8,50 | 479,30 |
| 555 | 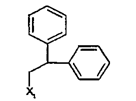 | 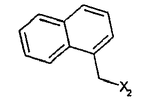 | 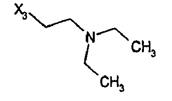 | 8,90 | 521,30 |
| 556 | 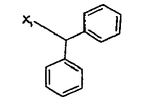 | 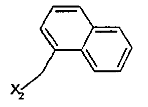 | 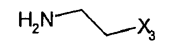 | 8,95 | 465,33 |
| 557 | 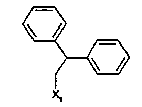 | 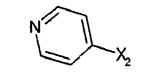 | 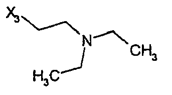 | 7,90 | 458,20 |
| 558 | 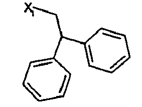 | 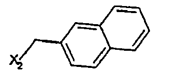 | 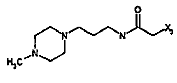 | 8,40 | 619,30 |
| 559 | 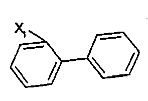 | 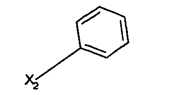 | 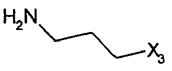 | 8,39 | 401,31 |
| 560 | 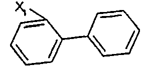 | 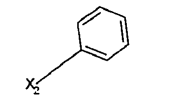 | 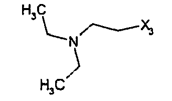 | 8,71 | 443,35 |
| 561 | 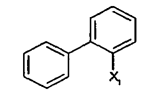 | 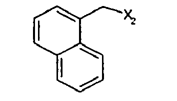 | 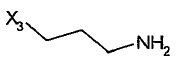 | 8,30 | 461,30 |
| 562 | 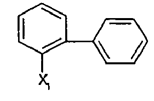 | 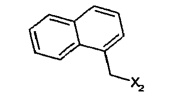 | 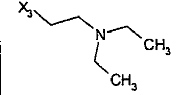 | 8,70 | 493,30 |
| 563 | 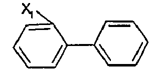 | 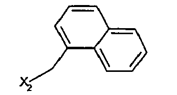 | 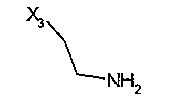 | 8,80 | 437,29 |
| 564 | 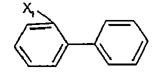 | 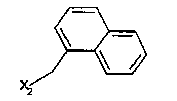 | 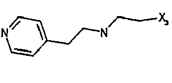 | 8,21 | 542,33 |
| 565 | 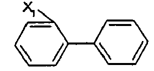 | 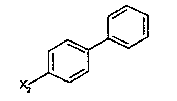 | 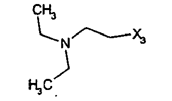 | 9,50 | 505,20 |
| 566 | 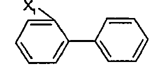 | 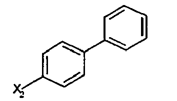 | 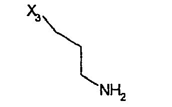 | 8,74 | 462,93 |
| 567 | 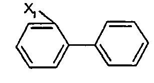 | 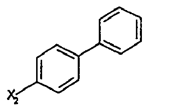 | 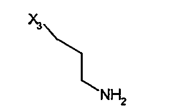 | 9,10 | 463,30 |
| 568 | 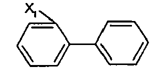 | 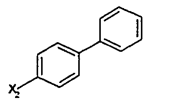 | 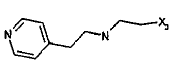 | 8,40 | 554,20 |
| 569 | 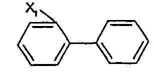 | 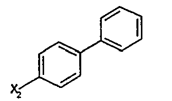 | 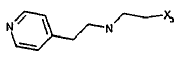 | 8,38 | 554,33 |
| 570 | 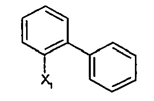 | 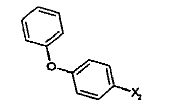 | 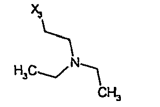 | 9,40 | 521,20 |
| 571 | 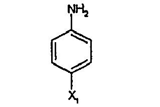 | 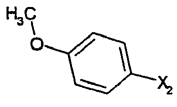 | 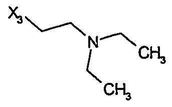 | 6,90 | 398,20 |
| 572 | 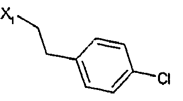 | 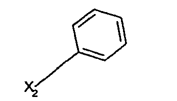 | 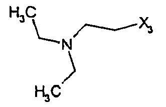 | 8,68 | 429,30 |
| 573 | 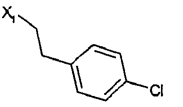 | 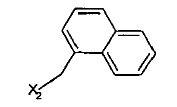 | 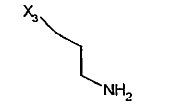 | 8,72 | 437,26 |
| 574 | 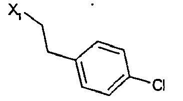 | 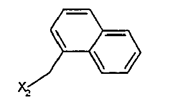 | 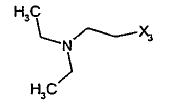 | 9,05 | 479,34 |
| 575 | 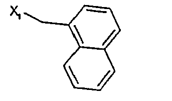 | 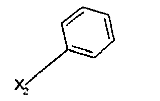 | 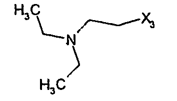 | 8,62 | 431,34 |
| 576 | 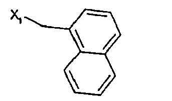 | 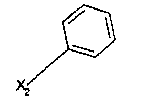 | 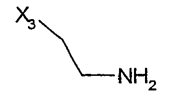 | 8,31 | 375,31 |
| 577 | 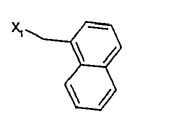 | 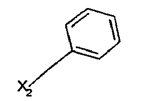 | 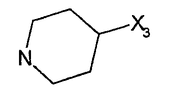 | 8,37 | 415,29 |
| 578 | 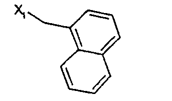 | 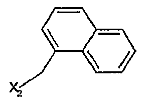 | 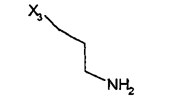 | 8,72 | 439,31 |
| 579 | 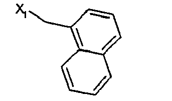 | 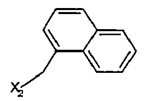 | 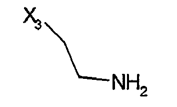 | 8,68 | 425,29 |
| 580 | 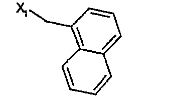 | 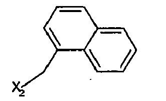 | 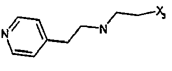 | 8,16 | 530,37 |
| 581 | 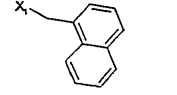 | 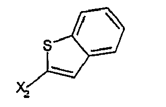 | 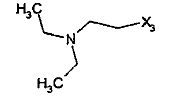 | 9,02 | 473,29 |
| 582 | 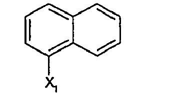 | 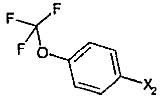 | 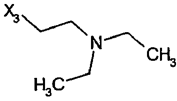 | 8,90 | 487,20 |
| 583 | 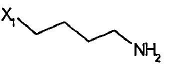 | 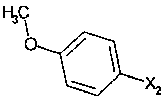 | 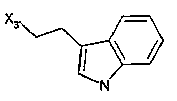 | 7,84 | 422,16 |
| 584 | 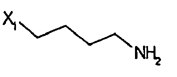 | 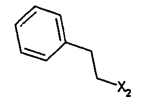 | 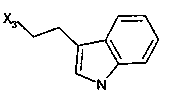 | 7,95 | 420,18 |
| 585 | 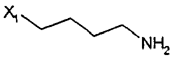 | 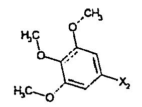 | 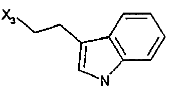 | 7,90 | 482,20 |
| 586 | 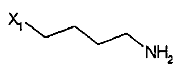 | 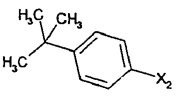 | 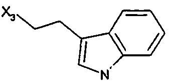 | 8,35 | 448,21 |
| 587 | 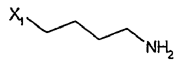 | 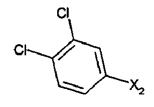 | 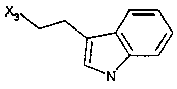 | 8,22 | 460,08 |
| 588 | 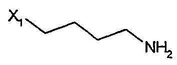 | 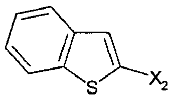 | 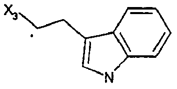 | 8,22 | 448,13 |
| 589 | 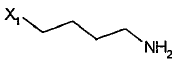 | 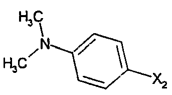 | 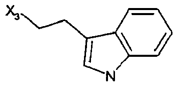 | 7,81 | 435,19 |
| 590 | 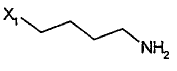 | 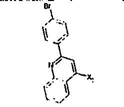 | 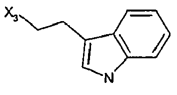 | 8,90 | 597,10 |
| 591 | 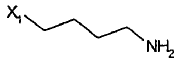 | 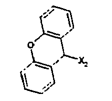 | 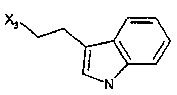 | 8,30 | 496,20 |
| 592 | 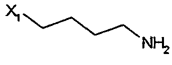 | 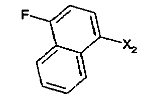 | 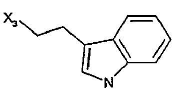 | 8,30 | 460,00 |
| 593 | 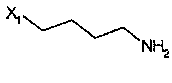 | 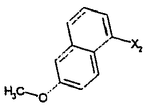 | 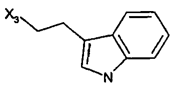 | 8,30 | 486,20 |
| 594 | 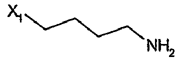 | 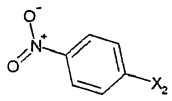 | 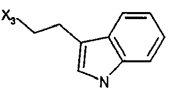 | 7,98 | 437,15 |
| 595 | 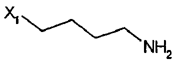 | 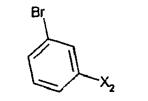 | 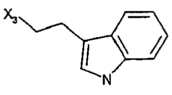 | 8,07 | 470,06 |
| 596 | 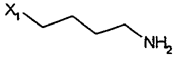 | 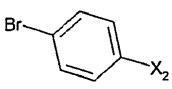 | 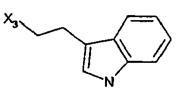 | 8,07 | 470,08 |
| 597 | 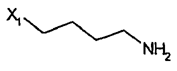 | 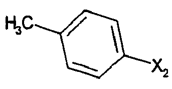 | 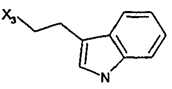 | 7,91 | 406,20 |
| 598 | 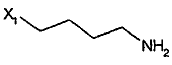 | 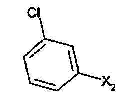 | 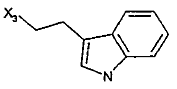 | 8,02 | 426,13 |
| 599 | 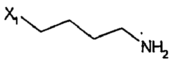 | 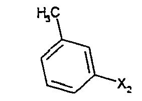 | 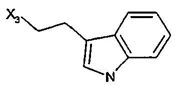 | 7,93 | 406,19 |
| 600 | 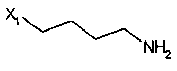 | 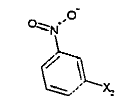 | 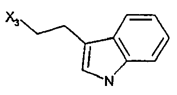 | 8,00 | 437,20 |
| 601 | 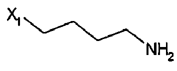 | 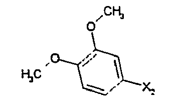 | 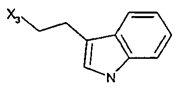 | 7,80 | 452,20 |
| 602 | 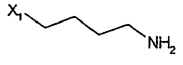 | 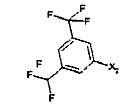 | 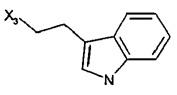 | 8,58 | 528,16 |
| 603 | 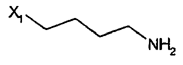 | 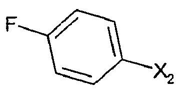 | 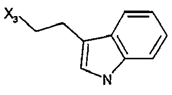 | 7,88 | 410,16 |
| 604 | 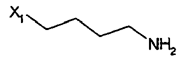 | 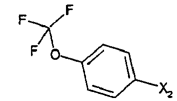 | 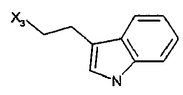 | 8,27 | 476,17 |
| 605 | 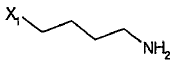 | 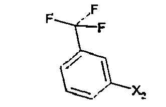 | 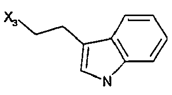 | 8,19 | 460,15 |
| 606 | 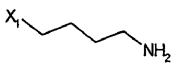 | 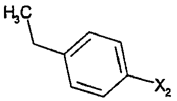 | 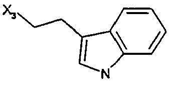 | 8,09 | 420,20 |
| 607 | 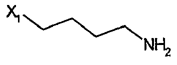 | 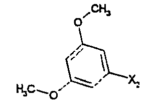 | 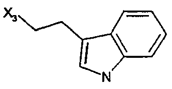 | 7,99 | 452,19 |
| 608 | 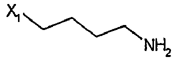 | 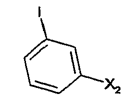 | 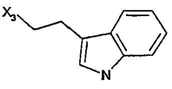 | 8,14 | 518,07 |
| 609 | 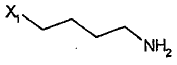 | 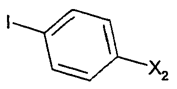 | 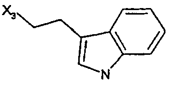 | 8,15 | 518,07 |
| 610 | 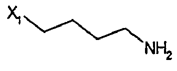 | 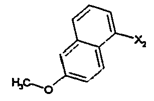 | 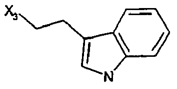 | 8,20 | 472,20 |
| 611 | 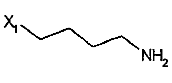 | 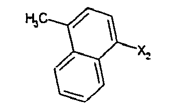 | 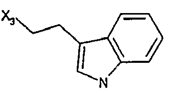 | 8,20 | 456,20 |
| 612 | 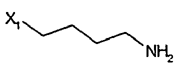 | 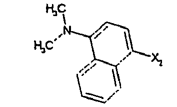 | 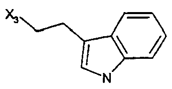 | 8,00 | 485,20 |
| 613 | 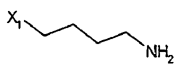 | 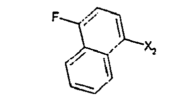 | 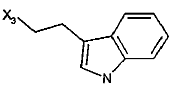 | 8,20 | 460,20 |
| 614 | 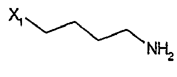 | 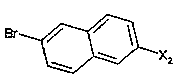 | 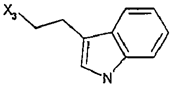 | 8,50 | 520,09 |
| 615 | 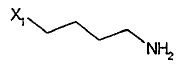 | 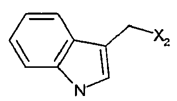 | 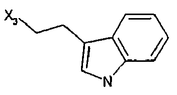 | 7,90 | 445,20 |
| 616 | 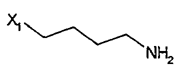 | 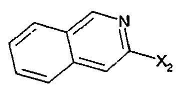 | 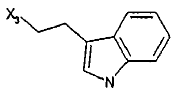 | 8,16 | 443,18 |
| 617 | 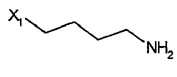 | 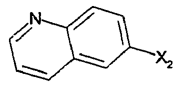 | 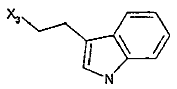 | 7,55 | 443,18 |
| 618 | 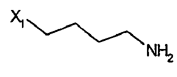 | 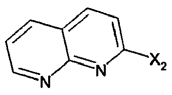 | 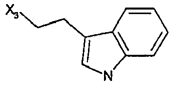 | 7,90 | 443,20 |
| 619 | 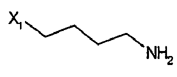 | 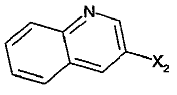 | 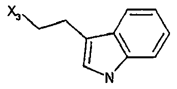 | 8,00 | 443,20 |
| 620 | 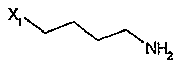 | 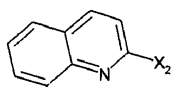 | 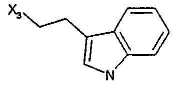 | 8,30 | 443,20 |
| 621 | 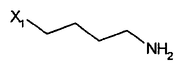 | 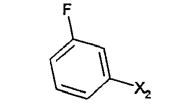 | 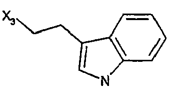 | 8,60 | 410,10 |
| 622 | 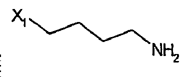 | 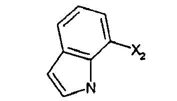 | 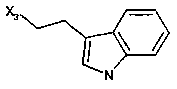 | 8,10 | 431,10 |
| 623 | 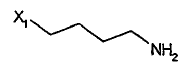 | 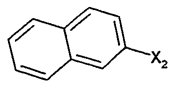 | 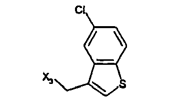 | 8,50 | 479,10 |
| 624 | 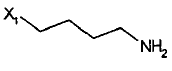 | 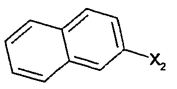 | 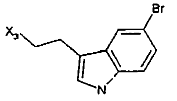 | 8,40 | 520,10 |
| 625 | 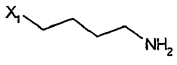 | 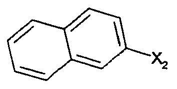 | 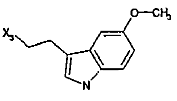 | 8,10 | 472,20 |
| 626 | 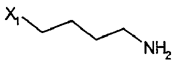 | 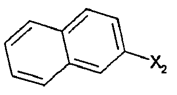 | 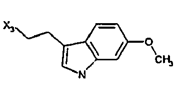 | 8,10 | 472,10 |
| 627 | 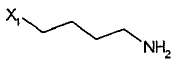 | 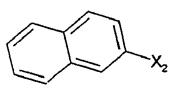 | 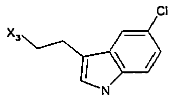 | 8,50 | 476,20 |
| 628 | 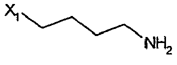 | 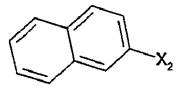 | 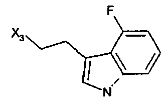 | 8,30 | 460,10 |
| 629 | 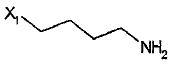 | 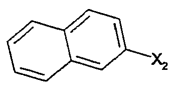 | 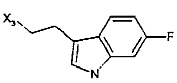 | 8,43 | 460,19 |
| 630 | 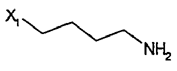 | 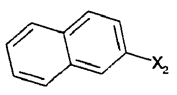 | 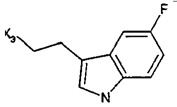 | 8,20 | 460,10 |
| 631 | 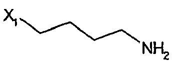 | 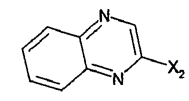 | 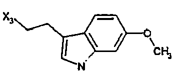 | 8,19 | 474,11 |
| 632 | 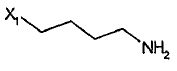 | 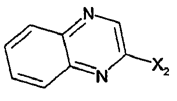 | 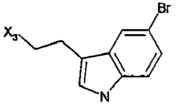 | 8,60 | 522,07 |
| 633 | 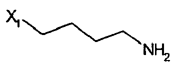 | 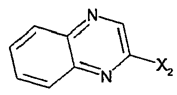 | 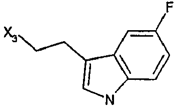 | 8,38 | 462,16 |
| 634 | 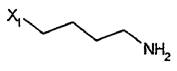 | 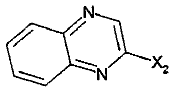 | 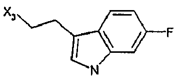 | 8,38 | 462,18 |
| 635 | 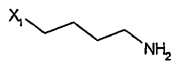 | 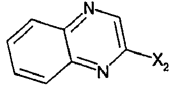 | 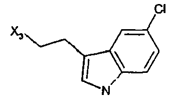 | 8,40 | 478,10 |
| 636 | 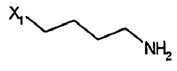 | 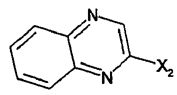 | 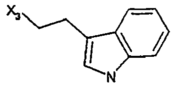 | 8,13 | 444,16 |
| 637 | 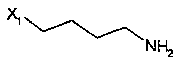 | 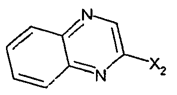 | 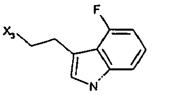 | 8,20 | 462,20 |
| 638 | 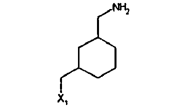 | 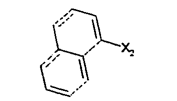 | 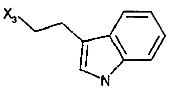 | 8,50 | 496,20 |
| 639 | 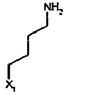 | 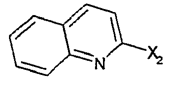 | 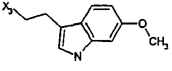 | 8,31 | 473,03 |
| 640 | 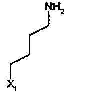 | 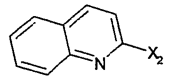 | 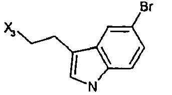 | 8,70 | 520,93 |
| 641 | 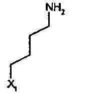 | 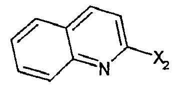 | 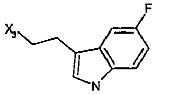 | 8,46 | 461,02 |
| 642 | 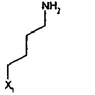 | 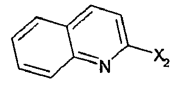 | 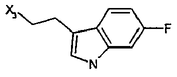 | 8,48 | 461,02 |
| 643 | 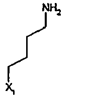 | 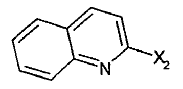 | 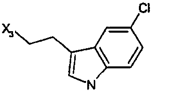 | 8,64 | 477,02 |
| 644 | 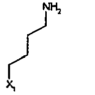 | 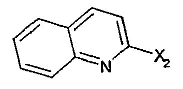 | 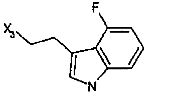 | 8,43 | 461,02 |
| 645 | 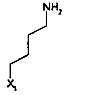 | 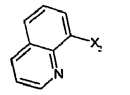 | 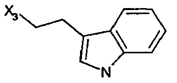 | 7,80 | 443,20 |
| 646 | 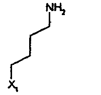 | 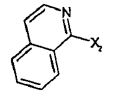 | 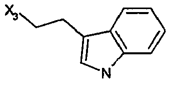 | 8,10 | 443,10 |
| 647 | 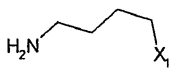 | 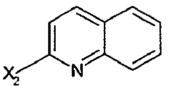 | 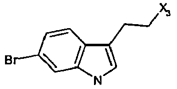 | 8,74 | 521,03 |
| 648 | 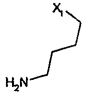 | 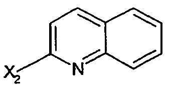 | 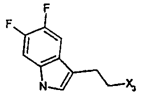 | 8,58 | 479,16 |
| 649 | 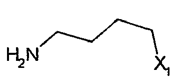 | 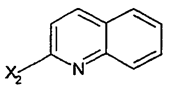 | 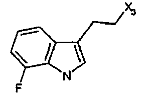 | 8,49 | 461,13 |
| 650 | 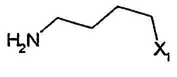 | 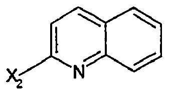 | 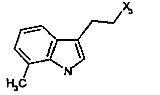 | 8,56 | 457,12 |
| 651 | 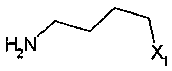 | 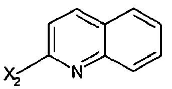 | 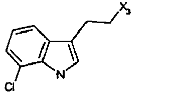 | 8,66 | 477,13 |
| 652 | 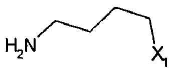 | 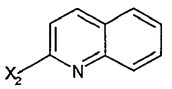 | 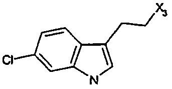 | 8,67 | 477,09 |
| 653 | 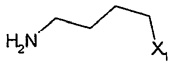 | 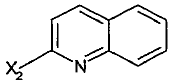 | 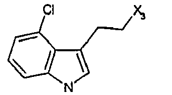 | 8,62 | 477,06 |
| 654 | 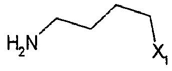 | 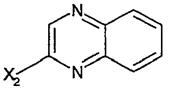 | 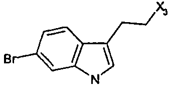 | 8,50 | 522,06 |
| 655 | 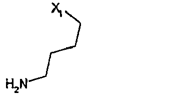 | 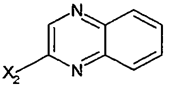 | 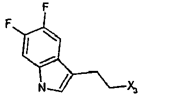 | 8,39 | 480,11 |
| 656 | 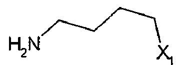 | 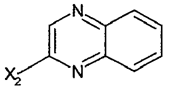 | 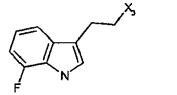 | 8,24 | 462,13 |
| 657 | 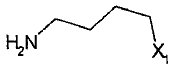 | 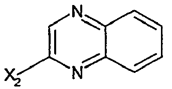 | 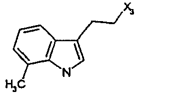 | 8,30 | 458,10 |
| 658 | 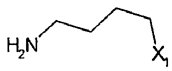 | 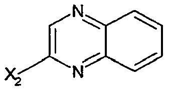 | 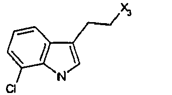 | 8,39 | 478,09 |
| 659 | 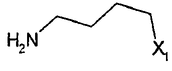 | 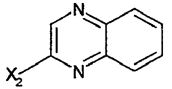 | 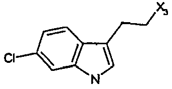 | 8,45 | 478,09 |
| 660 | 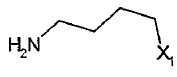 | 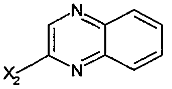 | 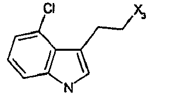 | 8,42 | 478,09 |
| 661 | 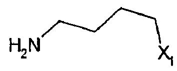 | 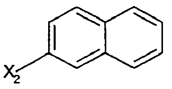 | 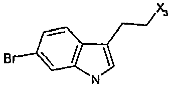 | 8,57 | 520,06 |
| 662 | 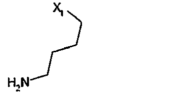 | 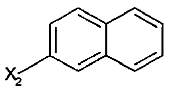 | 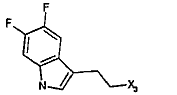 | 8,41 | 478,14 |
| 663 | 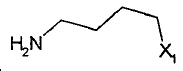 | 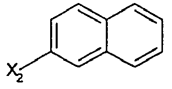 | 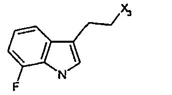 | 8,31 | 460,13 |
| 664 | 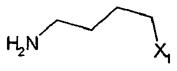 | 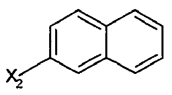 | 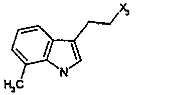 | 8,37 | 456,16 |
| 665 | 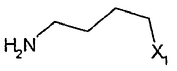 | 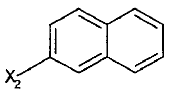 | 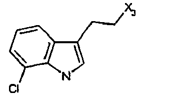 | 8,46 | 476,10 |
| 666 | 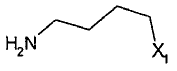 | 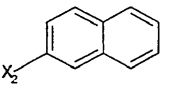 | 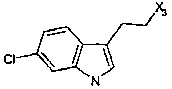 | 8,50 | 476,10 |
| 667 | 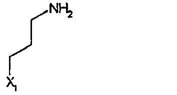 | 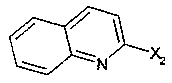 | 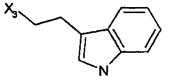 | 7,77 | 429,09 |
| 668 | 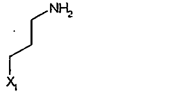 | 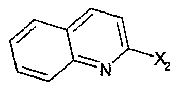 | 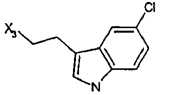 | 8,05 | 463,05 |
| 669 | 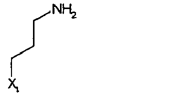 | 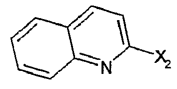 | 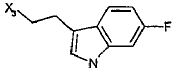 | 7,91 | 447,08 |
| 670 | 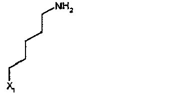 | 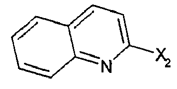 | 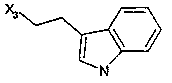 | 7,92 | 457,13 |
| 671 | 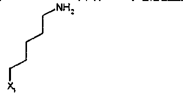 | 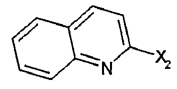 | 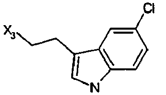 | 8,17 | 491,07 |
| 672 | 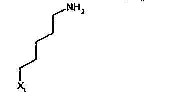 | 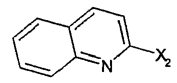 | 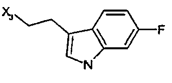 | 8,03 | 475,12 |
| 673 | 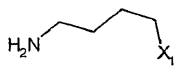 | 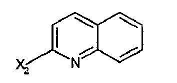 | 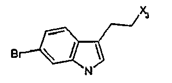 | 8,7 | 521,01 |
| 674 | 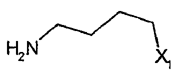 | 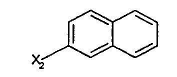 | 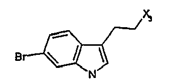 | 8,52 | 520,02 |
| 675 | 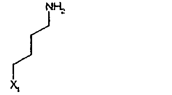 | 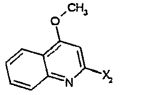 | 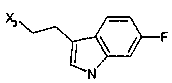 | 8,66 | 491,09 |
| 676 | 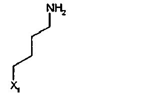 | 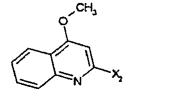 | 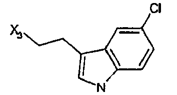 | 8,83 | 507,11 |
| 677 | 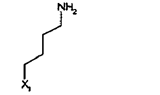 | 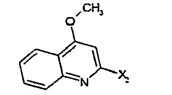 | 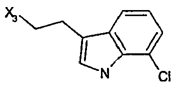 | 8,77 | 507,11 |
| 678 | 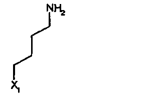 | 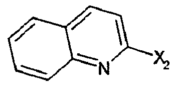 | 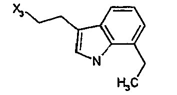 | 8,73 | 471,19 |
| 679 | 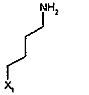 | 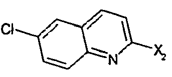 | 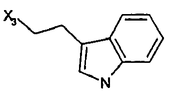 | 8,68 | 477,20 |
| 680 | 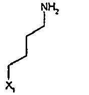 | 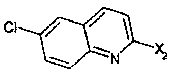 | 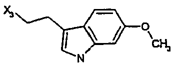 | 8,62 | 507,22 |
| 681 | 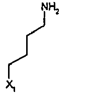 | 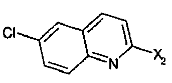 | 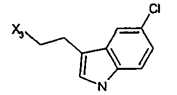 | 8,94 | 511,17 |
| 682 | 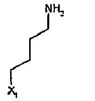 | 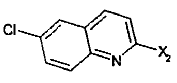 | 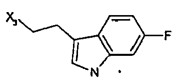 | 8,77 | 495,22 |
| 683 | 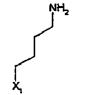 | 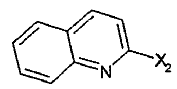 | 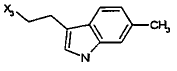 | 8,5 | 457,20 |
| 684 | 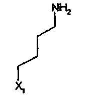 | 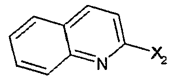 | 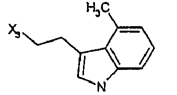 | 8,5 | 457,20 |
| 685 | 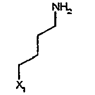 | 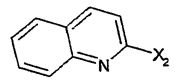 | 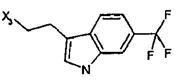 | 8,77 | 511,08 |
| 686 | 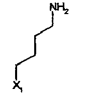 | 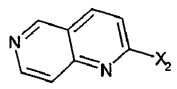 | 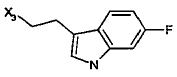 | 7,77 | 462,08 |
| 687 | 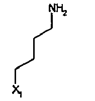 | 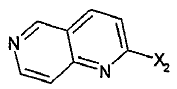 | 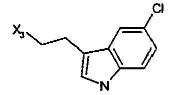 | 7,91 | 478,03 |
| 688 | 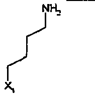 | 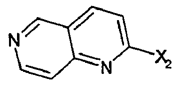 | 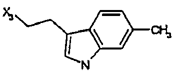 | 7,83 | 458,14 |
| 689 | 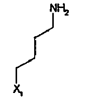 | 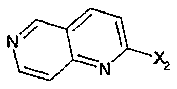 | 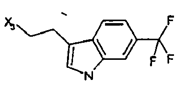 | 8,08 | 512,13 |
| 690 | 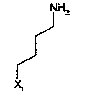 | 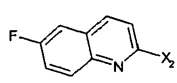 | 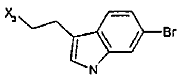 | 8,79 | 539,06 |
| 691 | 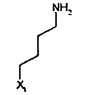 | 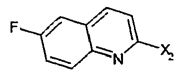 | 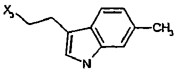 | 8,64 | 475,17 |
| 692 | 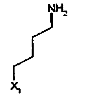 | 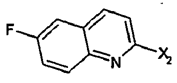 | 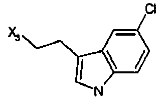 | 8,72 | 495,13 |
| 693 | 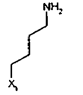 | 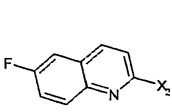 | 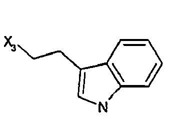 | 8,48 | 461,16 |
| 694 | 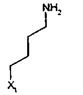 | 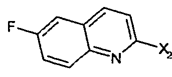 | 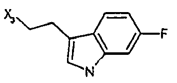 | 8,56 | 479,15 |
| 695 | 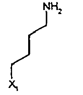 | 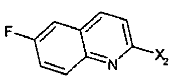 | 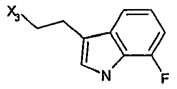 | 8,55 | 479,17 |
| 696 | 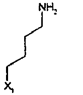 | 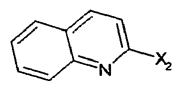 | 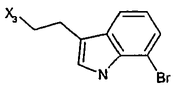 | 8,6 | 521,00 |
| 697 | 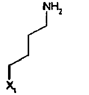 | 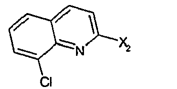 | 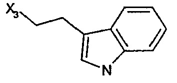 | 8,60 | 477,11 |
| 698 | 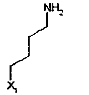 | 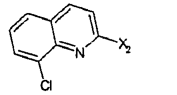 | 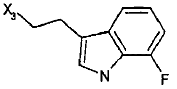 | 8,66 | 495,13 |
| 699 | 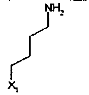 | 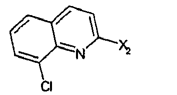 | 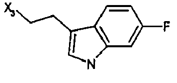 | 8,67 | 495,11 |
| 700 | 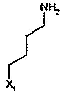 | 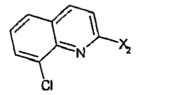 | 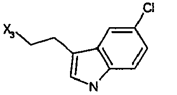 | 8,83 | 511,08 |
| 701 | 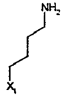 | 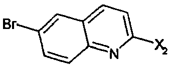 | 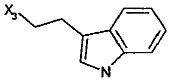 | 8,77 | 521,03 |
| 702 | 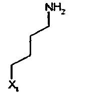 | 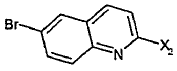 | 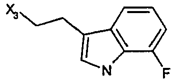 | 8,86 | 539,02 |
| 703 | 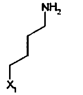 | 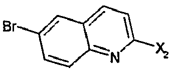 | 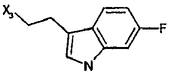 | 8,85 | 539,04 |
| 704 | 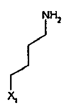 | 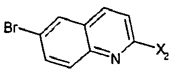 | 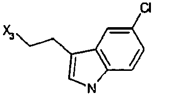 | 9,03 | 555,01 |
| 705 | 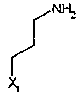 | 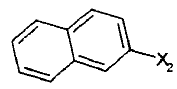 | 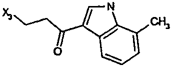 | 8,62 | 470,16 |
| 706 | 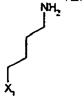 | 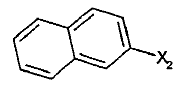 | 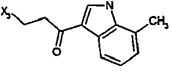 | 8,64 | 484,15 |
| 707 | 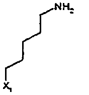 | 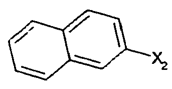 | 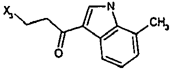 | 8,73 | 498,20 |
| 708 | 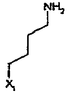 | 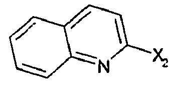 | 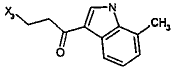 | 8,72 | 485,13 |

### Etude pharmacologique

Les composés de la présente invention ont été testés en ce qui concerne leur affinité pour différents sous-types de récepteurs de la somatostatine selon les procédures décrites ci-après.

### Etude de l'affinité pour les sous-types de récepteurs de la somatostatine humaine :

L'affinité d'un composé de l'invention pour le récepteur sous-type 2 de la somatostatine est déterminée par la mesure de l'inhibition de la liaison de [¹²⁵I-Tyr¹¹]SRIF-14 à des cellules transfectées CHO-K1. Les composés montrant une affinité sont testés sur les autres sous types, et subissent éventuellement un test fonctionnel quant à leur inhibition de la production d'AMPc intracellulaire.

Le gène du récepteur sst₁ de la somatostatine humaine a été cloné sous forme d'un fragment génomique. Un segment *Pst*I*-*X*mn*I de 1,5 Kb contenant 100 pb de la région 5' non transcrite, 1,17 Kb de la région codante en totalité, et 230 bp de la région 3' non transcrite est modifié par l'addition du linker Bg1II. Le fragment d'ADN résultant est souscloné dans le site *BamH*I d'un pCMV-81 pour donner le plasmide d'expression chez les mammifères (fourni par Dr. Graeme Bell, Univ. Chicago). Une lignée de cellules clonées exprimant de façon stable le récepteur sst₁ est obtenue par transfection dans des cellules CHO-K1 (ATCC) grâce à la méthode de co-précipitation calcium phosphate. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le gène du récepteur sst₂ de la somatostatine humaine, isolé sous forme d'un fragment génomique d'ADN de 1,7 Kb *Bam*HI*-Hind*III et souscloné dans un vecteur plasmidique pGEM3Z (Promega), a été fourni par le Dr. G. Bell (Univ. of Chicago). Le vecteur d'expression des cellules de mammifères est construit en insérant le fragment *Bam*H1-*Hind*II de 1,7 Kb dans des sites de restriction endonucléase compatibles du plasmide pCMV5. Une lignée de cellules clonées est obtenue par transfection dans des cellules CHO-K1 grâce à la méthode de co-précipitation calcium phosphate. Le plasmide pRSV-neo est inclus comme marqueur de sélection.

Le récepteur sst₃ est isolé comme fragment génomique, et la séquence codante complète est contenue dans un fragment *Bam*HI/*Hind*III de 2,4 Kb. Le plasmide d'expression chez les mammifères, pCMV-h3, est construit par insertion du fragment *Nco*I*-Hind*III de 2,0 Kb dans le site EcoR1 du vecteur pCMV après modification des terminaisons et addition de liners EcoR1. Une lignée de cellules clonées exprimant de façon stable le récepteur sst₃ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le plasmide d'expression du récepteur sst₄ humain, pCMV-HX, a été fourni par le Dr. Graeme Bell (Univ. Chicago). Ce vecteur contient le fragment génomique codant pour le récepteur sst₄ humain de 1,4 Kb *Nhe*I*-Nhe*I*,* 456 pb de la région 5' non transcrite, et 200 pb de la région 3' non transcrite, cloné dans les sites *Xba*I/*Eco*R1 de PCMV-HX. Une lignée de cellules clonées exprimant de façon stable le récepteur sst₄ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le gène correpondant au récepteur sst₅ humain, obtenu par la méthode PCR en utilisant un clone génomique λ comme sonde, a été fourni par le Dr. Graeme Bell (Univ. Chicago). Le fragment PCR résultant de 1,2 Kb contient 21 paires de bases de la région 5' non transcrites, la région codante en totalité, et 55 pb de la région 3' non transcrite. Le clone est inséré dans un site EcoR1 du plasmide pBSSK(+). L'insert est récupéré sous la forme d'un fragment *Hind*III-X*ba*I de 1,2 Kb pour sousclonage dans un vecteur d'expression chez les mammifères, pCVM5. Une lignée de cellules clonées exprimant de façon stable le récepteur sst₅ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Les cellules CHO-K1 exprimant de façon stable l'un des récepteurs sst humain sont cultivées dans un milieu RPMI 1640 contenant 10 % de sérum foetal de veau et 0,4 mg/ml de généticine. Les cellules sont collectées avec de l'EDTA 0,5 mM et centrifugées à 500 g pendant environ 5 min à environ 4° C. Le centrifugat est resuspendu dans un milieu tampon 50 mM Tris à pH 7,4 et centrifugé deux fois à 500 g pendant environ 5 min à environ 4° C. Les cellules sont lysées par sonication et centrifugées à 39000 g pendant environ 10 min à 4° C. Le centrifugat est resuspendu dans le même milieu tampon et centrifugé at 50000 g pendant 10 min à environ 4° C et les membranes dans le centrifugat obtenu sont stockées à -80° C.

Des tests d'inhibition compétitive de la liaison de la [¹²⁵I-Tyr¹¹]SRIF-14 sont effectués en double à l'aide de plaques en polypropylène de 96 puits. Les membranes cellulaires sont incubées avec [¹²⁵I-Tyr¹¹]SRIF-14 pendant environ 60 min à environ 37° C dans un milieu tampon 50 mM HEPES (pH 7,4) comprenant 0,2 % BSA, 5 mM de MgCl₂, 200 KIU/ml de Trasylol, 0,02 mg/ml de bacitracine et 0,02 mg/ml de fluorure de phénylméthylsulphonyle.

La [¹²⁵I-Tyr¹¹]SRIF-14 liée est séparée de la [¹²⁵I-Tyr¹¹]SRIF-14 libre par filtration immédiate à travers des plaques filtres en fibre de verre GF/C (Unifilter, Packard) préimprégné avec 0,1 % de polyéthylènimine (P.E.I.), en utilisant un Filtermate 196 - (Packard). Les filtres sont lavés avec du tampon 50 mM HEPES à environ 0-4° C pendant environ 4 secondes et leur radioactivité est déterminée à l'aide d'un compteur (Packard Top Count).

La liaison spécifique est obtenue en soustrayant la liaison non spécifique (déterminée en présence de 0,1 µM SRIF-14) de la liaison totale. Les données relatives à la liaison sont analysées pour calculer les pourcentages d'inhibition à une concentration donnée ou pour déterminer les valeurs des constantes d'inhibition (Ki) selon l'expérience.

La détermination du caractère agoniste ou antagoniste d'un composé de la présente invention est effectuée à l'aide du test décrit ci-après.

### Test fonctionnel : Inhibition de la production d'AMPc intracellulaire :

Des cellules CHO-K1 exprimant les sous-types de récepteurs de la somatostatine humaine (SRIF-14) sont cultivées dans des plaques à 24 puits dans un milieu RPMI 1640 avec 10 % de sérum foetal de veau et 0,4 mg/ml de généticine. Le milieu est changé le jour précédant l'expérience.

Les cellules à raison de 10⁵ cellules/puits sont lavées 2 fois avec 0,5 ml de nouveau milieu RPMI comprenant 0,2 % BSA complété par 0,5 mM de 3-isobutyl-1-méthylxanthine (IBMX) et incubées pendant environ 5 min à environ 37° C.
- la production d'AMP cyclique est stimulée par l'addition de 1 µM de forskoline (FSK) pendant 15-30 minutes à environ 37° C ;
- l'effet inhibiteur de la somatostatine d'un composé agoniste est mesuré par l'addition simultanée de FSK (1 µM) et du composé à tester (10⁻¹⁰ M à 10⁻⁵ M) ;
- l'effet antagoniste d'un composé est mesuré par l'addition simultanée de FSK (1 µM), SRIF-14 (1 nM) et du composé à tester (10⁻¹⁰ M à 10⁻⁵ M).

Le milieu réactionnel est éliminé et 200 µl de HCl 0,1 N sont ajoutés. La quantité d'AMPc est mesurée par un test radioimmunologique (Kit FlashPlate SMP001A, New England Nuclear).

### Résultants :

Les tests effectués selon les protocoles décrits ci-dessus ont permis de montrer que les produits de formule générale (I) définie dans la présente demande ont une bonne affinité pour au moins l'un des sous-types de récepteurs de la somatostatine, la constante d'inhibition Kᵢ étant inférieure au micromolaire pour certains des composés exemplifiés.

## Revendications

1. Composés de formule générale 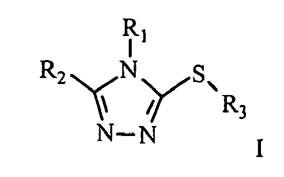 sous forme racémique, d'énantiomère ou toutes combinaisons de ces formes, dans laquelle
R₁ représente un radical de formule -(CH₂)ₙ-[Q]ₚ-(CH₂)ₘ-NXY dans lequel
X et Y représentent, indépendamment, l'atome d'hydrogène ou un (C₁-C₆)alkyle ;
p et n représentent 0, et m représente un entier de 2 à 6.
et les deux autres radicaux R₂ et R₃ représentent, indépendamment, un radical de formule -(CH₂)_{n'}[Q']_{p'}[C(X')(Y')]_{m'}Z' dans laquelle
Q' représente -O-, -S-, -C(O)-, -NH-, -CH=CH- ou -C≡C- ;
X', Y' et Z' représentent, indépendamment, un atome d'hydrogène, (C₁-C₆)alkyle, (C₁-C₆)alkoxy, (C₁-C₆)alkoxy-carbonyle, cyano, amino, (C₁-C₆)alkylamino, di((C₁-C₆)alkyl)amino, (C₃-C₇)cycloalkyle, hétérocycloalkyle, aryle ou hétéroaryle, ou bien un radical de formule 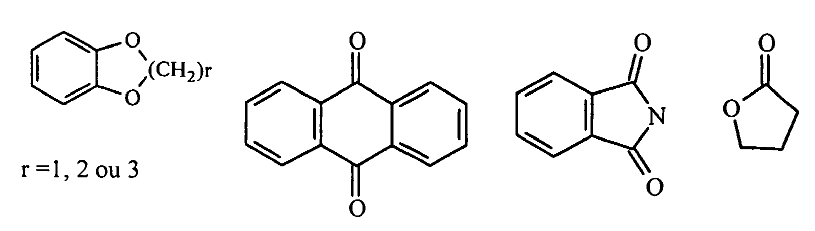 les radicaux (C₃-C₇)cycloalkyle, hétérocycloalkyle, aryle et hétéroaryle étant éventuellement substitués par un ou plusieurs substituants identiques ou différents choisis parmi : -(CH₂)_{q'}-X"-Y", hydroxy, halo, nitro, cyano, amino, (C₁-C₆)alkylamino et di((C₁-C₆)alkyl)amino ;
X" représente -O-, -S-, -C(O)-, -C(O)-O-, -SO₂- ou une liaison covalente ;
Y" représente un radical (C₁-C₆)alkyle éventuellement substitué par un ou plusieurs radicaux halo identiques ou différents ; ou aryle ou hétéroaryle éventuellement substitué par un ou plusieurs substituants identiques ou différents choisis parmi : (C₁-C₆)alkoxy, hydroxy, halo, nitro cyano, amino, (C₁-C₆)alkylamino et di((C₁-C₆)alkyl)amino ;
p' représente 0 ou 1, et n', m' et q' représentent, indépendamment, un entier de 0 à 6;
ou leurs sels d'addition avec les acides minéraux ou organiques pharmaceutiquement acceptables,
à l'exclusion des composés pour lesquels :
- R₃ représente un atome d'hydrogène, R₂ le radical méthyle et R₁ le radical 2-(diméthylamino)éthyle ;
- R₃ et R₂ représentent un atome d'hydrogène, et R₁ le radical 2-(diméthylamino)éthyle, 3-(diméthylamino)propyle, 6-(diméthylamino)hexyle, 2-(dipropylamino)éthyle ou 2-(diéthylamino)éthyle ;
- R₃ représente un atome d'hydrogène, R₂ le radical amino et R₁ le radical 2-(diméthylamino)éthyle.
- R₃ représente un atome d'hydrogène, R₂ le radical 2-hydroxyéthyle et R₁ le radical 2-(diéthylamino)éthyle.

2. Composés de formule générale I selon la revendications 1, **caractérisés en ce que** R₂ représente un radical de formule -(CH₂)_{n'}[Q']_{p'}[C(X')(Y')]_{m'}Z' dans laquelle
Q' représente -O- ;
X' représente l'atome d'hydrogène ;
Y' et Z' représentent, indépendamment, un atome d'hydrogène, (C₁-C₆)alkyl, cyano, amino, (C₃-C₇)cycloalkyle, aryle ou hétéroaryle ;
les radicaux aryle et hétéroaryle étant éventuellement substitués par un ou plusieurs substituants identiques ou différents choisis parmi : -(CH₂)_{q'}-X"-Y", hydroxy, halo, nitro, amino, (C₁-C₆)alkylamino, di((C₁-C₆)alkyl)amino ;
X" représente -O-, -S- ou une liaison covalente ;
Y" représente un radical (C₁-C₆)alkyle éventuellement substitué par un ou plusieurs radicaux halo identiques ou différents, ou aryle éventuellement substitué par un ou plusieurs radicaux halo identiques ou différents ;
p' représente 0 ou 1 ; n' représente 0, 1 ou 2 ; et m' représente un entier de 0 à 6.

3. Composés de formule générale I selon l'une des revendications 1 à 2, **caractérisés en ce que** R₂ représente un radical aryle ou hétéroaryle éventuellement substitué et plus particulièrement naphtyle, phényle, benzothiényle, quinoxalyle, quinolyle, isoquinolyle ou indolyle, les radicaux phényle, naphtyle et quinolyle étant éventuellement substitués par un ou plusieurs radicaux (C₁-C₆)alkoxy, halo, nitro, hydroxy,(C₁-C₆)alkyle identiques ou différents, le (C₁-C₆) alkyle étant lui-même éventuellement substitué par un ou plusieurs radicaux halo identiques ou différents.

4. Composés de formule générale I selon l'une des revendications 1 à 3, **caractérisés en ce que** R₃ représente un radical de formule -(CH₂)_{n'}[Q']_{p'}[C(X')(Y')]_{m'}Z' dans laquelle
X' et Y' représentent l'atome d'hydrogène ;
Z' représente indolyle ou benzothiényle, le radical indolyle étant éventuellement substitué par un ou plusieurs substituants identiques ou différents choisis parmi : -(CH₂)_{q'}-X"-Y", (C₁-C₆)alkoxy ou halo ;
X" représente -SO₂- ou une liaison covalente ;
Y" représente phényle ou alkyle éventuellement substitué par un ou plusieurs radicaux halo identiques ou différents;;
q' représente 0 ou 1 ; p' représente 0 ; n' représente 0 ou 1 ; et m' représente 0 ou 1.

5. Composés de formule générale I selon la revendication 1, **caractérisés en ce que** :
R₁ représente un radical de formule -(CH₂)ₙ-[Q]ₚ-(CH₂)ₘ-NXY dans lequel
X et Y représentent, indépendamment, l'atome d'hydrogène ou un (C₁-C₆)alkyle
p et n représentent 0 , et m représente un entier de 2 à 6.
R₂ représente quinoxalyle, quinolyle ou naphtyl, les radicaux quinolyle et naphtyle étant éventuellement substitués par un ou plusieurs radicaux (C₁-C₆)alkyl, (C₁-C₆)alkoxy, halo, identiques ou différents ;
R₃ représente un radical de formule -(CH₂)_{n'}[Q']_{p'}[C(X')(Y')]_{m'}Z' dans laquelle
X' et Y' représentent l'atome d'hydrogène ;
Z' représente indolyle éventuellement substitués par un ou plusieurs substituants identiques ou différents choisis parmi : -(CH₂)_{q'}-X"-Y", (C₁-C₆)alkoxy ou halo ;
X" représente une liaison covalente ;
Y" représente un radical alkyl éventuellement substitué par un ou plusieurs radicaux halo identiques ou différents ;
q' représente 0 ou 1 ; p' représente 0 ; n' représente 0 ou 1 ; et m' représente 0 ou 1

6. Procédé de préparation, en phase liquide, de composés de formule générale I telle que définie à la revendication 1, **caractérisé en ce qu'**il comprend
la réaction des isothiocyanates de formule R₁-NCS sur des hydrazides de formule R₂-C(O)-NH-NH₂ dans lesquels R₁ et R₂ ont la signification indiquée aux revendications 1 et 2 respectivement, pour obtenir les composés de formule (5) 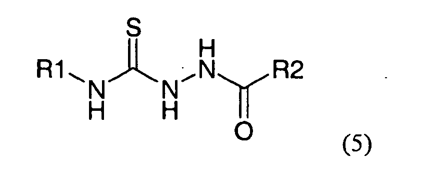 composés de formule (5) qui peuvent être soumis à un traitement basique pour obtenir les composés correspondants de formule (6) 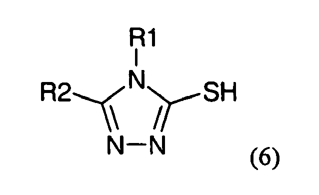 composés de formule (6) que l'on fait réagir avec
A) soit un composé de formule Br-(CH₂)_{n'}[Q']_{p'}[C(X')(Y')]_{m'}Z' où n' = 1, p' = m' = 0 et Z' a la signification indiquée à la revendication 1 pour obtenir, après déprotection de la fonction amine présente sur la molécule, le composé de formule (I) correspondant,
B) soit un composé de formule Br-(CH₂)_{n'}[Q']_{p'}[C(X')(Y')]_{m'}Z' où n' = 1, Q' = -C(O)-, m' = 0 et Z' a la signification indiquée à la revendication 1 pour obtenir, après déprotection de la fonction amine présente sur la molécule, le composé de formule (I) correspondant,
C) soit un composé de formule Br-(CH₂)_{n'}[Q']_{p'}[C(X')(Y')]_{m'}Z' où Q', X', Y', Z', n', p' et m' ont la signification indiquée à la revendication 1 pour obtenir, après déprotection de la fonction amine présente sur la molécule, le composé de formule (I) correspondant.

7. Compositions pharmaceutiques contenant, à titre de principe actif, un au moins des composés tels que définis à l'une des revendications 1 à 5, en association avec un support pharmaceutiquement acceptable.

8. Compositions pharmaceutiques pour une utilisation comme médicament et contenant, à titre de principe actif, un au moins des composés de formule 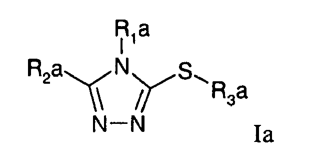 sous forme racémique, d'énantiomère ou toutes combinaisons de ces formes, dans laquelle
R₁ₐ représente un radical de formule -(CH₂)ₙ-[Q]ₚ-(CH₂)ₘ-NXY dans lequel
X et Y représentent, indépendamment, l'atome d'hydrogène ou un (C₁-C₆)alkyle ;
p et n représentent 0, et m représente un entier de 2 à 6.
et les deux autres radicaux R₂ₐ et R₃ₐ représentent, indépendamment, un radical de formule -(CH₂)_{n'}[Q']_{p'}[C(X')(Y')]_{m'}Z' dans laquelle
Q' représente -O-, -S-, -C(O)-, -NH-, -CH=CH- ou -C≡C- ;
X', Y' et Z' représentent, indépendamment, un atome d'hydrogène, (C₁-C₆)alkyle, (C₁-C₆)alkoxy, (C₁-C₆)alkoxy-carbonyle, cyano, amino, (C₁-C₆)alkylamino, di((C₁-C₆)alkyl)amino, (C₃-C₇)cycloalkyle, hétérocycloalkyle, aryle ou hétéroaryle, ou bien un radical de formule 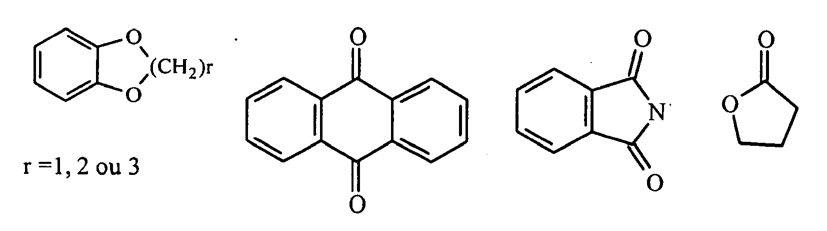 les radicaux (C₃-C₇)cycloalkyle, hétérocycloalkyle, aryle et hétéroaryle étant éventuellement substitués par un ou plusieurs substituants identiques ou différents choisis parmi : -(CH₂)_{q'}-X"-Y", hydroxy, halo, nitro, cyano, amino, (C₁-C₆)alkylamino et di((C₁-C₆)alkyl)amino ;
X" représente -O-, -S-, -C(O)-, -C(O)-O-, -SO₂ ou une liaison covalente ;
Y" représente un radical (C₁-C₆)alkyle éventuellement substitué par un ou plusieurs radicaux halo identiques ou différents ; ou aryle ou hétéroaryle éventuellement substitué par un ou plusieurs substituants identiques ou différents choisis parmi : (C₁-C₆)alkoxy, hydroxy, halo, nitro cyano, amino, (C₁-C₆)alkylamino et di((C₁-C₆)alkyl)amino ;
p' représente 0 ou 1, et n', m' et q' représentent, indépendamment, un entier de 0 à 6 ;
ou leurs sels d'addition avec les acides minéraux ou organiques pharmaceutiquement acceptables, en association avec un support pharmaceutiquement acceptable.

## Claims

1. Compounds of general formula 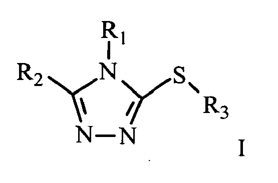 in racemic, enantiomeric form or any combination of these forms, in which:
R₁ represents a radical of formula -(CH₂)ₙ-[Q]ₚ-(CH₂)ₘ-NXY in which,
X and Y represent, independently, the hydrogen atom, a (C₁-C₆)alkyl,
p and n represent 0, and m represents an integer from 2 to 6;
and the two other radicals R₂ and R₃ represent, independently, a radical of formula -(CH₂)_{n'}[Q']_{p'}[C(X')(Y')]_{m'}Z' in which
Q' represents -O-, -S-, -C(O)-, -NH-, -CH=CH- or -C≡C- ;
X', Y' and Z' represent, independently, a hydrogen atom, (C₁-C₆)alkyl, (C₁-C₆)alkoxy, (C₁-C₆)alkoxy-carbonyl, cyano, amino, (C₁-C₆)alkylamino, di((C₁-C₆)alkyl)amino, (C₃-C₇)cycloalkyl, heterocycloalkyl, aryl or heteroaryl, or a radical of formula 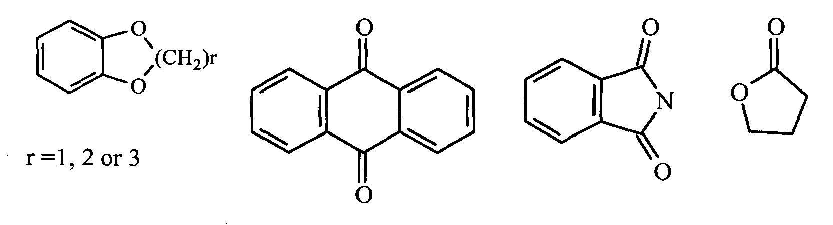 the (C₃-C₇)cycloalkyl, heterocycloalkyl, aryl and heteroaryl radicals being optionally substituted by one or more identical or different substituents chosen from: -(CH₂)_{q'}-X"-Y", hydroxy, halo, nitro, cyano, amino, (C₁-C₆)alkylamino and di((C₁-C₆)alkyl)amino;
X" represents -O-, -S-, -C(O)-, -C(O)-O-, -SO₂ or a covalent bond;
Y" represents a (C₁-C₆)alkyl radical optionally substituted by one or more identical or different halo radicals; or aryl or heteroaryl radical optionally substituted by one or more identical or different substituents chosen from: (C₁-C₆)alkoxy, hydroxy, halo, nitro cyano, amino, (C₁-C₆)alkylamino and di((C₁-C₆)alkyl)amino;
p' represents 0 or 1, and n', m' and q' represent, independently, an integer from 0 to 6;
or their addition salts with pharmaceutically acceptable mineral or organic acids,
excluding compounds in which:
- R₃ represents a hydrogen atom, R₂ the methyl radical, and R₁ the 2-(dimethylamino)ethyl radical;
- R₃ and R₂ represent a hydrogen atom, and R₁ the 2-(dimethylamino)ethyl, 3-(dimethylamino)propyl, 6-(dimethylamino)hexyl, 2-(dipropylamino)ethyl or 2-(diethylamino)ethyl radical ;
- R₃ represents a hydrogen atom, R₂ the amino radical, and R₁ the 2-(dimethylamino)ethyl radical ;
- R₃ represents a hydrogen atom, R₂ the 2-hydroxyethyl radical, and R₁ the 2-(diethylamino)ethyl radical ;

2. Compounds of general formula I according to claim 1, **characterized in that** R₂ represents a radical of formula -(CH₂)_{n'}[Q']_{p'}[C(X')(Y')]_{m'}Z';
Q' represents -O-;
X' represents the hydrogen atom;
Y' and Z' represent, independently, a hydrogen atom, (C₁-C₆)alkyl, cyano, amino, (C₃-C₇)cycloalkyl, aryl or heteroaryl;
the aryl and heteroaryl radicals being optionally substituted by one or more identical or different substituents chosen from: -(CH₂)_{q'}-X"-Y", hydroxy, halo, nitro, amino, (C₁-C₆)alkylamino, di((C₁-C₆)alkyl)amino;
X" represents -O-, -S- or a covalent bond;
Y" represents a (C₁-C₆)alkyl radical optionally substituted by one or more identical or different halo radicals, or aryl radical optionally substituted by one or more identical or different halo radicals;
p' represents 0 or 1; n' represents 0, 1 or 2; and m' represents an integer from 0 to 6.

3. Compounds of general formula I according to one of claims 1 or 2, **characterized in that** R2 represents a aryl or heteroaryl radical optionally substituted and more particularly naphthyl, phenyl, benzothienyl, quinoxalyl, quinolyl, isoquinolyl, or indolyl, the phenyl, naphthyl and quinolyl radicals being optionally substituted by one or more identical or different (C₁-C₆)alkoxy, halo, nitro, hydroxyl, (C₁-C₆)alkyl radicals, the (C₁-C₆)alkyl being optionally substituted by one or more identical or different halo radicals.

4. Compounds of general formula I according to one of claims 1 to 3, **characterized in that** R₃ represents a radical of formula -(CH₂)_{n'}[Q']_{p'}[C(X')(Y')]_{m'}Z' in which
X' and Y' represent the hydrogen atom;
Z' represents indolyl or benzothienyl, the indolyl radical being optionally substituted by one or more identical or different substituents chosen from: -(CH₂)_{q'}-X"-Y", (C₁-C₆)alkoxy or halo;
X" represents -SO₂ or a covalent bond;
Y" represents phenyl or alkyl optionally substituted by one or more identical or different halo radicals.;
q' represents 0 or 1; p' represents 0; n' represents 0 or 1; and m' represents 0 or 1.

5. Compounds of general formula I according to claim 1, **characterized in that**
R₁ represents a radical of formula -(CH₂)ₙ-[Q]ₚ-(CH₂)ₘ-NXY in which,
X and Y represent, independently, the hydrogen atom or (C₁-C₆)alkyl ;
p and n represent 0 , and m represents an integer from 2 to 6.
R₂ represents quinoxalyl, quinolyl or naphthyl, the quinolyl and naphthyl radicals being optionally substituted by one or more identical or different (C₁-C₆)alkyl, (C₁-C₆)alkoxy or halo radicals ;
R₃ represents a radical of formula -(CH₂)_{n'}[Q']_{p'}[C(X')(Y')]_{m'}Z'
X' and Y' represent the hydrogen atom ;
Z' represents indolyl optionally substituted by one or more identical or different substituents chosen from : -(CH₂)_{q'}-X"-Y", (C₁-C₆)alkoxy or halo ;
X" represents a covalent bond ;
Y" represents alkyl optionally substituted by one or more identical or different halo radicals ;
q' represents 0 or 1 ; p' representse 0 ; n' represents 0 or 1 ; and m' represents 0 or 1.

6. Process for the preparation, in liquid phase, of compounds of general formula I as defined in claim 1, **characterized in that** it comprises
the reaction of the isothiocyanates of formula R₁-NCS on of the hydrazides of formula R₂-C(O)-NH-NH₂ in which R₁ and R₂ have the meaning indicated in claims 1 and 2 respectively, in order to obtain the compounds of formula (5) 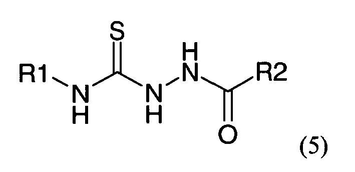 which compounds of formula (5) can be subjected to a basic treatment in order to obtain the corresponding compounds of formula (6) 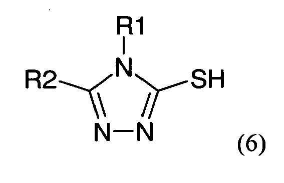 which compounds of formula (6) are reacted with
A) either a compound of formula Br-(CH₂)_{n'}[Q']_{p'}[C(X')(Y')]_{m'}Z' where n' = 1, p' = m' = 0 and Z' has the meaning indicated in claim 1 in order to obtain, after deprotection of the amine function present on the molecule, the corresponding compound of formula (I),
B) or a compound of formula Br-(CH₂)ₙ-[Q']_{p'}[C(X')(Y')]_{m'}Z' where n' = 1, Q' = -C(O)-, m' = 0 and Z' has the meaning indicated in claim 1 in order to obtain, after deprotection of the amine function present on the molecule, the corresponding compound of formula (I),
C) or a compound of formula Br-(CH₂)_{n'}[Q']_{p'}[C(X')(Y')]_{m'}Z' where Q', X', Y', Z', n', p' and m' have the meaning indicated in claim 1 in order to obtain, after deprotection of the amine function present on the molecule, the corresponding compound of formula (I).

7. Pharmaceutical compositions containing, as active ingredient, at least of the compounds as defined in one of claims 1 to 5, combined with a pharmaceutically acceptable support.

8. Pharmaceutical compositions for use as medicament and containing, as active ingredient, in association with a pharmaceutically acceptable support, at least one compound of the general formula 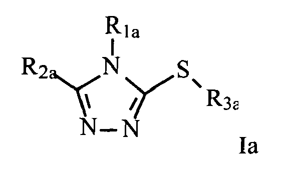 in racemic, enantiomeric form or all combinations of these forms, in which:
R₁ₐ, represents a radical of formula -(CH₂)ₙ-[Q]ₚ-(CH₂)ₘ-NXY in which, X and Y represent, independently, the hydrogen atom, or a (C₁-C₆)alkyl,; p and n represent 0, and m represents an integer from 2 to 6;
and the two other radicals R₂ₐ and R₃ₐ represent, independently, a radical of formula -(CH₂)_{n'}[Q']_{p'}[C(X')(Y')]_{m'}Z' in which,
Q' represents -O-, -S-, -C(O)-, -NH-, -CH=CH- or -C≡C- ; X', Y' and Z' represent, independently, a hydrogen atom, (C₁-C₆)alkyl, (C₁-C₆)alkoxy, (C₁-C₆)alkoxy-carbonyl, cyano, amino, (C₁-C₆)alkylamino, di((C₁-C₆)alkyl)amino, (C₃-C₇)cycloalkyl, heterocycloalkyl, aryl or heteroaryl, or a radical of formula 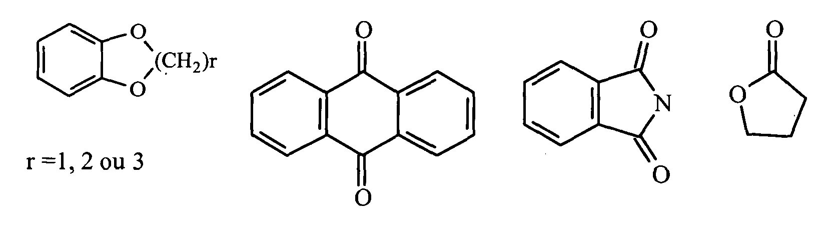 the (C₃-C₇)cycloalkyl, heterocycloalkyl, aryl and heteroaryl radicals being optionally substituted by one or more identical or different substituents chosen from: -(CH₂)_{q'}-X"-Y", hydroxy, halo, nitro, cyano, amino, (C₁-C₆)alkylamino and die((C₁-C₆)alkyl)amino;
X" represents -O-, -S-, -C(O)-, -C(O)-O-, -SO₂ or a covalent bond;
Y" represents a (C₁-C₆)alkyl radical optionally substituted by one or more identical or different halo radicals; or aryl or heteroaryl radical optionally substituted by one or more identical or different substituents chosen from: (C₁-C₆)alkoxy, hydroxy, halo, nitro cyano, amino, (C₁-C₆)alkylamino and di((C₁-C₆)alkyl)amino;
p' represents 0 or 1, and n', m' and q' represent, independently, an integer from 0 to 6 ;
or their addition salts with pharmaceutically acceptable mineral or organic acids, combined with a pharmaceutically acceptable support.

## Patentansprüche

1. Verbindungen der allgemeinen Formel 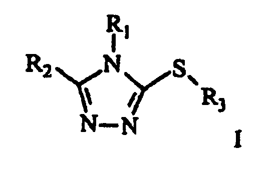 in racemischer Form, enantiomerer Form oder allen Kombinationen dieser Formen, in der
R₁ einen Rest der Formel -(CH₂)ₙ-[Q]ₚ-(CH₂)ₘ-NXY darstellt, in der
X und Y unabhängig ein Wasserstoffatom oder (C₁₋₆)-Alkyl darstellen;
p und n 0 darstellen und m eine ganze Zahl von 2 bis 6 darstellt,
und die zwei anderen Reste R₂ und R₃ unabhängig einen Rest der Formel -(CH₂)_{n'}[Q']_{p'}[C(X')(Y')]_{m'}Z' darstellen, in der
Q' -O-, -S-, -C(O)-, -NH-, -CH=CH- oder -C≡C-darstellt;
X', Y' und Z' unabhängig ein Wasserstoffatom, (C₁₋₆)-Alkyl, (C₁₋₆)-Alkoxy, (C₁₋₆)-Alkoxycarbonyl,
Cyano, Amino, (C₁₋₆)-Alkylamino, Di((C₁₋₆)-alkyl)amino, (C₃₋₇)-Cycloalkyl, Heterocycloalkyl, Aryl oder Heteroaryl oder auch einen Rest der Formel 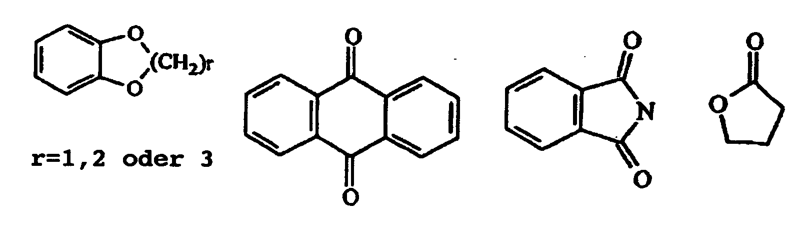 darstellen, wobei die Reste (C₃₋₇)-Cycloalkyl, Heterocycloalkyl, Aryl und Heteroaryl gegebenenfalls mit einem Substituenten oder mehreren Substituenten, die identisch oder verschieden sind, ausgewählt aus -(CH₂)_{q'}-X"-Y", Hydroxy, Halogen, Nitro, Cyano, Amino, (C₁₋₆)-Alkylamino und Di((C₁₋₆)-alkyl)amino, substituiert sind;
X" -O-, -S-, -C(O)-, -C(O)-O-, -SO₂- oder eine kovalente Bindung darstellt;
Y" einen (C₁₋₆)-Alkyl-Rest, gegebenenfalls substituiert mit einem Halogen-Rest oder mehreren Halogen-Resten, die identisch oder verschieden sind, oder einen Aryl- oder Heteroaryl-Rest, gegebenenfalls substituiert mit einem Substituenten oder mehreren Substituenten, die identisch oder verschieden sind, ausgewählt aus: (C₁₋₆)-Alkoxy, Hydroxy, Halogen, Nitro, Cyano, Amino, (C₁₋₆)-Alkylamino und Di((C₁₋₆)-alkyl)amino, darstellt;
p' 0 oder 1 darstellt und n', m' und q' unabhängig eine ganze Zahl von 0 bis 6 darstellen;
oder ihre Additionssalze mit pharmazeutisch verträglichen Mineral- oder organischen Säuren,
mit Ausnahme der Verbindungen, für die:
- R₃ ein Wasserstoffatom darstellt, R₂ einen MethylRest darstellt und R₁ einen 2-(Dimethylamino)ethyl-Rest darstellt;
- R₃ und R₂ ein Wasserstoffatom darstellen und R₁ einen 2-(Dimethylamino)ethyl-, 3-(Dimethylamino)propyl-, 6-(Dimethylamino)hexyl-, 2-(Dipropylamino)ethyl- oder 2-(Diethylamino)ethyl-Rest darstellt;
- R₃ ein Wasserstoffatom darstellt, R₂ einen AminoRest und R₁ einen 2-(Dimethylamino)ethyl-Rest darstellt;
- R₃ ein Wasserstoffatom, R₂ einen 2-Hydroxyethyl-Rest und R₁ einen 2-(Diethylamino)ethyl-Rest darstellt.

2. Verbindungen der allgemeinen Formel (I) gemäß Anspruch 1, **dadurch gekennzeichnet, daß** R₂ einen Rest der Formel -(CH₂)ₙ'[Q']_{p'}[C(X')(Y')]_{m'}Z' darstellt, in der
Q' -O- darstellt;
X' ein Wasserstoffatom darstellt;
Y' und Z' unabhängig ein Wasserstoffatom, (C₁₋₆)-Alkyl, Cyano, Amino, (C₃₋₇)-Cycloalkyl, Aryl oder Heteroaryl darstellen;
wobei die Aryl- und Heteroaryl-Reste gegebenenfalls mit einem Substituenten oder mehreren Substituenten, die identisch oder verschieden sind, ausgewählt aus: -(CH₂)_{q'}-X"-Y", Hydroxy, Halogen, Nitro, Amino, (C₁₋₆)-Alkylamino, Di((C₁₋₆)-alkyl)amino, substituiert sind;
X" -O-, -S- oder eine kovalente Bindung darstellt;
Y" einen (C₁₋₆)-Alkyl-Rest, gegebenenfalls substituiert mit einem Halogen-Rest oder mehreren Halogen-Resten, die identisch oder verschieden sind, oder einen Aryl-Rest, gegebenenfalls substituiert mit einem Halogen-Rest oder mehreren Halogen-Resten, die identisch oder unterschiedlich sind, darstellt;
p' 0 oder 1 darstellt; n' 0, 1 oder 2 darstellt und m' eine ganze Zahl von 0 bis 6 darstellt.

3. Verbindungen der allgemeinen Formel (I) gemäß einem der Ansprüche 1 bis 2, **dadurch gekennzeichnet, daß** R₂ einen Aryl- oder Heteroaryl-Rest, der gegebenenfalls substituiert ist, darstellt und insbesondere Naphthyl, Phenyl, Benzothienyl, Chinoxalyl, Chinolyl, Isochinolyl oder Indolyl darstellt, wobei die Phenyl-, Naphthyl- und Chinolyl-Reste gegebenenfalls mit einem oder mehreren (C₁₋₆)-Alkoxy-, Halogen-, Nitro-, Hydroxy-, (C₁₋₆)-Alkyl-Rest(en), die identisch oder verschieden sind, substituiert sind, wobei (C₁₋₆)-Alkyl gegebenenfalls selbst mit einem Halogen-Rest oder mehreren Halogen-Resten, die identisch oder verschieden sind, substituiert ist.

4. Verbindungen der allgemeinen Formel (I) gemäß einem der Ansprüche 1 bis 3, **dadurch gekennzeichnet, daß** R₃ einen Rest der Formel -(CH₂)ₙ'[Q']ₚ'[C(X')(Y')]_{m'}Z' darstellt, in der
X' und Y' ein Wasserstoffatom darstellen;
Z' Indolyl oder Benzothienyl darstellt, wobei der Indolyl-Rest gegebenenfalls mit einem Substituenten oder mehreren Substituenten, die identisch oder verschieden sind, ausgewählt aus -(CH₂)_{q'}-X"-Y", (C₁₋₆)-Alkoxy und Halogen, substituiert ist;
X" -SO₂- oder eine kovalente Bindung darstellt;
Y" Phenyl oder Alkyl, gegebenenfalls substituiert mit einem Halogen-Rest oder mehreren Halogen-Resten, die identisch oder verschieden sind, darstellt;
q' 0 oder 1 darstellt; p' 0 darstellt; n' 0 oder 1 darstellt und m' 0 oder 1 darstellt:

5. Verbindungen der allgemeinen Formel (I) gemäß Anspruch 1, **dadurch gekennzeichnet, daß:**
R₁ einen Rest der Formel -(CH₂)ₙ-[Q]ₚ-(CH₂)ₘ-NXY darstellt, in der
X und Y unabhängig ein Wasserstoffatom oder (C₁₋₆)-Alkyl darstellen;
p und n 0 darstellen und m eine ganze Zahl von 2 bis 6 darstellt;
R₂ Chinoxalyl, Chinolyl oder Naphthyl darstellt, wobei die Chinolyl- und Naphthyl-Reste gegebenenfalls mit einem oder mehreren (C₁₋₆)-Alkyl-, (C₁₋₆)-Alkoxy-, Halogen-Rest(en), die identisch oder verschieden sind, substituiert sind;
R₃ einen Rest der Formel - (CH₂)_{n'}[Q']_{p'}[C(X')(Y')]_{m'}Z' darstellt, in der
X' und Y' ein Wasserstoffatom darstellen;
Z' Indolyl, gegebenenfalls substituiert mit einem Substituenten oder mehreren Substituenten, die identisch oder verschieden sind, ausgewählt aus -(CH₂)_{q'}-X"-X", (C₁₋₆)-Alkoxy und Halogen, darstellt;
X" eine kovalente Bindung darstellt;
Y" einen Alkyl-Rest, gegebenenfalls substituiert mit einem Halogen-Rest oder mehreren Halogen-Resten, die identisch oder verschieden sind, darstellt;
q' 0 oder 1 darstellt; p' 0 darstellt; n' 0 oder 1 darstellt und m' 0 oder 1 darstellt.

6. Verfahren zur Herstellung von Verbindungen der allgemeinen Formel (I), wie sie in Anspruch 1 definiert sind, in flüssiger Phase, **dadurch gekennzeichnet, daß** es umfaßt:
die Reaktion von Isothiocyanaten der Formel R₁-NCS mit Hydraziden der Formel R₂-C(O)-NH-NH₂, in denen R₁ und R₂ die für die Ansprüche und 1 bzw. 2 angegebene Bedeutung haben, unter Erhalt der Verbindungen der Formel (5) 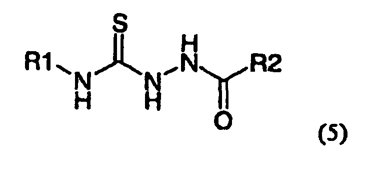
wobei Verbindungen der Formel (5) einer basischen Behandlung unterworfen werden können, um die entsprechenden Verbindungen der Formel (6) zu erhalten 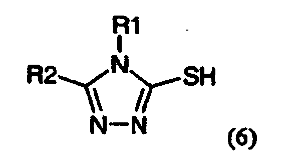 wobei man Verbindungen der Formel (6) reagieren läßt mit
A) entweder einer Verbindung der Formel Br-(CH₂)_{n'}[Q']_{p'}[C(X')(Y')_{m'}Z', worin n' = 1, p' = m' = 0 und Z' die für Anspruch 1 angegebene Bedeutung hat, um nach Entschützen der in dem Molekül vorliegenden Aminfunktion die entsprechende Verbindung der Formel (I) zu erhalten;
B) oder einer Verbindung der Formel Br-(CH₂)_{n'}[Q']_{p'}[C(X')(Y')]_{m'}Z', worin n' = 1, Q' = -C(O)-, m' = 0 und Z' die für Anspruch 1 angegebene Bedeutung hat, um nach Entschützen der im Molekül vorhandenen Aminfunktion die entsprechende Verbindung der Formel (I) zu erhalten;
C) oder einer Verbindung der Formel Br-(CH₂)n'[Q']_{p'}[C(X')(Y')]_{m'}Z', worin Q' , X' , Y', Z', n', p' und m' die für Anspruch 1 angegebene Bedeutung haben, um nach Entschützen der im Molekül vorhandenen Aminfunktion die entsprechende Verbindung der Formel (I) zu erhalten.

7. Pharmazeutische Zusammensetzungen, die wenigstens eine der Verbindungen, wie sie in einem der Ansprüche 1 bis 5 definiert sind, als Wirkstoff in Verbindung mit einem pharmazeutisch verträglichen Träger enthalten.

8. Pharmazeutische Zusammensetzungen für eine Verwendung als Medikament enthaltend als Wirkstoff wenigstens eine der Verbindungen der Formel 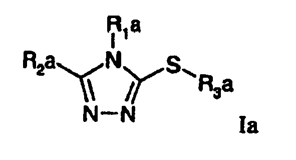 in racemischer Form, enantiomerer Form oder allen Kombinationen dieser Formen, in der
R₁ₐ einen Rest der Formel -(CH₂)ₙ-[Q]ₚ-(CH₂)ₘ-NXY darstellt,
in der X und Y unabhängig ein Wasserstoffatom oder (C₁₋₆)-Alkyl darstellen;
p und n 0 darstellen, und m eine ganze Zahl von 2 bis 6 darstellt,
und die zwei anderen Reste R₂ₐ und R₃ₐ unabhängig einen Rest der Formel -(CH₂)_{n'}[Q']_{p'}[C(X')(Y')]_{m'}Z-darstellen, in der
Q' -O-, -S-, -C(O)-, -NH-, -CH=CH- oder -C≡C- darstellt;
X', Y' und Z' unabhängig ein Wasserstoffatom, (C₁₋₆)-Alkyl, (C₁₋₆)-Alkoxy, (C₁₋₆)-Alkoxycarbonyl, Cyano, Amino, (C₁₋₆)-Alkylamino, Di((C₁₋₆)-alkyl)amino, (C₃₋₇)-Cycloalkyl, Heterocycloalkyl, Aryl oder Heteroaryl oder auch einen Rest der Formel 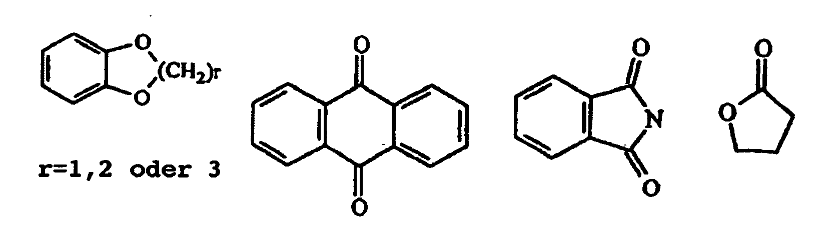 darstellen,
wobei die (C₃₋₇)-Cycloalkyl-, Heterocycloalkyl-, Aryl- und Heteroaryl-Reste gegebenenfalls mit einem oder mehreren Substituenten, die identisch oder verschieden sind, ausgewählt aus -(CH₂)_{q'}-X"-X", Hydroxy, Halogen, Nitro, Cyano, Amino, (C₁₋₆)-Alkylamino und Di((C₁₋₆)-alkyl)amino, substituiert sind;
X" -O-, -S-, -C(O)-, -C(O)-O-, -SO₂ oder eine kovalente Bindung darstellt;
Y" einen (C₁₋₆)-Alkyl-Rest, gegebenenfalls substituiert mit einem Halogen-Rest oder mehreren Halogen-Resten, die identisch oder unterschiedlich sind, oder einen Aryl- oder Heteroaryl-Rest, gegebenenfalls substituiert mit einem Substituenten oder mehreren Substituenten, die identisch oder verschieden sind, ausgewählt aus: (C₁₋₆)-Alkoxy, Hydroxy, Halogen, Nitro, Cyano, Amino, (C₁₋₆)-Alkylamino und Di((C₁₋₆)-alkyl)amino, darstellt;
p' 0 oder 1 darstellt und n', m' und q' unabhängig eine ganze Zahl von 0 bis 6 darstellen;
oder ihre Additionssalze mit pharmazeutisch verträglichen Mineral- oder organischen Säuren in Verbindung mit einem pharmazeutisch verträglichen Träger.
